(19) Europäisches Patentamt
European Patent Office
Office européen des brevets

(11) EP 4 598 608 B1

(12) **EUROPEAN PATENT SPECIFICATION**

(45) Date of publication and mention of the grant of the patent:
**25.03.2026 Bulletin 2026/13**

(21) Application number: **24783820.4**

(22) Date of filing: **09.09.2024**

(51) International Patent Classification (IPC):
***A61M 3/02*** *(2006.01)*

(52) Cooperative Patent Classification (CPC):
(C-Sets available)
**A61M 3/0233; A61K 31/155; A61K 31/444; A61P 31/00;** A61M 3/0262; A61M 3/0279 (Cont.)

(86) International application number:
**PCT/US2024/045798**

(87) International publication number:
**WO 2025/144475 (03.07.2025 Gazette 2025/27)**

(54) **WOUND IRRIGATION DEVICES AND METHODS FOR DELIVERING SYNERGISTIC ACTIVE AGENTS**

WUNDSPÜLUNGS-VORRICHTUNGEN UND -VERFAHREN ZUR ABREICHUNG VON SYNERGISCHEN WIRKSTOFFEN

DISPOSITIFS ET MÉTHODES D'IRRIGATION DE BLESSURE POUR L'ADMINISTRATION D'AGENTS ACTIFS SYNÉRGIQUES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.12.2023 US 202318401051**

(43) Date of publication of application:
**13.08.2025 Bulletin 2025/33**

(60) Divisional application:
**26158402.3**

(73) Proprietor: **Irrimax Corporation**
**Lawrenceville, GA 30043 (US)**

(72) Inventors:
- **RUCINSKI, Paul**
  **Lawrenceville, GA 30043 (US)**
- **GIL, Michael A.**
  **Lawrenceville, GA 30043 (US)**
- **KATRAGADDA, Venkata**
  **Lawrenceville, GA 30043 (US)**

(74) Representative: **Maiwald GmbH**
**Elisenhof**
**Elisenstraße 3**
**80335 München (DE)**

(56) References cited:
**EP-A1- 3 117 849 US-A1- 2022 054 734**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 598 608 B1

(52) Cooperative Patent Classification (CPC): (Cont.)

C-Sets
**A61K 31/155, A61K 2300/00;**
**A61K 31/444, A61K 2300/00**

## Description

### BACKGROUND OF THE INVENTION

One of the great challenges in the health care profession is delivering drugs and other therapeutic agents to the site(s) where their activity is needed. There are many therapeutic agents whose usefulness is compromised because current methods of delivery and administration do not result in optimal presentation of the agent to the site(s) where its activity would be most beneficial.

There is a wide variety of options for administering therapeutic agents to a patient. Each route of administration poses unique challenges. These challenges include formulating the active agent in a physiologically-acceptable carrier, directing the agent to the appropriate site, delivering a concentration and amount of active agent that is effective and not toxic, and avoiding degradation of the agent such as that which occurs when an agent is administered systemically and is exposed to enzymes, the immune system and various metabolic processes.

One particularly challenging environment for delivering active ingredients is to wound sites (e.g., surgical sites, and other tissue openings). These sites often require the administration of active ingredients of a nature and at a concentration that is difficult, if not impossible, to achieve utilizing systemic routes, such as oral and intravenous administration. Direct administration of an active ingredient is often desirable, yet such direct administration using conventional techniques faces formidable challenges in terms of delivering active agents to the specific tissues and cells where the beneficial activities are most needed. In this regard, it should be noted that conventional methods of wound irrigation have not typically been combined with contemporaneous drug delivery.

In the management and treatment of a wound there are three primary objectives: (1) prevention of infection, (2) preservation and/or restoration of function, and (3) preservation and/or restoration of cosmetic appearance. The most important of these objectives is the prevention of infection. Success in the prevention of infection directly affects the healing process and the degree to which function and cosmetic appearance can be preserved and/or restored. However, heretofore, wound irrigation has not been directly combined with the administration of drugs (e.g., a synergistic combination of two or more antiseptic agents) that can reduce infection or otherwise promote healing.

It is known that the number of bacteria present in a wound is a critical determinant of whether a wound becomes infected. Experimental evidence suggests that a critical level of bacteria is approximately $10^5$ organisms per gram of tissue. Below this level, wounds typically heal; at levels greater than $10^5$ bacteria per gram of tissue, wounds often become infected. All traumatic wounds are contaminated by the time the wound is presented to a medical care facility for treatment (Dire, Daniel I. [1990] "A comparison of Wound Irrigation Solutions Used in the Emergency Department," Annals of Emergency Medicine 19(6):704-708). Dirty wounds, or those that have not been treated within six hours, are likely to be contaminated with bacteria at levels that are higher than the critical level. Reducing the number of bacteria in and around the wound is critical for avoiding infection and expediting wound healing.

Methicillin-resistant *Staphylococcus aureus* (MRSA) infection is caused by *Staphylococcus aureus* bacteria-often called "staph." Decades ago, strains of staph emerged in hospitals that were resistant to the broad-spectrum antibiotics commonly used to treat them. These antibiotics include methicillin and other more common antibiotics such as oxacillin, penicillin, and amoxicillin. Dubbed methicillin-resistant *Staphylococcus aureus* (MRSA), it was one of the first germs to be resistant to all but the most powerful antibiotics.

Staph bacteria are generally harmless unless they enter the body through a cut or other wound. In older adults and people who are ill or have weakened immune systems, ordinary staph infections can cause serious illness. Staph infections, including MRSA, occur most frequently among persons in hospitals and healthcare facilities, such as nursing homes and dialysis centers, who have weakened immune systems.

In the 1990s, a type of MRSA began appearing in the wider community. Today, that form of staph, known as community-associated MRSA, or CA-MRSA, is responsible for many serious skin and soft tissue infections and for a serious form of pneumonia. When not treated properly, MRSA infection can be fatal.

MRSA infections are spreading rapidly in the United States and worldwide. According to the Center for Disease Control and Prevention (CDC), the proportion of infections that are antimicrobial resistant has been growing. In 1974, MRSA infections accounted for two percent of the total number of staph infections; in 1995 it was 22%; and in 2004 it was nearly 63%. Additionally, recent research has suggested that 30-50% of the population carries MRSA colonies on their bodies all the time, helping to facilitate the spread of infection.

Although MRSA has traditionally been seen as a hospital-associated infection, there has also been an epidemic of CA-MRSA in the United States. MRSA infections in the community are usually manifested as skin infections, such as pimples and boils. These CA-MRSA infections can occur in otherwise healthy people, and commonly occur among athletes who share equipment or personal items including towels and razors. In fact, from 2000 to present, there have been several reported outbreaks of CA-MRSA affecting high school athletic teams. This epidemic among athletes is aided by the fact that MRSA grows very rapidly in warm, moist areas, such as gyms and gym locker rooms. Common cuts and abrasions such as those frequently associated with football and baseball now pose significant threats due to the possibility of a MRSA

infection.

Vancomycin is one of the few antibiotics still effective against hospital strains of MRSA infection, although vancomycin is no longer effective in every case. Several antibacterial agents continue to work against CA-MRSA, but CA-MRSA is a rapidly evolving bacterium, and it may only be a matter of time before it, too, becomes resistant to most antibiotics.

Different procedures of wound management have been developed to help decrease the level of bacteria present in a wound, e.g., reduce the incidence of infection. The cleansing of a wound and the site surrounding the wound to remove blood clots, debris, dirt, or other foreign materials that can introduce contaminants, including pathogenic microorganisms, is critical in reducing levels of bacteria, fungi, viruses, and protozoa in and around the wound. There are numerous wound cleansing procedures presently used by healthcare professionals such as debridement, excision, and irrigation. See, for example, Sinkinson, Craig Alan, ed. (1989) "Maximizing A Wound's Potential For Healing," Emergency Medicine Reports 10(11): 83-89; Lammers, Richard L. (1991) "Soft Tissue Procedures: Principles of Wound Management," in Clinical Procedures in Emergency Medicine, Roberts and Hedges, eds., 2nd Ed., W.B. Saunders Company, pp. 515-521; Cracroft, Davis (1987) "Minor Lacerations and Abrasions," Emergency Medicine: A Comprehensive Review, Kravis and Warner, eds., 2nd cd., Aspen Publishing Co., pp. 107-110; and Mulliken, John B. (1984) "Management of Wounds," in Emergency Medicine, May ed., John Wiley & Sons, pp. 283-286.

Irrigation is the most commonly used procedure for cleansing of open contaminated wounds. Irrigation involves the application of fluids to wounds to remove loose devitalized tissue, microbial inoculum (e.g., bacterial inoculum, fungal inoculum, viral inoculum, protozoal inoculum), blood clots, loose debris, and foreign bodies proximate to and within the depths of the wound. Two critical components of any effective wound irrigation method and/or device are: (1) the application of an adequate volume of sterile irrigation solution to the wound, and (2) the use of sufficient pressure applied in an effective dispersal pattern in the delivery of the solution to effectively remove contaminants. Regarding volume, the amount of irrigation solution required will depend upon the type of wound and the level of contamination. Injuries which can introduce a high amount of bacteria into a wound (such as puncture wounds and bites) may require 1 liter or more of irrigation solution.

U.S. Pat. No. 5,071,104 describes a wound irrigation apparatus and process for cleansing wounds which includes a pressure bladder, e.g., a blood pressure cuff, disposed proximate a reservoir holding a cleaning solution. The device in the '104 patent also includes a flexible tubular conduit for transmitting the solution from the reservoir to a single nozzle. The conduit and reservoir form a two-part system which is time consuming to set up, inconvenient to use, and costly.

U.S. Pat. No. 5,133,701 describes a disposable pressurized wound irrigation device which has a pressurized chamber for providing a force upon the reservoir such that a single liquid stream of cleansing solution is expelled from the device at a constant pressure. A propellant is used in evacuating the cleanser contents of the device. This invention requires a propellant and involves a relatively elaborate manufacturing and filling process which is labor intensive and requires specialized machinery. This device is also inconvenient to use and costly.

More recently, an advantageous wound irrigation system has been developed whereby a dispersed stream of irrigation fluid is easily and effectively applied to wounds. This system is described at, for example, U.S. Pat. Nos. 5,830,197 and 6,468,253 and International Patent Application publications WO 200015279A1 and WO 2002007799A2.

Chlorhexidine is a chemical antiseptic that combats both gram-positive and gram-negative microbes. It is bacteriostatic, hampering the growth of bacteria, and bactericidal, killing bacteria. It is often used as an active ingredient in mouthwash designed to kill dental plaque and other oral bacteria. Chlorhexidine also has non-dental applications. For example, chlorhexidine is used for general skin cleansing, as a surgical scrub, and as a pre-operative skin preparation. US 2022/054734 discloses a wound irrigation solution comprising chlorhexidine and octenidine.

Chlorhexidine is typically used in the form of acetate, gluconate, or hydrochloride. It can be deactivated by anionic compounds, including the anionic surfactants commonly used as detergents in toothpastes and mouthwashes.

## BRIEF SUMMARY OF THE INVENTION

The invention is a non-sterile or sterile wound irrigation solution for use in a method for irrigating a wound comprising the steps of:

(a) providing the non-sterile or sterile wound irrigation solution in a wound irrigation device comprising a reservoir housing, wherein the wound irrigation device comprises a discharge means that directs a pressurized stream of the wound irrigation solution when the reservoir housing is pressurized, and wherein the wound irrigation solution comprises (i) sterile water, purified water, or water for pharmaceutical purposes; (ii) a chlorhexidine at a concentration that is between about 100 μg/mL to about 5 mg/mL; and (iii) octenidine (OCT) or a pharmaceutically acceptable salt thereof at a concentration that is between about 1 μg/mL to about 100 pg/mL;

(b) directing the discharge means and the reservoir housing so as to discharge the wound irrigation solution toward the

wound; and

(c) discharging a stream of the wound irrigation solution from the reservoir housing and through the discharge means directed at the wound.

Described herein are highly effective methods and devices for efficient delivery of two or more medications or other active ingredients to a target site in a patient. Methods for irrigating a wound are described herein. Methods can include the step of providing a sterile wound irrigation solution in a wound irrigation device. Wound irrigation devices can contain a reservoir housing and a discharge means that directs a pressurized stream of a wound irrigation solution when a reservoir housing is pressurized. Methods can include the step of directing the discharge means and the reservoir housing to discharge a wound irrigation solution towards a wound. Methods can further include the step of discharging a stream of a wound irrigation solution from a reservoir housing and through a discharge means directed at a wound.

The wound irrigation solution can have any volume. An amount of wound irrigation solution applied to the wound can be any amount, such as about 10 mL to about 1000 mL of wound irrigation solution. The wound irrigation solution can have any acidity, such as between pH 5.5 and pH 7.0.

The wound irrigation solution can comprise a mixture (e.g., a synergistic combination) of antiseptic agents in solution. A wound irrigation solution can contain a combination of sterile water, a chlorhexidine, and octenidine or a pharmaceutically acceptable salt of octenidine. The concentration of chlorhexidine in the wound irrigation solution can be between about 100 μg/mL to about 5 mg/mL. The concentration of chlorhexidine can be about 200 μg/mL to about 1 mg/mL or about 100 μg/mL to about 500 μg/mL. The concentration of chlorhexidine can be about 500 μg/mL. The chlorhexidine can be any pharmaceutically acceptable salt of chlorhexidine, such as chlorhexidine gluconate or the like. The concentration of a octenidine (or a pharmaceutically acceptable salt of octenidine) in the wound irrigation solution can be between about 1 μg/mL to about 100 μg/mL. For example, the concentration of octenidine (or a pharmaceutically acceptable salt of octenidine) in the wound irrigation solution can be between about 1 μg/mL to about 20 μg/mL. The concentration of octenidine (or a pharmaceutically acceptable salt of octenidine) in the wound irrigation solution can be between about 1 μg/mL to about 5 μg/mL or about 2 μg/mL to about 10 μg/mL. The concentration of octenidine (or a pharmaceutically acceptable salt of octenidine) can be about 1.875 μg/mL or about 3.75 μg/mL. The octenidine (or a pharmaceutically acceptable salt of octenidine) can be any pharmaceutically acceptable salt, such as octenidine dihydrochloride and the like.

Methods described herein can reduce the amount of irrigation time required to reduce a microbial burden (e.g., a fungal burden, a bacterial burden, a viral burden) of a wound (e.g., reduce the time compared to another, alternative method for wound irrigation). The amount of irrigation time required can be any duration of time, such as 1 hour, 45 minutes, 30 minutes, 15 minutes, 10 minutes, 5 minutes, or 1 minute. For example, the irrigation time can be 15 minutes. The amount of time to reduce the microbial burden by any amount, such as 100-fold, can be reduced by practicing the method compared to practicing an analogous method using an alternative irrigation solution. The alternative irrigation solution can be saline (e.g., 0.9% w/w saline without an antiseptic agent). The alternative irrigation solution can be a solution containing chlorhexidine in the same concentration as wound irrigation solutions described herein, but without an additional antiseptic agent (e.g., without octenidine or a pharmaceutically acceptable salt of octenidine). The alternative irrigation solution can be a solution containing octenidine (or a pharmaceutically acceptable salt of octenidine) in the same concentration as wound irrigation solutions described herein, but without an additional antiseptic agent (e.g., without chlorhexidine). The amount of time required to reduce a bacterial burden by methods described herein compared to an analogous method with an alternative irrigation solution can be reduced by about 50%.

The irrigation solutions of methods and devices described herein can contain a combination of a chlorhexidine and octenidine (or a pharmaceutically acceptable salt of octenidine) that is synergistically microbiocidal or microbiostatic for any microorganism (e.g., a fungus, a bacterium). The microorganism can be any described herein, including but not limited to *Candida* species, *E. coli, Staphylococcus* species, *Ralstonia* species, *Pseudomonas* species, or a combination of any of the foregoing. The microorganism can be *E. coli, P. aeruginosa,* or a combination of *E. coli* and *P. aeruginosa.*

Methods described herein can be used to kill any microorganism (e.g., any microorganism described herein). Methods can be used to kill *Candida* species, *E. coli, Staphylococcus* species, *Ralstonia* species, *Pseudomonas* species, or combinations of any of the foregoing. Methods can be used to kill C. *albicans, E. coli,* methicillin-resistant *Staphylococcus aureus* (MRSA), *Staphylococcus aureus, Staphylococcus epidermidis, Ralstonia pickettii, P. aeruginosa,* or a combination thereof.

Methods described herein can be used to irrigate (e.g., treat) any type of wound. The type of wound can be a cut, a scrape, a surgical wound, a puncture, or an abrasion. The wound can be on any subject (e.g., any animal, any mammal). The wound can be on a human, such as a human skin wound.

## BRIEF DESCRIPTION OF THE FIGURES

FIG. 1 shows one embodiment of the device described herein.
FIGs. 2A-2D show embodiments of the ports of a device described herein.
FIG. 3 shows a cross-sectional view of a port of a device described herein.
FIGs. 4A-4C are histology images of wounds in a mouse model of delayed wound healing. FIG. 4A, FIG. 4B, and FIG. 4C are images of hematoxylin and eosin stained wound tissues from three mice following treatment with 0.9% w/w saline solution. The details of the corresponding experiment are described in Example 7.
FIGs. 5A-5C are histology images of wounds in a mouse model of delayed wound healing. FIG. 5A, FIG. 5B, and FIG. 5C are images of hematoxylin and eosin stained wound tissues from three mice following treatment with chlorhexidine gluconate (0.05% w/w) only solution (i.e., a solution without an additional antiseptic agent). The details of the corresponding experiment are described in Example 7.
FIGs. 6A-6C are histology images of wounds in a mouse model of delayed wound healing. FIG. 6A, FIG. 6B, and FIG. 6C are images of hematoxylin and eosin stained wound tissues from three mice following treatment containing chlorhexidine gluconate (0.05% w/w) and octenidine dihydrochoride (3.75 μg/mL). The details of the corresponding experiment are described in Example 7.

## DETAILED DESCRIPTION OF THE INVENTION

Described herein are novel, convenient, inexpensive, and effective drug delivery devices that comprise, in some embodiments, a reservoir housing and a discharge means having a plurality of nozzles for delivering an active agent to a target site. This disclosure also provides methods of use for drug delivery devices described herein.

The devices and methods described herein can make it possible to conveniently and easily apply one or more streams of fluid containing for example, a medicinal agent to, for example, a wound, with the stream having an appropriate volume, pressure, and dispersal pattern.

Devices and methods for irrigating a wound described herein can contain or use an irrigation solution that comprises a chlorhexidine (e.g., chlorhexidine gluconate) and an octenidine (e.g., octenidine dihydrochloride). The chlorhexidine and octenidine (or a pharmaceutically acceptable salt thereof) can be at concentrations to achieve a synergistic antimicrobial (e.g., microbiostatic, microbiocidal) effect on particular microorganisms (e.g., bacterium, fungus, virus, protozoan, any microorganism described herein).

Without wishing to be bound by any one particular mechanism or theory, it is believed that by reducing the contact time of the antiseptic agents (e.g., chlorhexidine gluconate and octenidine) with the skin or exposed tissue of a subject (e.g., human, mammal, animal), potential hypersensitivity and allergic side effects of the antiseptic agent(s) can be reduced or eliminated. Wound irrigation solutions used in methods and devices described herein are able to reduce the contact time with the subject before rinsing with a solution that does not contain an antiseptic agent (e.g., does not contain an octenidine and a chlorhexidine), while maintaining (or expanding the spectrum through synergistic effects) microbial killing, bacteriostatic, or fungistatic capabilities. The inventors discovered that wound irrigation solutions described herein, comprising a mixture of two or more antiseptic agents (e.g., a chlorhexidine and octenidine (or a pharmaceutically acceptable salt thereof)) at an effective concentration, advantageously reduce the required contact time (e.g., irrigation time) of the antiseptic agent for a desired antiseptic effect and can further increase the spectrum of antimicrobial (e.g., antibacterial, antifungal) activity when compared to single antiseptic agent wound irrigation solutions. It was also discovered that chlorhexidine and octenidine (or a pharmaceutically acceptable salt of the foregoing) display a selective synergy against particular microorganisms (e.g., bacteria, fungi, viruses, protozoa, *E. coli)* that commonly infect patients through wounds (e.g., surgical wounds, abrasions, lacerations, puncture wounds, cuts, scrapes). This is despite the fact that many antiseptic agents, for example cationic antiseptic agents (e.g., chlorhexidine and octenidine), were previously believed to share the same mechanism of action (e.g., binding to microbial cell walls and proteins, compromising cell wall integrity). See F. P. Deus, A. Ouanounou, Chlorhexidine in Dentistry: Pharmacology, Uses, and Adverse Effects. Int. Dent. J. (2022) 72(3), 269-277. This synergy between antiseptic agents advantageously allows for a lower concentration of antiseptic agent(s) present in the wound irrigation solution compared to single antiseptic wound irrigation solutions, further decreasing the risk of potential hypersensitivity and allergic side effects of an antiseptic agent.

This disclosure relates to methods for irrigating a wound. Methods described herein can include the step of providing a sterile wound irrigation solution in a wound irrigation device comprising a reservoir housing. The wound irrigation device in methods described herein can contain a discharge means that directs a pressurized stream of the wound irrigation solution when the reservoir housing is pressurized. The wound irrigation solution can contain sterile water and two or more antiseptic agents (e.g., two or more cationic antiseptic agents). The wound irrigation solution can contain sterile water, a chlorhexidine (e.g., chlorhexidine gluconate) and octenidine (OCT) or a pharmaceutically acceptable salt thereof (e.g., octenidine dihydrochloride). The concentration of chlorhexidine can be between about 100 μg/mL to about 5 mg/mL. The concentration of octenidine (OCT) or a pharmaceutically acceptable salt thereof can be between 0.25 μg/mL to about 50 μg/mL. Methods can include the step of directing the discharge means and the reservoir housing so as to discharge a wound irrigation solution towards a wound. Methods can include the step of discharging a stream of a wound

irrigation solution from a reservoir housing and through a discharge means directed at the wound. In some embodiments, the wound irrigation solution consists of sterile water, a chlorhexidine, and a octenidine (OCT) or a pharmaceutically acceptable salt thereof. In some embodiments, the method for irrigating a wound contains the steps of

(a) providing a sterile wound irrigation solution in a wound irrigation device comprising a reservoir housing, wherein the wound irrigation device comprises a discharge means that directs a pressurized stream of the wound irrigation solution when the reservoir housing is pressurized, and wherein the wound irrigation solution comprises sterile water, a chlorhexidine at a concentration that is between about 100 μg/mL to about 5 mg/mL, and a octenidine (OCT) or a pharmaceutically acceptable salt thereof at a concentration that is between about 0.25 μg/mL to about 50 μg/mL;
(b) directing the discharge means and the reservoir housing so as to discharge the wound irrigation solution toward the wound; and
(c) discharging a stream of the wound irrigation solution from the reservoir housing and through the discharge means directed at the wound.

Definitions

Various terms relating to aspects of the description are used throughout the specification and claims. Such terms are to be given their ordinary meaning in the art unless otherwise indicated. Other specifically defined terms are to be construed in a manner consistent with the definitions provided herein.
As used herein, the term "active agents" refers to compounds or other entities that perform a therapeutic and/or diagnostic function. This function may be direct, such as promoting tissue repair or killing pathogenic microorganisms (e.g., bacteria, fungi, viruses, protozoa), or may be indirect by eliciting a physiological response that ultimately results in the desired beneficial result. For example, the active agent can be an antiseptic agent that is microbiostatic (e.g., inhibits the proliferation of a microorganism such as a bacterium, fungus, virus, protozoa, or the like) and/or microbiocidal (e.g., kills a microorganism such as a bacterium, fungus, virus, protozoa, or the like).
As used herein, the term "pharmaceutically acceptable salt" refers to a salt of the compound prepared with relatively nontoxic acids or bases, depending on the particular substituents found on the respective compound. When compounds of the present disclosure contain relatively acidic functionalities, base addition salts can be obtained by contacting the neutral form of such compounds with a sufficient amount of the desired base, either neat or in a suitable solvent (e.g., an inert solvent). Examples of pharmaceutically acceptable base addition salts include sodium, potassium, calcium, ammonium, organic amino, magnesium salt, or a similar salt. When compounds relating to the present disclosure contain relatively basic functionalities, acid addition salts can be obtained by contacting the neutral form of such compounds with a sufficient amount of the desired acid, either neat or in a suitable solvent (e.g., an inert solvent). Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from organic acids like acetic (acetate), propionic, isobutyric, maleic, malonic, benzoic, succinic, suberic, fumaric, lactic, mandelic, pamoic (pamoate), phthalic, benzenesulfonic, p-toluenesulfonic, citric, tartaric, methanesulfonic (mesylate), and the like. Also included are salts of amino acids such as arginate and the like, and salts of organic acids like glucuronic or galacturonic acids and the like. Certain compounds of the present disclosure can contain both basic and acidic functionalities that allow the compounds to be converted into either base or acid addition salts. These salts may be prepared by methods known to those skilled in the art. Other pharmaceutically acceptable salts known to those of skill in the art are suitable for pharmaceutical compositions relating to the present disclosure.
Throughout this disclosure, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.
The articles "a" and "an" are used herein to refer to one or more than one (e.g., to at least one) of the grammatical object of the article. By way of example, "an element" means one element or more than one element.
The term "about" when referring to a measurable value such as an amount, a temporal duration, and the like, is meant to encompass variations of ±20% or less, or in some instances ±15% or less, or in some instances ±10% or less, or in some instances ±5% or less, or in some instances ±1% or less, or in some instances ±0.1% or less, from the specified value, as such variations are appropriate.
The phrase "and/or" as used herein should be understood to mean "either or both" of the elements so conjoined, e.g., elements that are conjunctively present in some cases and disjunctively present in other cases. Multiple elements listed with "and/or" should be construed in the same fashion, e.g., "one or more" of the elements so conjoined. Other elements may optionally be present other than the elements specifically identified by the "and/or" clause, whether related or unrelated to those elements specifically identified. Thus, as a non-limiting example, a reference to "A and/or B," when

used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

The term "effective amount" or a "therapeutically effective amount" as used herein refers to an amount of a compound, or a pharmaceutical composition, described herein which is sufficient to achieve a desired result under the conditions of administration. A skilled clinician can determine appropriate dosing based on a variety of considerations including the severity of the disease, the subject's age, weight, general health, and other considerations. A pharmaceutical composition disclosed herein may be administered to provide an amount of about 0.01 mg to about 1800 mg of a therapeutic agent.

The term "pharmaceutically acceptable excipient" as used herein refers to a non-toxic material that may be formulated with a compound disclosed herein to provide a pharmaceutical composition. Preferably, the pharmaceutically acceptable excipient is inert and does not interfere with the pharmacological activity of a compound which it is formulated with. Pharmaceutically acceptable excipients useful in the manufacture of the pharmaceutical compositions disclosed herein are any of those well known in the art, and include diluents, dispersing agents, granulating agents, surface active agents, emulsifiers, disintegrating agents, binding agents, preservatives, buffering agents, lubricating agents, ion exchangers, salts, electrolytes, waxes, and/or oils. For example, a pharmaceutically acceptable excipient may be alumina, aluminum stearate, lecithin, a serum protein (e.g., human serum albumin), a phosphate, glycine, sorbic acid, potassium sorbate, a glyceride mixture (e.g., saturated vegetable fatty acids), water, protamine sulfate, disodium hydrogen phosphate, potassium hydrogen phosphate, sodium chloride, a zinc salt, colloidal silica, magnesium trisilicate, polyvinyl pyrrolidone, cellulose or a derivative thereof, polyethylene glycol or a derivative thereof (e.g., PEG-300), sodium carboxymethylcellulose, a polyacrylate, a polyethylene-polyoxypropylene-block polymer, wool fat, a cyclodextrin (e.g., CAPTISOL®), dimethylacetamide (DMA), a polysorbate (e.g., a TWEEN®, e.g., TWEEN-20®), ethylenediaminetetraacetic acid (EDTA) or a salt thereof, and any combination thereof.

The term "subject" as used herein refers to any animal, such as any mammal, including but not limited to, humans, non-human primates, rodents, dogs, and the like. Non-human primates include chimpanzees, cynomolgus monkeys, spider monkeys, and macaques (e.g., Rhesus). Rodents include mice, rats, woodchucks, ferrets, rabbits, and hamsters. Domestic and game animals include cows, horses, pigs, deer, bison, buffalo, feline species (e.g., domestic cat), canine species (e.g., dog, fox, wolf), avian species, and fish. In some embodiments, the subject is a mammal (e.g., a human, a rat, or a mouse). The subject can be male or female. The subject may be of any age, including an elderly human subject (e.g., 65 years or older), a human subject that is not elderly (e.g., less than 65 years old), or a human pediatric subject (e.g., 18 years old or less). In preferred aspects, the subject is a human.

As used herein, the terms "treat," "treatment," "treating," or grammatically related terms, refer to a method of reducing the effects of a disease or disorder. As is readily appreciated in the art, full eradication of the disease, disorder, or symptoms thereof is preferred but not a requirement for treatment. Desirable effects of treatment include, but are not limited to, preventing occurrence or recurrence of the disease or disorder, alleviation of symptoms, diminishment of any direct or indirect pathological consequences of the disease or disorder, or other improvement of any sign, symptom, or consequence of the disease or disorder, such as prolonged survival, less morbidity, and/or a lessening of side effects.

Throughout this disclosure, various embodiments can be presented in a range format (e.g., from X - Y). The description of a range should be considered to have specifically disclosed all the possible subranges as well as individual numerical values within that range. For example, description of a range such as from 1 to 6 should be considered to have specifically disclosed subranges such as from 1 to 5, from 1 to 4, from 1 to 3, from 2 to 6, from 2 to 4, from 3 to 6, etc., as well as individual numbers within that range, e.g., 1, 2, 2.8, 3, 3.6, 4, 5, 5.4, and 6. As another example, a range such as 95-99% includes 95%, 96%, 97%, 98%, or 99% and all subranges such as 96-99%, 96-98%, 96-97%, 97-99%, 97-98%, etc. This applies regardless of the breadth of the range.

## Wound Irrigation Solutions

Wound irrigation solutions described herein contain two or more antiseptic agents. Without wishing to be bound by any one particular mechanism of action or theory, it is believed that wound irrigation solutions containing a chlorhexidine and octenidine (or a pharmaceutically acceptable salt of octenidine) have a synergistic antimicrobial (e.g., microbiostatic, fungistatic, bacteriostatic, microbiocidal, fungicidal, bactericidal) effect and/or reduce the contact time (e.g., irrigation time) required for an antimicrobial effect against particular microorganisms (e.g., a microorganism described herein). It is further believed that, through reduction of the contact time (e.g., irrigation time) required to obtain a desired antimicrobial effect in methods described herein, the likelihood of a subject experiencing an adverse reaction (e.g., sensitivity, allergic response, pain) to one or more antiseptics present in the wound irrigation solution can be reduced.

The wound irrigation solution can contain a synergistic combination of two or more antiseptic agents in solution. The wound irrigation solution can be a solution an aqueous solution, including but not limited to, water or salt solution (e.g., saline). The wound irrigation solution can be an emulsion, a biphasic mixture, or a triphasic mixture (e.g., a solution of water

with a non-miscible cosolvent). The wound irrigation solution can be non-sterile, sterile filtered or terminally sterilized (e.g., can contain sterile water). In some embodiments, the irrigation solution is a sterile water (not saline) solution containing 0.05% or less (or even less than 0.04% or even less than 0.03%) of chlorhexidine that is applied to a wound in the skin of a human. Preferably the wound is then rinsed within five minutes (e.g., more preferably within 1-3 minutes) with a sterile saline, water, or other liquid that does not contain an antiseptic agent (e.g., does not contain a chlorhexidine, does not contain octenidine or a pharmaceutically acceptable salt of octenidine).

In some embodiments, the synergistic mixture of antiseptics used according to the methods and devices described herein includes a chlorhexidine and has the chemical structure according to Formula (I):

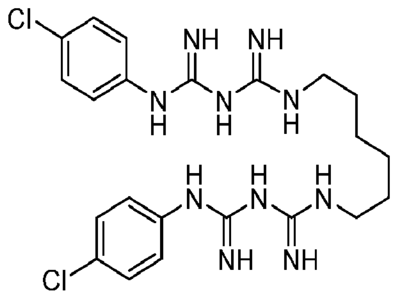

Chlorhexidine

Systematic (IUPAC) Name: 1-[amino-[6-[amino-[amino-(4-chlorophenyl)amino-methylidene]amino-methylidene]aminohexylimino]methyl]imino-N-(4-chlorophenyl)-methanediamine;

Chemical Data

| | |
|---|---|
| Formula: | $C_{22}H_{30}Cl_2N_{10}$ |
| Mol. Weight: | 505.446 g/mol |

The chlorhexidine as used in methods and devices described herein can be in the form of a salt, such as any pharmaceutically acceptable salt of chlorhexidine. The anion (e.g., the counter anion) of the chlorhexidine salt can be any anion including, but not limited to, any carboxylate (e.g., formate, acetate, propionate, isopropionate, butyrate, isobutyrate, maleate, malonate, benzoate, succinate, suberate, fumarate, lactate, mandelate, pamoate, phthalate, citrate, tartrate, gluconate, and the like) or halide (e.g., fluoride, chloride, bromide, iodide). In some embodiments, the synergistic mixture of antiseptics used according to the methods and devices described herein includes a chlorhexidine acetate salt (e.g., chlorhexidine diacetate). In some embodiments, the synergistic mixture of antiseptics used according to the methods and devices described herein includes a chlorhexidine gluconate salt (e.g., a chlorhexidine gluconate salt, chlorhexidine digluconate). In some embodiments, the synergistic mixture of antiseptics used according to the methods and devices described herein includes chlorhexidine digluconate and has chemical structure according to Formula (II):

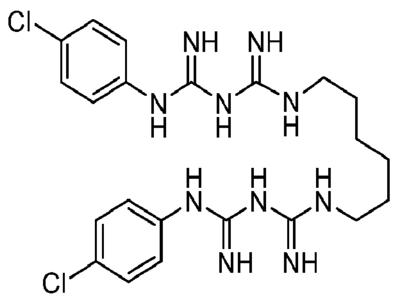

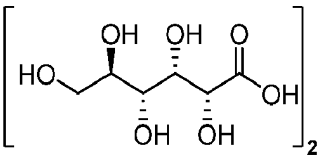

Chlorhexidine digluconate

Systematic (IUPAC) Name: 1-[amino-[6-[amino-[amino-(4-chlorophenyl)amino-methylidene]amino-methylidene]aminohexylimino]methyl]imino-N-(4-chlorophenyl)-methanediamine; (2R,3S,4R,5R)-2,3,4,5,6-pentahydroxyhexanoic acid.

Chemical Data

| | |
|---|---|
| Formula: | $C_{34}H_{54}Cl_2N_{10}O_{14}$ |
| Mol. Weight: | 897.762 g/mol |

In some embodiments, the synergistic mixture of antiseptics used according to the methods and devices described herein includes octenidine (e.g., free base octenidine) and has a chemical structure of Formula (III):

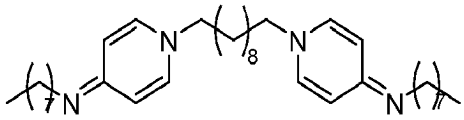
.

Octenidine

Systematic (IUPAC) Name: 1-Octanamine, N,N'-(1,10-decanediyldi-1(4H)-pyridinyl-4- ylidene)bis-

Chemical Data

| | |
|---|---|
| Formula: | $C_{36}H_{62}N_4$ |
| Mol. Weight: | 550.9 g/mol |

In some embodiments, the synergistic mixture of antiseptics used according to methods and devices described herein includes a pharmaceutically acceptable salt of octenidine and has a chemical structure of Formula (IV):

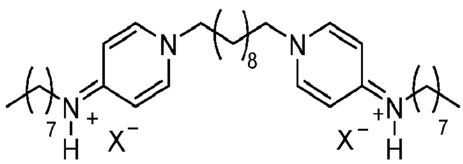
,

wherein X is a counter anion (e.g., any counter anion of a pharmaceutically acceptable salt).

The octenidine salt can be any pharmaceutically acceptable salt. The pharmaceutically acceptable salt of octenidine can be generated by adding acid to free base octenidine. The acid added to free base octenidine could be any acid including but not limited to an organic acid (e.g., formic acid, acetic acid, propionic acid, lactic acid, isopropionic acid, butyric acid, gluconic acid, tartaric acid, maleic acid, carbonic acid), an inorganic acid (e.g., hydrofluoric acid, hydrochloric acid, hydrobromic acid, hydroiodic acid, nitric acid, sulfuric acid, phosphoric acid, perchloric acid), or a combination of the foregoing. The counter anion ($X^-$) of the pharmaceutically acceptable salt of octenidine ( Formula (III)) can be any anion including, but not limited to, a halide, sulfate, mesylate, maleate, citrate, tartrate, phosphate, acetate, or gluconate. The

counter anion ($X^-$) can be any halogen anion including, but not limited to, fluoride ($F^-$), chloride ($Cl^-$), bromide ($Br^-$), or iodide ($I^-$).

The octenidine can be a mixture of pharmaceutically acceptable salts and/or free base octenidine. The mixture of octenidine or a pharmaceutically acceptable salt of octenidine can be a mixture of pharmaceutically acceptable salts of octenidine containing differing counter ions (e.g., $X^-$, counter anions, a mixture containing dihydroiodide and dihydrochloride octenidine salts).The mixture of pharmaceutically acceptable salts of octenidine can contain a number of differing salts that is 2, 3, 4, 5, 6, 7, 8, 9, 10, 11, 12, 13, 14, 15, 16, 17, 18, 19, or 20. The number of differing pharmaceutically acceptable salts of octenidine in a mixture can range from 2 to 3, 2 to 4, 2 to 5, 3 to 4, 3 to 5, or 4 to 5.

In some embodiments, the octenidine or a pharmaceutically acceptable salt of octenidine is octenidine free base. In some embodiments, the octenidine or a pharmaceutically acceptable salt of octenidine is octenidine dihydrochloride.

Any amount of chlorhexidine (e.g., chlorhexidine gluconate) can be applied to a wound in methods described herein. The amount of chlorhexidine applied to a wound can be between about 1 mg to about 750 mg, about 1 mg to about 700 mg, about 1 mg to about 650 mg, about 1 mg to about 600 mg, about 1 mg to about 550 mg, about 1 mg to about 500 mg, about 1 mg to about 450 mg, about 1 mg to about 400 mg, about 1 mg to about 350 mg, about 1 mg to about 300 mg, about 1 mg to about 250 mg, about 1 mg to about 200 mg, about 1 mg to about 150 mg, about 1 mg to about 100 mg, about 1 mg to about 75 mg, about 1 mg to about 50 mg, about 1 mg to about 25 mg, about 5 mg to about 750 mg, about 10 mg to about 750 mg, about 15 mg to about 750 mg, about 20 mg to about 750 mg, about 25 mg to about 750 mg, about 30 mg to about 750 mg, about 35 mg to about 750 mg, about 40 mg to about 750 mg, about 45 mg to about 750 mg, about 50 mg to about 750 mg, about 55 mg to about 750 mg, about 60 mg to about 750 mg, about 65 mg to about 750 mg, about 70 mg to about 750 mg, about 75 mg to about 750 mg, about 80 mg to about 750 mg, about 85 mg to about 750 mg, about 90 mg to about 750 mg, about 95 mg to about 750 mg, about 100 mg to about 750 mg, about 110 mg to about 750 mg, about 120 mg to about 750 mg, about 140 mg to about 750 mg, about 160 mg to about 750 mg, about 180 mg to about 750 mg, about 200 mg to about 750 mg, about 240 mg to about 750 mg, about 280 mg to about 750 mg, about 300 mg to about 750 mg, about 350 mg to about 750 mg, about 400 mg to about 750 mg, about 450 mg to about 750 mg, about 500 mg to about 750 mg, about 550 mg to about 750 mg, about 600 mg to about 750 mg, about 650 mg to about 750 mg, about 700 mg to about 750 mg, about 10 mg to about 30 mg, about 5 mg to about 40 mg, about 5 mg to about 50 mg, about 5 mg to about 60 mg, about 5 mg to about 70 mg, or about 5 mg to about 80 mg. In some embodiments, about 15 to about 25 mg of chlorhexidine gluconate is applied to a wound. In some embodiments, the wound is an abrasion or laceration, and the wound irrigation solution is applied prior to repair/closure.

Any amount of octenidine or a pharmaceutically acceptable salt of octenidine (e.g., octenidine free base, octenidine dihydrochloride) can be applied to a wound in methods described herein. The amount of octenidine or a pharmaceutically acceptable salt of octenidine (e.g., octenidine dihyrdrochloride) applied to a wound can be between about 60 μg to about 40 mg, about 80 μg to about 40 mg, about 100 μg to about 40 mg, about 150 μg to about 40 mg, about 200 μg to about 40 mg, about 250 μg to about 40 mg, about 300 μg to about 40 mg, about 350 μg to about 40 mg, about 400 μg to about 40 mg, about 450 μg to about 40 mg, about 500 μg to about 40 mg, about 600 μg to about 40 mg, about 700 μg to about 40 mg, about 800 μg to about 40 mg, about 900 μg to about 40 mg, about 1 mg to about 40 mg, about 1.2 mg to about 40 mg, about 1.4 mg to about 40 mg, about 1.6 mg to about 40 mg, about 1.8 mg to about 40 mg, about 2 mg to about 40 mg, about 3 mg to about 40 mg, about 4 mg to about 40 mg, about 5 mg to about 40 mg, about 6 mg to about 40 mg, about 7 mg to about 40 mg, about 8 mg to about 40 mg, about 9 mg to about 40 mg, about 10 mg to about 40 mg, about 12 mg to about 40 mg, about 14 mg to about 40 mg, about 16 mg to about 40 mg, about 18 mg to about 40 mg, about 20 mg to about 40 mg, about 24 mg to about 40 mg, about 28 mg to about 40 mg, about 32 mg to about 40 mg, about 36 mg to about 40 mg, about 250 μg to about 15 mg, about 300 μg to about 15 mg, about 350 μg to about 15 mg, about 400 μg to about 15 mg, about 450 μg to about 15 mg, about 500 μg to about 15 mg, about 600 μg to about 15 mg, about 700 μg to about 15 mg, about 800 μg to about 15 mg, about 900 μg to about 15 mg, about 1 mg to about 15 mg, about 1.5 mg to about 15 mg, about 2 mg to about 15 mg, about 2.5 mg to about 15 mg, about 3 mg to about 15 mg, about 3.5 mg to about 15 mg, about 4 mg to about 15 mg, about 4.5 mg to about 15 mg, about 5 mg to about 15 mg, about 6 mg to about 15 mg, about 7 mg to about 15 mg, about 8 mg to about 15 mg, about 9 mg to about 15 mg, about 10 mg to about 15 mg, about 11 mg to about 15 mg, about 12 mg to about 15 mg, about 13 mg to about 15 mg, about 14 mg to about 15 mg, about 250 μg to about 4 mg, about 300 μg to about 4 mg, about 350 μg to about 4 mg, about 400 μg to about 4 mg, about 450 μg to about 4 mg, about 500 μg to about 4 mg, about 600 μg to about 4 mg, about 700 μg to about 4 mg, about 800 μg to about 4 mg, about 900 μg to about 4 mg, about 1 mg to about 4 mg, about 1.5 mg to about 4 mg, about 2 mg to about 4 mg, about 2.5 mg to about 4 mg, about 3 mg to about 4 mg, about 3.5 mg to about 4 mg, about 0.5 mg to about 7.5 mg, about 1 mg to about 7.5 mg, about 1.5 mg to about 7.5 mg, about 2 mg to about 7.5 mg, about 2.5 mg to about 7.5 mg, about 3 mg to about 7.5 mg, about 3.5 mg to about 7.5 mg, about 4 mg to about 7.5 mg, about 4.5 mg to about 7.5 mg, about 5 mg to about 7.5 mg, about 5.5 mg to about 7.5 mg, about 6 mg to about 7.5 mg, about 6.5 mg to about 7.5 mg, about 7 mg to about 7.5 mg, about 1 mg to about 750 mg, about 1 mg to about 700 mg, about 1 mg to about 650 mg, about 1 mg to about 600 mg, about 1 mg to about 550 mg, about 1 mg to about 500 mg, about 1 mg to about 450 mg, about 1 mg to about 400 mg, about 1 mg to about 350 mg, about 1 mg to about 300 mg, about 1 mg to about 250 mg, about 1 mg to about 200 mg, about 1 mg to about 150 mg, about 1 mg to about 100 mg,

about 1 mg to about 75 mg, about 1 mg to about 50 mg, about 1 mg to about 25 mg, about 5 mg to about 750 mg, about 10 mg to about 750 mg, about 15 mg to about 750 mg, about 20 mg to about 750 mg, about 25 mg to about 750 mg, about 30 mg to about 750 mg, about 35 mg to about 750 mg, about 40 mg to about 750 mg, about 45 mg to about 750 mg, about 50 mg to about 750 mg, about 55 mg to about 750 mg, about 60 mg to about 750 mg, about 65 mg to about 750 mg, about 70 mg to about 750 mg, about 75 mg to about 750 mg, about 80 mg to about 750 mg, about 85 mg to about 750 mg, about 90 mg to about 750 mg, about 95 mg to about 750 mg, about 100 mg to about 750 mg, about 110 mg to about 750 mg, about 120 mg to about 750 mg, about 140 mg to about 750 mg, about 160 mg to about 750 mg, about 180 mg to about 750 mg, about 200 mg to about 750 mg, about 240 mg to about 750 mg, about 280 mg to about 750 mg, about 300 mg to about 750 mg, about 350 mg to about 750 mg, about 400 mg to about 750 mg, about 450 mg to about 750 mg, about 500 mg to about 750 mg, about 550 mg to about 750 mg, about 600 mg to about 750 mg, about 650 mg to about 750 mg, about 700 mg to about 750 mg, about 10 mg to about 30 mg, about 5 mg to about 40 mg, about 5 mg to about 50 mg, about 5 mg to about 60 mg, about 5 mg to about 70 mg, or about 5 mg to about 80 mg. In some embodiments, about 15 mg to about 25 mg of octenidine or a pharmaceutically acceptable salt of octenidine (octenidine dihydrochloride) is applied to a wound.

The pH of the wound irrigation solution can be neutral or slightly acidic. The pH can be between about 3.0 to about 7.0, about 3.5 to about 7.0, about 4.0 to about 7.0, about 4.5 to about 7.0, about 5.0 to about 7.0, about 6.0 to about 7.0, about 5.0 to about 6.5, or about 5.0 to about 6.0. In some embodiments, the pH is 5.0 to 7.5. In some embodiments, the pH is 5.5 to 7.0. In some embodiments, the chlorhexidine is applied without a sudsing agent.

Application of the wound irrigation solutions described herein reduce the number of microorganisms at the wound when compared to an untreated wound or a wound treated with an irrigation solution that contains saline and does not contain an antiseptic agent (e.g., a chlorhexidine, octenidine (or a pharmaceutically acceptable salt of octenidine), or a combination thereof). In some embodiments, the application of the wound irrigation solution in methods and devices described herein results in a reduction in the number of microorganisms (e.g., bacteria, fungi, protozoan, virus) at the wound when compared to either an untreated wound or a wound irrigation with saline that does not contain chlorhexidine and octenidine (or a pharmaceutically acceptable salt of octenidine). In some embodiments, the application of the wound irrigation solution in methods and devices described herein results in a reduction in the number of microorganisms (e.g., bacteria, fungi, protozoan, virus) at the wound when compared to either an untreated wound or a wound irrigation with saline that does not contain a synergistic combination of two or more antiseptic agents (e.g., a combination of octenidine and chlorhexidine). In some embodiments, the application of the wound irrigation solution in methods and devices described herein results in a reduction in the number of bacteria at the wound when compared to either an untreated wound or a wound irrigation with saline that does not contain chlorhexidine. In some embodiments, the application of the wound irrigation solution in methods and devices described herein results in a reduction in the number of bacteria at the wound when compared to either an untreated wound or a wound irrigation with saline that does not contain octenidine or a pharmaceutically acceptable salt of octenidine.

Advantageously, the wound irrigation solutions of the methods and devices described herein can be effective in combating infection, even when organic materials (including blood, desired tissue, and/or dirt and debris) are present.

In addition to killing bacteria, the formulations of the used in the methods and devices described herein can also "depathogenize" (e.g., reduce the pathogenicity of) certain bacteria or fungi including, for example, *E. coli* and *Klebsiella aerogenes,* making these bacteria less able to cause infection. Formulations used in the methods and devices described herein can reduce the pathogenicity of any bacteria or fungi, such as the bacteria and fungi described herein.

The drug delivery methods described herein can be used in conjunction with the delivery of an active agent by many of the routes set forth in Table 1. In some embodiments, the delivery route for wound irrigation solutions of devices and methods described herein is buccal, conjunctival, cutaneous, dental, intra-abdominal, intralesional, intraocular, intrathoracic (during surgery), irrigation, nasal, ophthalmic, periodontal, rectal, soft tissue, subcutaneous, topical, and vaginal.

The drug delivery devices and methods described herein can be utilized by trained medical technicians; however, because of the simplicity and convenience of the devices and methods described herein, they can be used to greatly enhance the effectiveness of drug delivery regardless of the training level of the operator performing the irrigation.

Delivering Active Agents

Examples of agents that can be administered to a subject (e.g., a human, a human patient) in accordance with the methods and devices described herein include, but are not limited to, bacterial agents, anti-viral agents, fungicidal agents, chemotherapy agents, topical antiseptics, anesthetic agents, oxygenated fluids and/or agents, antibiotics, diagnostic agents, homeopathic agents, and over the counter medications/agents. In some embodiments, the wound irrigation solution administered to a subject comprises sterile water, a chlorhexidine and octenidine or a pharmaceutically acceptable salt of octenidine.

The target sites to which an active ingredient can be administered according to the methods and devices described herein include, but are not limited to, wounds, the eyes, and surgical sites. The surgical sites may include, for

example, joint replacements, abdominal surgery, and oral/periodontal surgery sites. In each case, the ability to deliver the active agent to a specific site, at an appropriate dosage, at a carefully controlled pressure, is unique and highly advantageous.

The solution (e.g., solvent) that carries the active agent can be, for example, water, saline, or a balanced salt solution. The solution that carries the active agent can be sterile (e.g., a sterile aqueous solution of active agent). The device can be sterilized by known sterilization techniques, including boiling, autoclaving, gas sterilization, sterile filtration, and the like, either separately or together with the reservoir housing.

Buffered Ringer's solution or commercially available balanced salt solution (e.g., Tis-U-Sol® (Baxter International Inc.) or PhysioSol™ (ICU Medical, Inc.)) are physiologically compatible and are commonly used in wound irrigation procedures.

The antiseptic agents most commonly used in wound care at present include:
Povidone-iodine solution (Betadine preparation)-iodine added to the carrier polyvinylpyrrolidone (PVP), a water-soluble organic complex; this combination is called an iodophor. Standard solutions of Betadine preparation are 10 percent.

Povidone-iodine surgical scrub (Betadine scrub)-the iodophor PVP-J and an anionic detergent (pH 4.5).

pHisoHex (Sanofi S. A.)-an emulsion of an anionic detergent, entsulfon, lanolin cholesterols, petrolatum, and hexachlorophene (pH 5.5).

Hi-Bi-clens (Mölnlycke Health Care)-chlorhexidine gluconate plus a sudsing base (pH 5.1 to 6.5).

Tincture of green soap-potassium oleate, isopropanol, potassium coconut oil, soap.

Dakin's solution 0.2 percent solution hypochlorite solution.

Hydrogen peroxide-an oxidizing agent.

Nonionic surfactants-Pluronic F-68 (e.g., Shur-Clens® (ConvaTec Inc.)) and Poloxamer-188 (e.g., Pharma-Clens® (Ecolab Inc.))-agents that have no antimicrobial activity (pH 7.1).

The use of chlorhexidine is particularly advantageous because it is broad spectrum, binds to the skin (to provide residual activity), works rapidly and, when used according to the methods described herein, is non-toxic.

Chlorhexidine is a chemical antiseptic, which can be used to combat both gram-positive and gram-negative microbes. It is both bacteriostatic and bactericidal. Various species of bacteria are involved in the pathogenesis of wound infection and/or secondarily cellulitis. At times, these infections can result in disfigurement, loss of extremities, prolonged convalesces, and/or death. The therapeutic effects of wound irrigation solutions used in the devices and methods described herein are to combat microbes typically involved in the pathology of these infections by their antiseptic properties and those associated with the irrigation process itself. Controlling the microbial load in wounds is a vital factor in minimizing infection and thus decreasing and/or preventing disease.

Spectrum of Activity

Chlorhexidine is active against aerobic and anaerobic gram-positive and gram-negative bacteria. The drug also has some activity against *Chlamydia trachomatis,* certain fungi, and certain viruses.

As the use of broad-spectrum antibiotics has increased in prevalence, many emerging bacteria and fungal species of concern are evolving resistance to conventional antibiotic treatments. Emerging bacteria of concern include, but are not limited to, *Burkholderia* species (e.g., *Burkholderia mallei, Burkholderia pseudomallei, Burkholderia glumae), Bordetella* species (e.g., *Bordetella pertussis, Bordetella parapertussis, Bordetella bronchispetica), Campylobacter* species (e.g., drug-resistant *Campylobacter spp., C. jejuni, C. concisus, C. lari), Enterobacterales* species (e.g., extended-spectrum beta-lactamase-producing *Enterobacterales spp.,* carbapenem-resistant *Enterobacterales spp., E. coli, Klebsiella pneumoniae, K. oxytoca, Kluyvera spp., Proteus mirabilis, Serratia marcescens,* Drug-resistant *Shigella spp., Shigella boydii, Shigella dysenteriae, Shigella flexneri, Shigella sonnei, Enterobacter aerogenes, Enterobacter cloacae, Enterobacter sakasakii, Citrobacter koseri, Citrobacter braakii, Citrobacter freundii, Salmonella enterica, Salmonella bongori, Morganella morganii, Providencia rettgeri, Raoultella planticola), Acinetobacter* species (e.g., carbapenem-resistant *Acinetobacter spp., A. baumannii, A. haemolyticus, A. nosocomialis, A. pittii, A. calcoaceticus, A. ursingii, A. variabilis, A. soli, A. seifertii), Clostridioides difficile, Mycoplasma* species (e.g., *Mycoplasma pneumoniae, Mycoplasma genitalium, Mycoplasma amphoriforme, Mycoplasma buccale, Mycoplasma fermentans, Mycoplasma hominis), Neisseria* species (e.g., drug-resistant *Neisseria gonorrhoeae), Enterococci* species (e.g., vancomycin-resistant *Enterococci spp., E. faecalis, E. faecium, E. duran, E. avium, E. gallinarum, E. casseliflavus, E. hirae, E. mundtii, E. raffinosus), Pseudomonas* species (e.g., drug-resistant *Pseudomonas spp., P. aeruginosa), Ralstonia* species (e.g., *Ralstonia pickettii, Ralstonia insidiosa, Ralstonia mannitolilytica, Ralstonia pseudosolanacearum, Ralstonia solanacearum, Ralstonia syzygii), Streptococcus* species (e.g., drug-resistant *Streptococcus spp., Streptococcus pneumoniae, Streptococcus pyogenes, Streptococcus sanguinis, Streptococcus mutans, Streptococcus agalactiae,* erythromycin-resistant group A *Streptococcus spp.,* Clindamycin-resistant group B *Streptococcus spp.)* and combinations thereof. Emerging fungi of concern include, but are not limited to, *Candida* species (e.g., drug-resistant *Candida spp., Candida albicans, Candida glabrata, Candida krusei, Candida parapsilosis, Candida tropicalis, Candida auris),* Aspergillus species

(e.g., azole-resistant *Aspergillus spp., Aspergillus fumigatus, Aspergillus flavus, Aspergillus hiratsukae, Aspergillus niger, Aspergillus terreus), Fusarium* species (e.g., *Fusarium oxysporum, Fusarium solani), Mucor* species (e.g., *Mucor circinelloides), Rhizopus* species (e.g., *Rhizopus arrhizus),* and combinations thereof.

The methods and devices disclosed herein can be used to inhibit or kill any microbial organism (e.g., any bacteria, fungi, protozoa, virus). The methods and devices disclosed herein can use a wound irrigation solution comprising two or more antiseptic agents (e.g., a chlorhexidine and octenidine or a pharmaceutically acceptable salt of octenidine) to inhibit or kill any bacteria including, but not limited to, *Acinetobacter spp., Bordetella spp.,* Burkholderia spp., *Campylobacter* spp., *Clostridioides difficile, Cutibacterium spp.* (e.g., *Cutibacterium acnes, Cutibacterium avidum, Cutibacterium granulosum, Cutibacterium modestum, Cutibacterium namnetense, Cutibacterium porci), Lactobacillus* spp. *(L. rhamnosus, L. acidophilus, L. casei, L. fermentum, L. paracasei, L. corneformis), Enterobacterales spp., Enterococci spp., Mycoplasma spp., Neisseria spp., Pseudomonas spp., Ralstonia spp., Staphylococcus spp., Streptococcus spp.,* or combinations thereof. The methods and devices disclosed herein can be used to inhibit or kill any bacteria described herein. The methods and devices disclosed herein can be used to inhibit or kill any fungi including, but not limited to, *Candida spp., Aspergillus spp., Fusarium spp., Mucor spp., Rhizopus spp.,* or combinations thereof. The methods and devices disclosed herein can be used to inhibit or kill any fungi described herein. In some embodiments, the methods described herein are used to kill *Candida* species, *E. coli, Staphylococcus* species, *Ralstonia* species, *Pseudomonas* species, or a combination thereof. In some embodiments, the methods described herein are used to kill C. *albicans, E. coli,* methicillin-resistant *Staphylococcus aureus, Staphylococcus aureus, Staphylococcus epidermidis, Ralstonia pickettii, P. aeruginosa,* or a combination thereof. In some embodiments, the methods described herein are used to kill C. *albicans.* In some embodiments, the methods described herein are used to kill Cutibacterium spp. (e.g., *Cutibacterium acnes).* In some embodiments, the methods described herein are used to kill Lactobacillus spp. (L. rhamnosus). In some embodiments, the methods described herein are used to kill *E. coli.* In some embodiments, the methods described herein are used to kill *Staphylococcus aureus* (e.g., methicillin-resistant *Staphylococcus aureus,* MRSA). In some embodiments, the methods described herein are used to kill *Staphylococcus epidermidis.* In some embodiments, the methods described herein are used to kill *Ralstonia pickettii.* In some embodiments, the methods described herein are used to kill *P. aeruginosa.* In some embodiments, the methods described herein are used to kill *Candida sp.* In some embodiments, the methods described herein are used to kill *Escherichia sp.* In some embodiments, the methods described herein are used to kill *Staphylococcus sp.* In some embodiments, the methods described herein are used to kill *Ralstonia sp.* In some embodiments, the methods described herein are used to kill *Pseudomonas sp.*

### Aerobic Bacteria

Chlorhexidine is highly active against a variety of gram-positive aerobic bacteria, including *Streptococcus mutans, S. pyogenes* (group A β-hemolytic streptococci), S. *salivarius,* and *S. sanguis.* Chlorhexidine is active against *Staphylococcus aureus, S. epidermidis, S. haemolyticus, S. haominis,* and *S. simulans.* Chlorhexidine is active against both oxacillin-resistant (ORSA) and oxacillin-susceptible staphylococci (also known as methicillin-resistant [MRSA] or methicillin-susceptible staphylococci).

Chlorhexidine is active against Enterococcus, including *E. faecalis* and *E. faecium,* and is active against both vancomycin-susceptible and vancomycin-resistant strains.

### Anaerobic Bacteria

Chlorhexidine is active against some anaerobic bacteria. It is active against some strains of *Bacteroides, Propionibacterium, Clostridium difficile,* and *Selenomonas,* but is less active against *Veillonella.*

### Fungi

Chlorhexidine has some activity against *Candida albicans, C. dubliniensis, C. glabrata* (formerly *Torulopsis glabrata), C. guillermondii, C. kefyr* (formerly C. *pseudotropicalis), C. krusei, C. lusitaniae,* and C. *tropicalis* (formerly C. *parapsilosis).* Chlorhexidine also has some activity against dermatophytes, including *Epidermophyton floccosum, Microsporum gypseum, M. canis,* and *Trichophyton mentagrophytes.*

### Viruses

Chlorhexidine has antiviral activity against viruses that have a lipid component in their outer coat or have an outer envelope such as cytomegalovirus (CMV), human immunodeficiency virus (HIV), herpes simplex virus types 1 (HSV-1) and 2 (HSV-2), influenza virus, parainfluenza virus, coronavirus (e.g., SARS, SARS-CoV-2) and variola virus (smallpox virus). For example, *see* Y. H. Huang and J. T. Huang, Use of chlorhexidine to eradicate oropharyngeal SARS-CoV-2 in

COVID-19 patients, J. Med. Virol. 2021; 93 (7), 4370-4373.

Methods and Formulations

Advantageously, because the methods described herein can be used to accurately and efficiently deliver a combination of two or more antiseptic agents (e.g., a synergistic combination of two or more antiseptic agents) to a target site in a subject (e.g., a human, a human patient), it is possible, in certain embodiments, to utilize reduced concentrations of one or more antiseptic agent. In some embodiments, a low concentration solution of chlorhexidine can be used to effectively reduce infections and/or microbial burden (e.g., fungal burden, bacterial burden, viral burden, protozoal burden) at, for example, a wound, surgical site, or other tissue opening.

Wound irrigation solutions of methods and devices described herein can contain any concentration of an antiseptic agent. The concentration of the antiseptic agent can be between about 5 μg/mL to about 1 mg/mL, about 6 μg/mL to about 1 mg/mL, about 7 μg/mL to about 1 mg/mL, about 8 μg/mL to about 1 mg/mL, about 9 μg/mL to about 1 mg/mL, about 10 μg/mL to about 1 mg/mL, about 12 μg/mL to about 1 mg/mL, about 14 μg/mL to about 1 mg/mL, about 16 μg/mL to about 1 mg/mL, about 18 μg/mL to about 1 mg/mL, about 20 μg/mL to about 1 mg/mL, about 24 μg/mL to about 1 mg/mL, about 28 μg/mL to about 1 mg/mL, about 30 μg/mL to about 1 mg/mL, about 35 μg/mL to about 1 mg/mL, about 40 μg/mL to about 1 mg/mL, about 45 μg/mL to about 1 mg/mL, about 50 μg/mL to about 1 mg/mL, about 55 μg/mL to about 1 mg/mL, about 60 μg/mL to about 1 mg/mL, about 65 μg/mL to about 1 mg/mL, about 70 μg/mL to about 1 mg/mL, about 75 μg/mL to about 1 mg/mL, about 80 μg/mL to about 1 mg/mL, about 85 μg/mL to about 1 mg/mL, about 90 μg/mL to about 1 mg/mL, about 95 μg/mL to about 1 mg/mL, about 100 μg/mL to about 1 mg/mL, about 120 μg/mL to about 1 mg/mL, about 140 μg/mL to about 1 mg/mL, about 160 μg/mL to about 1 mg/mL, about 180 μg/mL to about 1 mg/mL, about 200 μg/mL to about 1 mg/mL, about 250 μg/mL to about 1 mg/mL, about 300 μg/mL to about 1 mg/mL, about 350 μg/mL to about 1 mg/mL, about 400 μg/mL to about 1 mg/mL, about 450 μg/mL to about 1 mg/mL, about 500 μg/mL to about 1 mg/mL, about 550 μg/mL to about 1 mg/mL, about 600 μg/mL to about 1 mg/mL, about 650 μg/mL to about 1 mg/mL, about 700 μg/mL to about 1 mg/mL, about 750 μg/mL to about 1 mg/mL, about 800 μg/mL to about 1 mg/mL, about 850 μg/mL to about 1 mg/mL, about 900 μg/mL to about 1 mg/mL, about 950 μg/mL to about 1 mg/mL, about 5 μg/mL to about 950 μg/mL, about 5 μg/mL to about 900 μg/mL, about 5 μg/mL to about 850 μg/mL, about 5 μg/mL to about 800 μg/mL, about 5 μg/mL to about 750 μg/mL, about 5 μg/mL to about 700 μg/mL, about 5 μg/mL to about 650 μg/mL, about 5 μg/mL to about 600 μg/mL, about 5 μg/mL to about 550 μg/mL, about 5 μg/mL to about 500 μg/mL, about 5 μg/mL to about 450 μg/mL, about 5 μg/mL to about 400 μg/mL, about 5 μg/mL to about 375 μg/mL, about 5 μg/mL to about 350 μg/mL, about 5 μg/mL to about 325 μg/mL, about 5 μg/mL to about 300 μg/mL, about 5 μg/mL to about 275 μg/mL, about 5 μg/mL to about 250 μg/mL, about 5 μg/mL to about 225 μg/mL, about 5 μg/mL to about 200 μg/mL, about 5 μg/mL to about 175 μg/mL, about 5 μg/mL to about 150 μg/mL, about 5 μg/mL to about 125 μg/mL, about 5 μg/mL to about 100 μg/mL, about 5 μg/mL to about 75 μg/mL, about 5 μg/mL to about 50 μg/mL, about 5 μg/mL to about 25 μg/mL, about 5 μg/mL to about 20 μg/mL, about 5 μg/mL to about 15 μg/mL, about 5 μg/mL to about 10 μg/mL, about 10 μg/mL to about 500 μg/mL, about 15 μg/mL to about 500 μg/mL, about 20 μg/mL to about 500 μg/mL, about 25 μg/mL to about 500 μg/mL, about 30 μg/mL to about 500 μg/mL, about 35 μg/mL to about 500 μg/mL, about 40 μg/mL to about 500 μg/mL, about 45 μg/mL to about 500 μg/mL, about 50 μg/mL to about 500 μg/mL, about 55 μg/mL to about 500 μg/mL, about 60 μg/mL to about 500 μg/mL, about 65 μg/mL to about 500 μg/mL, about 70 μg/mL to about 500 μg/mL, about 75 μg/mL to about 500 μg/mL, about 80 μg/mL to about 500 μg/mL, about 85 μg/mL to about 500 μg/mL, about 90 μg/mL to about 500 μg/mL, about 100 μg/mL to about 500 μg/mL, about 110 μg/mL to about 500 μg/mL, about 120 μg/mL to about 500 μg/mL, about 130 μg/mL to about 500 μg/mL, about 140 μg/mL to about 500 μg/mL, about 150 μg/mL to about 500 μg/mL, about 160 μg/mL to about 500 μg/mL, about 170 μg/mL to about 500 μg/mL, about 180 μg/mL to about 500 μg/mL, about 190 μg/mL to about 500 μg/mL, about 200 μg/mL to about 500 μg/mL, about 240 μg/mL to about 500 μg/mL, about 280 μg/mL to about 500 μg/mL, about 300 μg/mL to about 500 μg/mL, about 350 μg/mL to about 500 μg/mL, about 400 μg/mL to about 500 μg/mL, about 450 μg/mL to about 500 μg/mL, about 10 μg/mL to about 450 μg/mL, about 10 μg/mL to about 400 μg/mL, about 10 μg/mL to about 375 μg/mL, about 10 μg/mL to about 350 μg/mL, about 10 μg/mL to about 325 μg/mL, about 10 μg/mL to about 300 μg/mL, about 10 μg/mL to about 275 μg/mL, about 10 μg/mL to about 250 μg/mL, about 10 μg/mL to about 225 μg/mL, about 10 μg/mL to about 200 μg/mL, about 10 μg/mL to about 175 μg/mL, about 10 μg/mL to about 150 μg/mL, about 10 μg/mL to about 125 μg/mL, about 10 μg/mL to about 100 μg/mL, about 10 μg/mL to about 90 μg/mL, about 10 μg/mL to about 80 μg/mL, about 10 μg/mL to about 70 μg/mL, about 10 μg/mL to about 60 μg/mL, about 10 μg/mL to about 50 μg/mL, about 10 μg/mL to about 45 μg/mL, about 10 μg/mL to about 40 μg/mL, about 10 μg/mL to about 35 μg/mL, about 10 μg/mL to about 30 μg/mL, about 10 μg/mL to about 25 μg/mL, about 10 μg/mL to about 20 μg/mL, about 10 μg/mL to about 15 μg/mL, about 30 μg/mL to about 200 μg/mL, about 35 μg/mL to about 200 μg/mL, about 40 μg/mL to about 200 μg/mL, about 45 μg/mL to about 200 μg/mL, about 50 μg/mL to about 200 μg/mL, about 55 μg/mL to about 200 μg/mL, about 60 μg/mL to about 200 μg/mL, about 65 μg/mL to about 200 μg/mL, about 70 μg/mL to about 200 μg/mL, about 75 μg/mL to about 200 μg/mL, about 80 μg/mL to about 200 μg/mL, about 85 μg/mL to about 200 μg/mL, about 90 μg/mL to about 200 μg/mL, about 95 μg/mL to about 200 μg/mL, about 100 μg/mL to about 200 μg/mL, about 110 μg/mL to about 200 μg/mL, about 120 μg/mL

to about 200 μg/mL, about 130 μg/mL to about 200 μg/mL, about 140 μg/mL to about 200 μg/mL, about 150 μg/mL to about 200 μg/mL, about 160 μg/mL to about 200 μg/mL, about 170 μg/mL to about 200 μg/mL, about 180 μg/mL to about 200 μg/mL, about 190 μg/mL to about 200 μg/mL, about 30 μg/mL to about 190 μg/mL, about 30 μg/mL to about 180 μg/mL, about 30 μg/mL to about 170 μg/mL, about 30 μg/mL to about 160 μg/mL, about 30 μg/mL to about 150 μg/mL, about 30 μg/mL to about 140 μg/mL, about 30 μg/mL to about 130 μg/mL, about 30 μg/mL to about 120 μg/mL, about 30 μg/mL to about 110 μg/mL, about 30 μg/mL to about 100 μg/mL, about 30 μg/mL to about 95 μg/mL, about 30 μg/mL to about 90 μg/mL, about 30 μg/mL to about 85 μg/mL, about 30 μg/mL to about 80 μg/mL, about 30 μg/mL to about 75 μg/mL, about 30 μg/mL to about 70 μg/mL, about 30 μg/mL to about 65 μg/mL, about 30 μg/mL to about 60 μg/mL, about 30 μg/mL to about 55 μg/mL, about 30 μg/mL to about 50 μg/mL, about 30 μg/mL to about 45 μg/mL, about 30 μg/mL to about 40 μg/mL, about 60 μg/mL to about 180 μg/mL, about 65 μg/mL to about 180 μg/mL, about 70 μg/mL to about 180 μg/mL, about 75 μg/mL to about 180 μg/mL, about 80 μg/mL to about 180 μg/mL, about 85 μg/mL to about 180 μg/mL, about 90 μg/mL to about 180 μg/mL, about 95 μg/mL to about 180 μg/mL, about 100 μg/mL to about 180 μg/mL, about 105 μg/mL to about 180 μg/mL, about 110 μg/mL to about 180 μg/mL, about 115 μg/mL to about 180 μg/mL, about 120 μg/mL to about 180 μg/mL, about 125 μg/mL to about 180 μg/mL, about 130 μg/mL to about 180 μg/mL, about 135 μg/mL to about 180 μg/mL, about 140 μg/mL to about 180 μg/mL, about 145 μg/mL to about 180 μg/mL, about 150 μg/mL to about 180 μg/mL, about 155 μg/mL to about 180 μg/mL, about 160 μg/mL to about 180 μg/mL, about 165 μg/mL to about 180 μg/mL, about 170 μg/mL to about 180 μg/mL, about 175 μg/mL to about 180 μg/mL, about 60 μg/mL to about 175 μg/mL, about 60 μg/mL to about 170 μg/mL, about 60 μg/mL to about 165 μg/mL, about 60 μg/mL to about 160 μg/mL, about 60 μg/mL to about 155 μg/mL, about 60 μg/mL to about 150 μg/mL, about 60 μg/mL to about 145 μg/mL, about 60 μg/mL to about 140 μg/mL, about 60 μg/mL to about 135 μg/mL, about 60 μg/mL to about 130 μg/mL, about 60 μg/mL to about 125 μg/mL, about 60 μg/mL to about 120 μg/mL, about 60 μg/mL to about 115 μg/mL, about 60 μg/mL to about 110 μg/mL, about 60 μg/mL to about 105 μg/mL, about 60 μg/mL to about 100 μg/mL, about 60 μg/mL to about 95 μg/mL, about 60 μg/mL to about 90 μg/mL, about 60 μg/mL to about 85 μg/mL, about 60 μg/mL to about 80 μg/mL, about 60 μg/mL to about 75 μg/mL, about 60 μg/mL to about 70 μg/mL, about 60 μg/mL to about 65 μg/mL, about 35 μg/mL to about 90 μg/mL, about 40 μg/mL to about 90 μg/mL, about 45 μg/mL to about 90 μg/mL, about 50 μg/mL to about 90 μg/mL, about 55 μg/mL to about 90 μg/mL, about 60 μg/mL to about 90 μg/mL, about 65 μg/mL to about 90 μg/mL, about 70 μg/mL to about 90 μg/mL, about 75 μg/mL to about 90 μg/mL, about 80 μg/mL to about 90 μg/mL, or about 85 μg/mL to about 90 μg/mL. The concentration of the antiseptic agent can be about 5 μg/mL, about 10 μg/mL, about 15 μg/mL, about 20 μg/mL, about 25 μg/mL, about 30 μg/mL, about 35 μg/mL, about 40 μg/mL, about 45 μg/mL, about 50 μg/mL, about 55 μg/mL, about 60 μg/mL, about 65 μg/mL, about 70 μg/mL, about 75 μg/mL, about 80 μg/mL, about 85 μg/mL, about 90 μg/mL, about 95 μg/mL, about 100 μg/mL, about 110 μg/mL, about 120 μg/mL, about 130 μg/mL, about 140 μg/mL, about 150 μg/mL, about 160 μg/mL, about 170 μg/mL, about 180 μg/mL, about 190 μg/mL, about 200 μg/mL, about 220 μg/mL, about 240 μg/mL, about 260 μg/mL, about 280 μg/mL, about 300 μg/mL, about 330 μg/mL, about 360 μg/mL, about 390 μg/mL, about 400 μg/mL, about 440 μg/mL, about 480 μg/mL, about 500 μg/mL, about 550 μg/mL, about 600 μg/mL, about 650 μg/mL, about 700 μg/mL, about 750 μg/mL, about 800 μg/mL, about 850 μg/mL, about 900 μg/mL, about 950 μg/mL, or about 1 mg/mL.

Wound irrigation solutions used in the methods and devices described herein can contain any concentration of a chlorhexidine (e.g., chlorhexidine gluconate). The concentration of the chlorhexidine can be between about 50 μg/mL to about 100 mg/mL, about 50 μg/mL to about 90 mg/mL, about 50 μg/mL to about 80 mg/mL, about 50 μg/mL to about 70 mg/mL, about 50 μg/mL to about 60 mg/mL, about 50 μg/mL to about 50 mg/mL, about 50 μg/mL to about 40 mg/mL, about 50 μg/mL to about 30 mg/mL, about 50 μg/mL to about 20 mg/mL, about 50 μg/mL to about 100 mg/mL, about 100 μg/mL to about 100 mg/mL, about 150 μg/mL to about 100 mg/mL, about 200 μg/mL to about 100 mg/mL, about 300 μg/mL to about 100 mg/mL, about 400 μg/mL to about 100 mg/mL, about 500 μg/mL to about 100 mg/mL, about 50 μg/mL to about 10 mg/mL, about 50 μg/mL to about 5 mg/mL, about 50 μg/mL to about 2.5 mg/mL, about 50 μg/mL to about 2 mg/mL, about 50 μg/mL to about 1 mg/mL, about 50 μg/mL to about 750 μg /mL, about 50 μg/mL to about 500 μg /mL, about 50 μg/mL to about 400 μg /mL, about 50 μg/mL to about 300 μg /mL, about 50 μg/mL to about 200 μg /mL, about 50 μg/mL to about 100 μg /mL, about 50 μg/mL to about 90 μg /mL, about 50 μg/mL to about 80 μg /mL, about 50 μg/mL to about 60 μg /mL, about 60 μg/mL to about 10 mg/mL, about 80 μg/mL to about 10 mg/mL, about 100 μg/mL to about 10 mg/mL, about 150 μg/mL to about 10 mg/mL, about 200 μg/mL to about 10 mg/mL, about 250 μg/mL to about 10 mg/mL, about 300 μg/mL to about 10 mg/mL, about 350 μg/mL to about 10 mg/mL, about 400 μg/mL to about 10 mg/mL, about 450 μg/mL to about 10 mg/mL, about 500 μg/mL to about 10 mg/mL, about 550 μg/mL to about 10 mg/mL, about 600 μg/mL to about 10 mg/mL, about 650 μg/mL to about 10 mg/mL, about 700 μg/mL to about 10 mg/mL, about 750 μg/mL to about 10 mg/mL, about 800 μg/mL to about 10 mg/mL, about 900 μg/mL to about 10 mg/mL, about 1 mg/mL to about 10 mg/mL, about 2 mg/mL to about 10 mg/mL, about 3 mg/mL to about 10 mg/mL, about 4 mg/mL to about 10 mg/mL, about 5 mg/mL to about 10 mg/mL, about 6 mg/mL to about 10 mg/mL, about 7 mg/mL to about 10 mg/mL, about 8 mg/mL to about 10 mg/mL, about 9 mg/mL to about 10 mg/mL, about 100 μg/mL to about 5 mg/mL, about 150 μg/mL to about 5 mg/mL, about 200 μg/mL to about 5 mg/mL, about 250 μg/mL to about 5 mg/mL, about 300 μg/mL to about 5 mg/mL, about 350 μg/mL to about 5 mg/mL, about 400 μg/mL to about 5 mg/mL, about 450 μg/mL to about 5 mg/mL, about 500 μg/mL to about 5 mg/mL, about

550 µg/mL to about 5 mg/mL, about 600 µg/mL to about 5 mg/mL, about 650 µg/mL to about 5 mg/mL, about 700 µg/mL to about 5 mg/mL, about 750 µg/mL to about 5 mg/mL, about 800 µg/mL to about 5 mg/mL, about 850 µg/mL to about 5 mg/mL, about 900 µg/mL to about 5 mg/mL, about 950 µg/mL to about 5 mg/mL, about 1 mg/mL to about 5 mg/mL, about 2 mg/mL to about 5 mg/mL, about 3 mg/mL to about 5 mg/mL, about 4 mg/mL to about 5 mg/mL, about 100 µg/mL to about 4 mg/mL, about 100 µg/mL to about 3 mg/mL, about 100 µg/mL to about 2 mg/mL, about 100 µg/mL to about 1 mg/mL, about 100 µg/mL to about 950 µg/mL, about 100 µg/mL to about 900 µg/mL, about 100 µg/mL to about 850 µg/mL, about 100 µg/mL to about 800 µg/mL, about 100 µg/mL to about 750 µg/mL, about 100 µg/mL to about 700 µg/mL, about 100 µg/mL to about 650 µg/mL, about 100 µg/mL to about 600 µg/mL, about 100 µg/mL to about 550 µg/mL, about 100 µg/mL to about 500 µg/mL, about 100 µg/mL to about 450 µg/mL, about 100 µg/mL to about 400 µg/mL, about 100 µg/mL to about 350 µg/mL, about 100 µg/mL to about 300 µg/mL, about 100 µg/mL to about 250 µg/mL, about 100 µg/mL to about 200 µg/mL, about 100 µg/mL to about 175 µg/mL, about 100 µg/mL to about 150 µg/mL, about 100 µg/mL to about 125 µg/mL, about 200 µg/mL to about 1 mg/mL, 250 µg/mL to about 1 mg/mL, 300 µg/mL to about 1 mg/mL, 350 µg/mL to about 1 mg/mL, 400 µg/mL to about 1 mg/mL, 450 µg/mL to about 1 mg/mL, 500 µg/mL to about 1 mg/mL, 550 µg/mL to about 1 mg/mL, 600 µg/mL to about 1 mg/mL, 650 µg/mL to about 1 mg/mL, 700 µg/mL to about 1 mg/mL, 750 µg/mL to about 1 mg/mL, 800 µg/mL to about 1 mg/mL, 850 µg/mL to about 1 mg/mL, 900 µg/mL to about 1 mg/mL, 950 µg/mL to about 1 mg/mL, about 200 µg/mL to about 950 µg/mL, about 200 µg/mL to about 900 µg/mL, about 200 µg/mL to about 850 µg/mL, about 200 µg/mL to about 800 µg/mL, about 200 µg/mL to about 750 µg/mL, about 200 µg/mL to about 700 µg/mL, about 200 µg/mL to about 650 µg/mL, about 200 µg/mL to about 600 µg/mL, about 200 µg/mL to about 550 µg/mL, about 200 µg/mL to about 500 µg/mL, about 200 µg/mL to about 450 µg/mL, about 200 µg/mL to about 400 µg/mL, about 200 µg/mL to about 350 µg/mL, about 200 µg/mL to about 300 µg/mL, or about 200 µg/mL to about 250 µg/mL. The concentration of the chlorhexidine can be about 10 mg/mL, about 9 mg/mL, about 8 mg/mL, about 7 mg/mL, about 6 mg/mL, about 5 mg/mL, about 4 mg/mL, about 3 mg/mL, about 2.5 mg/mL, about 2 mg/mL, about 1.5 mg/mL, about 1.25 mg/mL, about 1 mg/mL, about 900 µg/mL, about 850 µg/mL, about 800 µg/mL, about 750 µg/mL, about 700 µg/mL, about 650 µg/mL, about 600 µg/mL, about 550 µg/mL, about 500 µg/mL, about 450 µg/mL, about 400 µg/mL, about 350 µg/mL, about 325 µg/mL, about 300 µg/mL, about 275 µg/mL, about 250 µg/mL, about 225 µg/mL, about 200 µg/mL, about 175 µg/mL, about 170 µg/mL, about 160 µg/mL, about 150 µg/mL, about 140 µg/mL, about 130 µg/mL, about 120 µg/mL, about 110 µg/mL, about 100 µg/mL, about 90 µg/mL, about 80 µg/mL, about 70 µg/mL, about 65 µg/mL, about 60 µg/mL, about 55 µg/mL, about 50 µg/mL, about 45 µg/mL, about 40 µg/mL, about 35 µg/mL, about 30 µg/mL, about 25 µg/mL, about 20 µg/mL, about 15 µg/mL, about 10 µg/mL, about 5 µg/mL, about 2 µg/mL, or about 1 µg/mL.

In some embodiments, the chlorhexidine concentration in the wound irrigation solution is less than 4% w/w. In some embodiments, the chlorhexidine is less than 2% w/w, or even less than 1% w/w. In one embodiment, the chlorhexidine concentration in the wound irrigation solution is 0.05% w/w. In a further embodiment, the chlorhexidine concentration in the wound irrigation solution is between 0.02% and 0.05% w/w. In some embodiments, the concentration of the chlorhexidine in the wound irrigation solution is between about 100 µg/mL to about 5 mg/mL. In some embodiments, the concentration of the chlorhexidine in the wound irrigation solution is between about 200 µg/mL to about 1 mg/mL or about 100 µg/mL to about 500 µg/mL. In some embodiments, the concentration of the chlorhexidine in the wound irrigation solution is about 500 µg/mL. Specifically exemplified herein is the use of chlorhexidine gluconate.

Wound irrigation solutions of methods and devices described herein can contain any concentration of octenidine or a pharmaceutically acceptable salt of octenidine (e.g., octenidine dihydrochloride, octenidine free base). The concentration of octenidine or a pharmaceutically acceptable salt of octenidine can be between about 5 µg/mL to about 10 mg/mL, about 5 µg/mL to about 9 mg/mL, about 5 µg/mL to about 8 mg/mL, about 5 µg/mL to about 7 mg/mL, about 5 µg/mL to about 6 mg/mL, about 5 µg/mL to about 5 mg/mL, about 5 µg/mL to about 4 mg/mL, about 5 µg/mL to about 3 mg/mL, about 5 µg/mL to about 2 mg/mL, about 10 µg/mL to about 10 mg/mL, about 20 µg/mL to about 10 mg/mL, about 30 µg/mL to about 10 mg/mL, about 40 µg/mL to about 10 mg/mL, about 50 µg/mL to about 10 mg/mL, about 60 µg/mL to about 10 mg/mL, about 0.125 µg/mL to about 1 mg/mL, about 0.15 µg/mL to about 1 mg/mL, about 0.175 µg/mL to about 1 mg/mL, about 0.2 µg/mL to about 1 mg/mL, about 0.225 µg/mL to about 1 mg/mL, about 0.25 µg/mL to about 1 mg/mL, about 0.275 µg/mL to about 1 mg/mL, about 0.3 µg/mL to about 1 mg/mL, about 0.325 µg/mL to about 1 mg/mL, about 0.35 µg/mL to about 1 mg/mL, about 0.375 µg/mL to about 1 mg/mL, about 0.4 µg/mL to about 1 mg/mL, about 0.425 µg/mL to about 1 mg/mL, about 0.45 µg/mL to about 1 mg/mL, about 0.475 µg/mL to about 1 mg/mL, about 0. 5 µg/mL to about 1 mg/mL, about 0.55 µg/mL to about 1 mg/mL, about 0.6 µg/mL to about 1 mg/mL, about 0.65 µg/mL to about 1 mg/mL, about 0.7 µg/mL to about 1 mg/mL, about 0.75 µg/mL to about 1 mg/mL, about 0.8 µg/mL to about 1 mg/mL, about 0.85 µg/mL to about 1 mg/mL, about 0.9 µg/mL to about 1 mg/mL, about 0.95 µg/mL to about 1 mg/mL, about 1 µg/mL to about 1 mg/mL, about 1.1 µg/mL to about 1 mg/mL, about 1.2 µg/mL to about 1 mg/mL, about 1.3 µg/mL to about 1 mg/mL, about 1.4 µg/mL to about 1 mg/mL, about 1.5 µg/mL to about 1 mg/mL, about 1.6 µg/mL to about 1 mg/mL, about 1.7 µg/mL to about 1 mg/mL, about 1.8 µg/mL to about 1 mg/mL, about 1.9 µg/mL to about 1 mg/mL, about 2 µg/mL to about 1 mg/mL, about 2.2 µg/mL to about 1 mg/mL, about 2.4 µg/mL to about 1 mg/mL, about 2.6 µg/mL to about 1 mg/mL, about 2.8 µg/mL to about 1 mg/mL, about 3 µg/mL to about 1 mg/mL, about 3.3 µg/mL to about 1 mg/mL, about 3.6 µg/mL to about 1 mg/mL, about 4 µg/mL to about 1 mg/mL, about 4.5 µg/mL to about 1 mg/mL, about 5 µg/mL to about 1 mg/mL, about 6

µg/mL to about 1 mg/mL, about 7 µg/mL to about 1 mg/mL, about 8 µg/mL to about 1 mg/mL, about 9 µg/mL to about 1 mg/mL, about 10 µg/mL to about 1 mg/mL, about 12 µg/mL to about 1 mg/mL, about 14 µg/mL to about 1 mg/mL, about 16 µg/mL to about 1 mg/mL, about 18 µg/mL to about 1 mg/mL, about 20 µg/mL to about 1 mg/mL, about 24 µg/mL to about 1 mg/mL, about 28 µg/mL to about 1 mg/mL, about 30 µg/mL to about 1 mg/mL, about 35 µg/mL to about 1 mg/mL, about 40 µg/mL to about 1 mg/mL, about 45 µg/mL to about 1 mg/mL, about 50 µg/mL to about 1 mg/mL, about 55 µg/mL to about 1 mg/mL, about 60 µg/mL to about 1 mg/mL, about 65 µg/mL to about 1 mg/mL, about 70 µg/mL to about 1 mg/mL, about 75 µg/mL to about 1 mg/mL, about 80 µg/mL to about 1 mg/mL, about 85 µg/mL to about 1 mg/mL, about 90 µg/mL to about 1 mg/mL, about 95 µg/mL to about 1 mg/mL, about 100 µg/mL to about 1 mg/mL, about 120 µg/mL to about 1 mg/mL, about 140 µg/mL to about 1 mg/mL, about 160 µg/mL to about 1 mg/mL, about 180 µg/mL to about 1 mg/mL, about 200 µg/mL to about 1 mg/mL, about 250 µg/mL to about 1 mg/mL, about 300 µg/mL to about 1 mg/mL, about 350 µg/mL to about 1 mg/mL, about 400 µg/mL to about 1 mg/mL, about 450 µg/mL to about 1 mg/mL, about 500 µg/mL to about 1 mg/mL, about 550 µg/mL to about 1 mg/mL, about 600 µg/mL to about 1 mg/mL, about 650 µg/mL to about 1 mg/mL, about 700 µg/mL to about 1 mg/mL, about 750 µg/mL to about 1 mg/mL, about 800 µg/mL to about 1 mg/mL, about 850 µg/mL to about 1 mg/mL, about 900 µg/mL to about 1 mg/mL, about 950 µg/mL to about 1 mg/mL, about 0.125 µg/mL to about 950 µg/mL, about 0.125 µg/mL to about 900 µg/mL, about 0.125 µg/mL to about 850 µg/mL, about 0.125 µg/mL to about 800 µg/mL, about 0.125 µg/mL to about 750 µg/mL, about 0.125 µg/mL to about 700 µg/mL, about 0.125 µg/mL to about 650 µg/mL, about 0.125 µg/mL to about 600 µg/mL, about 0.125 µg/mL to about 550 µg/mL, about 0.125 µg/mL to about 500 µg/mL, about 0.125 µg/mL to about 450 µg/mL, about 0.125 µg/mL to about 400 µg/mL, about 0.125 µg/mL to about 375 µg/mL, about 0.125 µg/mL to about 350 µg/mL, about 0.125 µg/mL to about 325 µg/mL, about 0.125 µg/mL to about 300 µg/mL, about 0.125 µg/mL to about 275 µg/mL, about 0.125 µg/mL to about 250 µg/mL, about 0.125 µg/mL to about 225 µg/mL, about 0.125 µg/mL to about 200 µg/mL, about 0.125 µg/mL to about 175 µg/mL, about 0.125 µg/mL to about 150 µg/mL, about 0.125 µg/mL to about 125 µg/mL, about 0.125 µg/mL to about 100 µg/mL, about 0.125 µg/mL to about 75 µg/mL, about 0.125 µg/mL to about 50 µg/mL, about 0.125 µg/mL to about 25 µg/mL, about 0.125 µg/mL to about 20 µg/mL, about 0.125 µg/mL to about 15 µg/mL, about 0.125 µg/mL to about 10 µg/mL, about 0.125 µg/mL to about 9 µg/mL, about 0.125 µg/mL to about 8 µg/mL, about 0.125 µg/mL to about 7 µg/mL, about 0.125 µg/mL to about 6 µg/mL, about 0.125 µg/mL to about 5 µg/mL, about 0.125 µg/mL to about 4.5 µg/mL, about 0.125 µg/mL to about 4 µg/mL, about 0.125 µg/mL to about 3.8 µg/mL, about 0.125 µg/mL to about 3.6 µg/mL, about 0.125 µg/mL to about 3.3 µg/mL, about 0.125 µg/mL to about 3 µg/mL, about 0.125 µg/mL to about 2.8 µg/mL, about 0.125 µg/mL to about 2.6 µg/mL, about 0.125 µg/mL to about 2.4 µg/mL, about 0.125 µg/mL to about 2.2 µg/mL, about 0.125 µg/mL to about 2 µg/mL, about 0.125 µg/mL to about 1.9 µg/mL, about 0.125 µg/mL to about 1.8 µg/mL, about 0.125 µg/mL to about 1.7 µg/mL, about 0.125 µg/mL to about 1.6 µg/mL, about 0.125 µg/mL to about 1.5 µg/mL, about 0.125 µg/mL to about 1.4 µg/mL, about 0.125 µg/mL to about 1.3 µg/mL, about 0.125 µg/mL to about 1.2 µg/mL, about 0.125 µg/mL to about 1.1 µg/mL, about 0.125 µg/mL to about 1 µg/mL, about 0.125 µg/mL to about 0.9 µg/mL, about 0.125 µg/mL to about 0.8 µg/mL, about 0.125 µg/mL to about 0.7 µg/mL, about 0.125 µg/mL to about 0.6 µg/mL, about 0.125 µg/mL to about 0.5 µg/mL, about 0.125 µg/mL to about 0.4 µg/mL, about 0.125 µg/mL to about 0.3 µg/mL, about 0.125 µg/mL to about 0.2 µg/mL, about 0.25 µg/mL to about 50 µg/mL, 0.3 µg/mL to about 50 µg/mL, 0.4 µg/mL to about 50 µg/mL, about 0.5 µg/mL to about 50 µg/mL, about 0.6 µg/mL to about 50 µg/mL, about 0.7 µg/mL to about 50 µg/mL, about 0.8 µg/mL to about 50 µg/mL, about 0.9 µg/mL to about 50 µg/mL, about 1 µg/mL to about 50 µg/mL, about 1.2 µg/mL to about 50 µg/mL, about 1.4 µg/mL to about 50 µg/mL, about 1.6 µg/mL to about 50 µg/mL, about 1.8 µg/mL to about 50 µg/mL, about 2 µg/mL to about 50 µg/mL, about 2.2 µg/mL to about 50 µg/mL, about 2.4 µg/mL to about 50 µg/mL, about 2.6 µg/mL to about 50 µg/mL, about 2.8 µg/mL to about 50 µg/mL, about 3 µg/mL to about 50 µg/mL, about 3.3 µg/mL to about 50 µg/mL, about 3.6 µg/mL to about 50 µg/mL, about 4 µg/mL to about 50 µg/mL, about 4.5 µg/mL to about 50 µg/mL, about 5 µg/mL to about 50 µg/mL, about 5.5 µg/mL to about 50 µg/mL, about 6 µg/mL to about 50 µg/mL, about 6.5 µg/mL to about 50 µg/mL, about 7 µg/mL to about 50 µg/mL, about 7.5 µg/mL to about 50 µg/mL, about 8 µg/mL to about 50 µg/mL, about 8.5 µg/mL to about 50 µg/mL, about 9 µg/mL to about 50 µg/mL, about 9.5 µg/mL to about 50 µg/mL, about 10 µg/mL to about 50 µg/mL, about 15 µg/mL to about 50 µg/mL, about 20 µg/mL to about 50 µg/mL, about 25 µg/mL to about 50 µg/mL, about 30 µg/mL to about 50 µg/mL, about 35 µg/mL to about 50 µg/mL, about 40 µg/mL to about 50 µg/mL, about 45 µg/mL to about 50 µg/mL, about 0.25 µg/mL to about 45 µg/mL, about 0.25 µg/mL to about 40 µg/mL, about 0.25 µg/mL to about 37.5 µg/mL, about 0.25 µg/mL to about 35 µg/mL, about 0.25 µg/mL to about 32.5 µg/mL, about 0.25 µg/mL to about 30 µg/mL, about 0.25 µg/mL to about 27.5 µg/mL, about 0.25 µg/mL to about 25 µg/mL, about 0.25 µg/mL to about 22.5 µg/mL, about 0.25 µg/mL to about 20 µg/mL, about 0.25 µg/mL to about 17.5 µg/mL, about 0.25 µg/mL to about 15 µg/mL, about 0.25 µg/mL to about 12.5 µg/mL, about 0.25 µg/mL to about 10 µg/mL, about 0.25 µg/mL to about 9 µg/mL, about 0.25 µg/mL to about 8 µg/mL, about 0.25 µg/mL to about 7 µg/mL, about 0.25 µg/mL to about 6 µg/mL, about 0.25 µg/mL to about 5 µg/mL, about 0.25 µg/mL to about 4.5 µg/mL, about 0.25 µg/mL to about 4 µg/mL, about 0.25 µg/mL to about 3.5 µg/mL, about 0.25 µg/mL to about 3 µg/mL, about 0.25 µg/mL to about 2.5 µg/mL, about 0.25 µg/mL to about 2 µg/mL, about 0.25 µg/mL to about 1.5 µg/mL, about 0.25 µg/mL to about 1.25 µg/mL, about 0.25 µg/mL to about 1 µg/mL, about 0.25 µg/mL to about 0.75 µg/mL, about 0.25 µg/mL to about 0.5 µg/mL, about 1 µg/mL to about 20 µg/mL, about 1.2 µg/mL to about 20 µg/mL, about 1.4 µg/mL to about 20 µg/mL, about 1.6 µg/mL to about 20 µg/mL, about 1.8 µg/mL to about 20 µg/mL, about 2 µg/mL to

about 20 μg/mL, about 2.2 μg/mL to about 20 μg/mL, about 2.4 μg/mL to about 20 μg/mL, about 2.6 μg/mL to about 20 μg/mL, about 2.8 μg/mL to about 20 μg/mL, about 3 μg/mL to about 20 μg/mL, about 3.5 μg/mL to about 20 μg/mL, about 4 μg/mL to about 20 μg/mL, about 4.5 μg/mL to about 20 μg/mL, about 5 μg/mL to about 20 μg/mL, about 5.5 μg/mL to about 20 μg/mL, about 6 μg/mL to about 20 μg/mL, about 6.5 μg/mL to about 20 μg/mL, about 7 μg/mL to about 20 μg/mL, about 7.5 μg/mL to about 20 μg/mL, about 8 μg/mL to about 20 μg/mL, about 8.5 μg/mL to about 20 μg/mL, about 9 μg/mL to about 20 μg/mL, about 9.5 μg/mL to about 20 μg/mL, about 10 μg/mL to about 20 μg/mL, about 11 μg/mL to about 20 μg/mL, about 12 μg/mL to about 20 μg/mL, about 13 μg/mL to about 20 μg/mL, about 14 μg/mL to about 20 μg/mL, about 15 μg/mL to about 20 μg/mL, about 16 μg/mL to about 20 μg/mL, about 17 μg/mL to about 20 μg/mL, about 18 μg/mL to about 20 μg/mL, about 19 μg/mL to about 20 μg/mL, about 1 μg/mL to about 19 μg/mL, about 1 μg/mL to about 18 μg/mL, about 1 μg/mL to about 17 μg/mL, about 1 μg/mL to about 16 μg/mL, about 1 μg/mL to about 15 μg/mL, about 1 μg/mL to about 14 μg/mL, about 1 μg/mL to about 13 μg/mL, about 1 μg/mL to about 12 μg/mL, about 1 μg/mL to about 11 μg/mL, about 1 μg/mL to about 10 μg/mL, about 1 μg/mL to about 9.5 μg/mL, about 1 μg/mL to about 9 μg/mL, about 1 μg/mL to about 8.5 μg/mL, about 1 μg/mL to about 8.0 μg/mL, about 1 μg/mL to about 7.5 μg/mL, about 1 μg/mL to about 7 μg/mL, about 1 μg/mL to about 6.5 μg/mL, about 1 μg/mL to about 6 μg/mL, about 1 μg/mL to about 5.5 μg/mL, about 1 μg/mL to about 5 μg/mL, about 1 μg/mL to about 4.5 μg/mL, about 1 μg/mL to about 4 μg/mL, about 1 μg/mL to about 3.5 μg/mL, about 1 μg/mL to about 3 μg/mL, about 1 μg/mL to about 2.8 μg/mL, about 1 μg/mL to about 2.6 μg/mL, about 1 μg/mL to about 2.4 μg/mL, about 1 μg/mL to about 2.2 μg/mL, about 1 μg/mL to about 2 μg/mL, about 1 μg/mL to about 1.8 μg/mL, about 1 μg/mL to about 1.6 μg/mL, about 1 μg/mL to about 1.4 μg/mL, about 1 μg/mL to about 1.2 μg/mL, about 1.2 μg/mL to about 5 μg/mL, about 1.4 μg/mL to about 5 μg/mL, about 1.6 μg/mL to about 5 μg/mL, about 1.8 μg/mL to about 5 μg/mL, about 2 μg/mL to about 5 μg/mL, about 2.2 μg/mL to about 5 μg/mL, about 2.4 μg/mL to about 5 μg/mL, about 2.6 μg/mL to about 5 μg/mL, about 2.8 μg/mL to about 5 μg/mL, about 3 μg/mL to about 5 μg/mL, about 3.3 μg/mL to about 5 μg/mL, about 3.6 μg/mL to about 5 μg/mL, about 3.9 μg/mL to about 5 μg/mL, about 4 μg/mL to about 5 μg/mL, about 4.5 μg/mL to about 5 μg/mL, about 2 μg/mL to about 10 μg/mL, about 2.2 μg/mL to about 10 μg/mL, about 2.4 μg/mL to about 10 μg/mL, about 2.6 μg/mL to about 10 μg/mL, about 2.8 μg/mL to about 10 μg/mL, about 3 μg/mL to about 10 μg/mL, about 3.2 μg/mL to about 10 μg/mL, about 3.4 μg/mL to about 10 μg/mL, about 3.6 μg/mL to about 10 μg/mL, about 3.8 μg/mL to about 10 μg/mL, about 4 μg/mL to about 10 μg/mL, about 4.5 μg/mL to about 10 μg/mL, about 5 μg/mL to about 10 μg/mL, about 5.5 μg/mL to about 10 μg/mL, about 6 μg/mL to about 10 μg/mL, about 6.5 μg/mL to about 10 μg/mL, about 7 μg/mL to about 10 μg/mL, about 7.5 μg/mL to about 10 μg/mL, about 8 μg/mL to about 10 μg/mL, about 8.5 μg/mL to about 10 μg/mL, about 9 μg/mL to about 10 μg/mL, about 9.5 μg/mL to about 10 μg/mL, about 2 μg/mL to about 9.5 μg/mL, about 2 μg/mL to about 9 μg/mL, about 2 μg/mL to about 8.5 μg/mL, about 2 μg/mL to about 8 μg/mL, about 2 μg/mL to about 7.5 μg/mL, about 2 μg/mL to about 7 μg/mL, about 2 μg/mL to about 6.5 μg/mL, about 2 μg/mL to about 6 μg/mL, about 2 μg/mL to about 5.5 μg/mL, about 2 μg/mL to about 5 μg/mL, about 2 μg/mL to about 4.5 μg/mL, about 2 μg/mL to about 4 μg/mL, about 2 μg/mL to about 3.8 μg/mL, about 2 μg/mL to about 3.6 μg/mL, about 2 μg/mL to about 3.4 μg/mL, about 2 μg/mL to about 3.2 μg/mL, about 2 μg/mL to about 3 μg/mL, about 2 μg/mL to about 2.8 μg/mL, about 2 μg/mL to about 2.6 μg/mL, about 2 μg/mL to about 2.4 μg/mL, or about 2 μg/mL to about 2.2 μg/mL. The concentration of octenidine or a pharmaceutically acceptable salt of octenidine can be about 0.125 μg/mL, about 0.25 μg/mL, about 0.375 μg/mL, about 0.5 μg/mL, about 0.625 μg/mL, about 0.75 μg/mL, about 0.875 μg/mL, about 1 μg/mL, about 1.125 μg/mL, about 1.25 μg/mL, about 1.375 μg/mL, about 1.5 μg/mL, about 1.625 μg/mL, about 1.75 μg/mL, about 1.875 μg/mL, about 2 μg/mL, about 2.125 μg/mL, about 2.25 μg/mL, about 2.375 μg/mL, about 2.5 μg/mL, about 2.625 μg/mL, about 2.75 μg/mL, about 2.875 μg/mL, about 3 μg/mL, about 3.125 μg/mL, about 3.25 μg/mL, about 3.375 μg/mL, about 3.5 μg/mL, about 3.625 μg/mL, about 3.75 μg/mL, about 3.875 μg/mL, about 4 μg/mL, about 4.125 μg/mL, about 4.25 μg/mL, about 4.375 μg/mL, about 4.5 μg/mL, about 4.625 μg/mL, about 4.75 μg/mL, about 4.875 μg/mL, about 5 μg/mL, about 6 μg/mL, about 7 μg/mL, about 8 μg/mL, about 9 μg/mL, about 10 μg/mL, about 15 μg/mL, about 20 μg/mL, about 25 μg/mL, about 30 μg/mL, about 35 μg/mL, about 40 μg/mL, about 45 μg/mL, about 50 μg/mL, about 55 μg/mL, about 60 μg/mL, about 65 μg/mL, about 70 μg/mL, about 75 μg/mL, about 80 μg/mL, about 85 μg/mL, about 90 μg/mL, about 95 μg/mL, about 100 μg/mL, about 110 μg/mL, about 120 μg/mL, about 130 μg/mL, about 140 μg/mL, about 150 μg/mL, about 160 μg/mL, about 170 μg/mL, about 180 μg/mL, about 190 μg/mL, about 200 μg/mL, about 220 μg/mL, about 240 μg/mL, about 260 μg/mL, about 280 μg/mL, about 300 μg/mL, about 330 μg/mL, about 360 μg/mL, about 390 μg/mL, about 400 μg/mL, about 440 μg/mL, about 480 μg/mL, about 500 μg/mL, about 550 μg/mL, about 600 μg/mL, about 650 μg/mL, about 700 μg/mL, about 750 μg/mL, about 800 μg/mL, about 850 μg/mL, about 900 μg/mL, about 950 μg/mL, or about 1 mg/mL. In some embodiments, the concentration of octenidine or a pharmaceutically acceptable salt of octenidine in the wound irrigation solution is between about 0.25 μg/mL to about 50 μg/mL. In some embodiments, the concentration of octenidine or a pharmaceutically acceptable salt of octenidine in the wound irrigation solution is between about 1 μg/mL to about 20 μg/mL. In some embodiments, the concentration of octenidine or a pharmaceutically acceptable salt of octenidine in the wound irrigation solution is between about 1 μg/mL to about 5 μg/mL. In some embodiments, the concentration of octenidine or a pharmaceutically acceptable salt of octenidine in the wound irrigation solution is between about 2 μg/mL to about 10 μg/mL. In some embodiments, the concentration of octenidine or a pharmaceutically acceptable salt of octenidine in the wound irrigation solution is about 1.875 μg/mL or about 3.75 μg/mL.

The wound irrigation solution can contain any ratio of two antiseptic agents. The ratio (weight-weight ratio (w/w ratio), ratio of concentrations) of two antiseptic agents can be about 1:1, about 1:1.25, about 1:1.5, about 1:1.75, about 1:2, about 1:2.25, about 1:2.5, about 1:2.75, about 1:3, about 1:3.25, about 1:3.5, about 1:3.75, about 1:4, about 1:4.25, about 1:4.5, about 1:4.75, about 1:5, about 1:5.25, about 1:5.5, about 1:5.75, about 1:6, about 1:6.25, about 1:6.5, about 1:6.75, about 1:7, about 1:7.25, about 1:7.5, about 1:7.75, about 1:8, about 1:8.25, about 1:8.5, about 1:8.75, about 1:9, about 1:9.25, about 1:9.5, about 1:9.75, about 1:10, about 1:11, about 1:12, about 1:13, about 1:14, about 1:15, about 1:16, about 1:17, about 1:18, about 1:19, about 1:20, about 1:25, about 1:30, about 1:35, about 1:40, about 1:45, about 1:50, about 1:55, about 1:60, about 1:65, about 1:70, about 1:75, about 1:80, about 1:85, about 1:90, about 1:95, about 1:100, about 1:125, about 1:150, about 1:175, about 1:200, about 1:300, about 1:400, about 1:500, about 1:600, about 1:700, about 1:800, about 1:900, about 1:1000, about 1:1100, about 1:1200, about 1:1300, about 1:1400, about 1:1500, about 1:1600, about 1:1700, about 1:1800, about 1:1900, about 1:2000, about 1:2200, about 1:2400, about 1:2600, about 1:2800, about 1:3000, about 1:3200, about 1:3400, about 1:3600, about 1:3800, about 1:4000, about 1:4200, about 1:4400, about 1:4600, about 1:4800, about 1:5000, about 2:3, about 3:4, about 3:5, about 4:5, about 5:6, about 6:7, about 7:8, about 7:9, about 8:9, about 7:10, or about 9:10. In some embodiments, the ratio of two antiseptic agents is about 1:267. In some embodiments, the ratio of two antiseptic agents is about 1:133.

Wound irrigation solutions used in the methods and devices described herein can be synergistically antimicrobial (e.g., microbiocidal or microbiostatic, synergistic with respect to two or more antiseptic agents contained in the solution) to any microorganism (e.g., any microorganism described herein), including but not limited to, a virus, a bacterium, a fungus, a protozoan, or combinations thereof. One way to measure synergy is by fractional inhibitory concentration (FIC) index. FIC index can be calculated by the following Equation 1:

$$FIC = \frac{AA1\ MIC\ in\ combination\ with\ AA2}{AA1\ MIC\ alone} + \frac{AA2\ MIC\ in\ combination\ with\ AA1}{AA2\ MIC\ alone}\ \text{(Equation 1)},$$

wherein AA1 is a first antiseptic agent and AA2 is a second antiseptic agent. Irrigation solutions described herein can be synergistically antimicrobial with an FIC index of at most about 0.5, at most about 0.45, at most about 0.40, at most about 0.35, at most about 0.30, at most about 0.25, at most about 0.20, at most about 0.15, or at most about 0.1. In some embodiments, the FIC index is at most about 0.5.

Wound irrigation solutions can contain a combination of two or more antiseptic agents that are synergistically microbiocidal or microbiostatic to any microorganism (e.g., any fungus, any bacterium, any protozoan, any virus, any microorganism described herein). For example, the wound irrigation solution can be synergistically microbiocidal or microbiostatic for any one of the following: *Acinetobacter spp., Bordetella spp., Burkholderia* spp., *Campylobacter* spp., *Clostridioides difficile, Cutibacterium spp.* (e.g., *Cutibacterium acnes, Cutibacterium avidum, Cutibacterium granulosum, Cutibacterium modestum, Cutibacterium namnetense, Cutibacterium porci), Enterobacterales spp., Enterococci spp., Lactobacillus* spp. *(L. rhamnosus, L. acidophilus, L. casei, L. fermentum, L. paracasei, L. corneformis), Mycoplasma spp., Neisseria spp., Pseudomonas spp., Ralstonia spp., Staphylococcus spp., Streptococcus spp.,* or combinations thereof. In some embodiments, the wound irrigation solution is synergistically microbiocidal or microbiostatic to a fungus. In some embodiments, the wound irrigation solution is synergistically microbiocidal or microbiostatic to a bacterium. In some embodiments, the wound irrigation solution is synergistically microbiocidal or microbiostatic to a virus. In some embodiments, the wound irrigation solution is synergistically microbiocidal or microbiostatic to a protozoan. In some embodiments, the wound irrigation solution is synergistically microbiocidal or microbiostatic to a fungus and/or a bacterium. In some embodiments, the wound irrigation solution is synergistically microbiocidal or microbiostatic to *a Candida* species. In some embodiments, the wound irrigation solution is synergistically microbiocidal or microbiostatic to an *E. coli.* In some embodiments, the wound irrigation solution is synergistically microbiocidal or microbiostatic to *Staphylococcus* species. In some embodiments, the wound irrigation solution is synergistically microbiocidal or microbiostatic to *Ralstonia* species. In some embodiments, the wound irrigation solution is synergistically micobiocidal or microbiostatic to *Pseudomonas* species. In some embodiments, the wound irrigation solution is synergistically microbiocidal or microbiostatic to *Candida* species, *E. coli, Staphylococcus* species, *Ralstonia* species, *Pseudomonas* species, or a combination thereof. In some embodiments, the wound irrigation solution is synergistically microbiocidal or microbiostatic to *E. coli, P. aeruginosa,* or a combination thereof. In some embodiments, the wound irrigation solution is synergistically microbiocidal or microbiostatic to C. *albicans, E. coli,* methicillin-resistant *Staphylococcus aureus, Staphylococcus aureus, Staphylococcus epidermidis, Ralstonia pickettii, P. aeruginosa,* or a combination thereof. In some embodiments, the wound irrigation solution is synergistically microbiocidal or microbiostatic to C. *albicans.* In some embodiments, the wound irrigation solution is synergistically microbiocidal or microbiostatic to *E. coli.* In some embodiments, the wound irrigation solution is synergistically microbiocidal or microbiostatic to *Staphylococcus aureus.* In some embodiments, the wound irrigation solution is synergistically microbiocidal or microbiostatic to methicillin-resistant *Staphylococcus aureus.* In some embodiments, the wound irrigation solution is synergistically microbiocidal or microbiostatic to *Staphylococcus epidermidis.* In some embodiments, the wound irrigation solution is synergistically microbiocidal or microbiostatic to *Ralstonia pickettii.* In

some embodiments, the wound irrigation solution is synergistically microbiocidal or microbiostatic to *P. aeruginosa.* In some embodiments, the wound irrigation solution is synergistically microbiocidal or microbiostatic to a *Cutibacterium* species (e.g., *Cutibacterium acnes).* In some embodiments, the wound irrigation solution is synergistically microbiocidal or microbiostatic to *a Lactobacillus* species *(L. rhamnosus).*

Wound irrigation solutions described herein can comprise any number of different antiseptic agents including, but not limited to, 2, 3, 4, 5, 6, 7, 8, 9, or 10. The number of antiseptic agents can be between 2 and 3, between 2 and 4, between 2 and 5, between 2 and 6, between 2 and 7, between 2 and 8, between 2 and 9, between 2 and 10, between 3 and 4, between 3 and 5, between 3 and 6, between 3 and 7, between 3 and 8, between 3 and 9, or between 3 and 10.

A microbial burden (e.g., a bacterial burden, a fungal burden, a viral burden, a protozoal burden, a microbial burden of a wound) of any microorganism (e.g., the microorganisms disclosed herein) treated with a method or device described herein can be reduced by any degree. One way to measure microbial burden is by colony forming units (CFU). The microbial burden can be reduced by any magnitude (e.g., the CFUs can be reduced by any magnitude) following treatment with a method or device described herein. The magnitude of microbial burden can be reduced by about 2-fold, about 3-fold, about 4-fold, about 5-fold, about 6-fold, about 7-fold, about 8-fold, about 9-fold, about 10-fold, about 15-fold, about 20-fold, about 25-fold, about 30-fold, about 40-fold, about 50-fold, about 60-fold, about 70-fold, about 80-fold, about 90-fold, about 100-fold, about 120-fold, about 130-fold, about 140-fold, about 150-fold, about 160-fold, about 170-fold, about 180-fold, about 190-fold, about 200-fold, about 250-fold, about 300-fold, about 350-fold, about 400-fold, about 450-fold, about 500-fold, about 550-fold, about 600-fold, about 650-fold, about 700-fold, about 750-fold, about 800-fold, about 850-fold, about 900-fold, about 950-fold, about 1000-fold, about 1200-fold, about 1400-fold, about 1600-fold, about 1800-fold, about 2000-fold, about 3000-fold, about 4000-fold, about 5000-fold, about 6000-fold, about 7000-fold, about 8000-fold, about 9000-fold, or about 10000-fold. The magnitude of microbial burden can be reduced by at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least at least about 30-fold, at least about 40-fold, at least about 50-fold, at least about 60-fold, at least about 70-fold, at least about 80-fold, at least about 90-fold, at least about 100-fold, at least about 120-fold, at least about 130-fold, at least about 140-fold, at least about 150-fold, at least about 160-fold, at least about 170-fold, at least about 180-fold, at least about 190-fold, at least about 200-fold, at least about 250-fold, at least about 300-fold, at least about 350-fold, at least about 400-fold, at least about 450-fold, at least about 500-fold, at least about 550-fold, at least about 600-fold, at least about 650-fold, at least about 700-fold, at least about 750-fold, at least about 800-fold, at least about 850-fold, at least about 900-fold, at least about 950-fold, at least about 1000-fold, at least about 1200-fold, at least about 1400-fold, at least about 1600-fold, at least about 1800-fold, at least about 2000-fold, at least about 3000-fold, at least about 4000-fold, at least about 5000-fold, at least about 6000-fold, at least about 7000-fold, at least about 8000-fold, at least about 9000-fold, or at least about 10000-fold. Wound irrigation solutions in methods and devices described herein can reduce a microbial burden (e.g., a microbial burden of a wound, a fungal burden, a bacterial burden, a viral burden, or a protozoal burden, the burden of any microorganism described herein) to any amount of microorganism (e.g., any number of colony forming units (CFUs)). Wound irrigation solutions can decrease the microbial burden (e.g., the burden of any bacterium, fungus, virus, protozoa described herein) to a number of CFUs per gram of tissue that is at most about $10^7$, at most about $5 \times 10^6$, at most about $10^6$, at most about $5 \times 10^5$, at most about $10^5$, at most about $5 \times 10^4$, at most about $10^4$, at most about $5 \times 10^3$, at most about $10^3$, at most about $5 \times 10^2$, at most about $10^2$, at most about $5 \times 10^1$, at most about $10^1$, at most about 5, at most about 1, or zero.

Wound irrigation solutions described herein containing two or more antiseptic agents (e.g., containing a synergistic combination of two or more antiseptic agents) can reduce the amount of irrigation time required to reduce a microbial burden (e.g., a microbial burden of a wound, a fungal burden, a bacterial burden, a viral burden, a protozoal burden, a burden of any microorganism described herein to any amount or magnitude described herein). The reduction in irrigation time can be measured by comparison to an analogous method or device that uses a wound irrigation solution that contains only one antiseptic agent present at an equivalent concentration (e.g., contains only chlorhexidine without an additional antiseptic agent, contains only octenidine or a pharmaceutically acceptable salt of octenidine without an additional antiseptic agent, contains a non-synergistic combination of two or more antiseptic agents). The degree of irrigation time reduction can be at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, at least about 99%, or at least about 99.5%. In some embodiments, an amount of irrigation time required to reduce a bacterial burden of the wound or a fungal burden of the wound by at least about 100-fold compared to an analogous method using an alternative irrigation solution comprising the same concentration of chlorhexidine without an additional antiseptic agent is reduced by at least about 50%.

A wound can be irrigated for any duration of time (e.g., any irrigation time). The amount of irrigation time required to reduce a microbial burden (e.g., a bacterial burden, a fungal burden, a viral burden, a protozoal burden, reduce a microbial burden by any magnitude described herein) can be within 30 minutes, 29 minutes, 28 minutes, 27 minutes, 26 minutes, 25 minutes, 24 minutes, 23 minutes, 22 minutes, 21 minutes, 20 minutes, 19 minutes, 18 minutes, 17 minutes, 16

minutes, 15 minutes, 14 minutes, 13 minutes, 12 minutes, 11 minutes, 10 minutes, 9 minutes, 8 minutes, 7 minutes, 6 minutes, 5 minutes, 4 minutes, 3.5 minutes, 3 minutes, 2.5 minutes, 2 minutes, 1.5 minutes, 1 minute, 45 seconds, 30 seconds, or 15 seconds. The required irrigation time can be between about 1 minute to about 30 minutes, about 1 minute to about 25 minutes, about 1 minute to about 20 minutes, about 1 minute to about 15 minutes, about 1 minute to about 10 minutes, about 1 minute to about 5 minutes, or about 1 minute to about 3 minutes. In some embodiments, the amount of irrigation time required is within 15 minutes. In some embodiments, wherein an amount of irrigation time required to reduce a bacterial burden of the wound or a fungal burden of the wound by at least 100-fold compared to an analogous method using an alternative irrigation solution comprising the same concentration of chlorhexidine without an additional antiseptic agent is reduced by at least about 50% and is within 15 minutes, within 10 minutes, within 5 minutes, within 3 minutes, or within 1 minute.

As described above, in some embodiments, the device described herein is used to deliver an active agent (e.g., a synergistic combination of two antiseptic agents), such as an antimicrobial agent, to a target site, such as a wound. Subsequent to the administration of the active agent, the site can then be flushed with, for example, saline to remove at least any excess of the active agent. Flushing of the site can occur within any period of time following the administration of the wound irrigation solution. The flushing of the site can occur within a period of time that is about 30 seconds, about 45 seconds, about 1 minute, about 1.5 minutes, about 2 minutes, about 2.5 minutes, about 3 minutes, about 3.5 minutes, about 4 minutes, about 4.5 minutes, about 5 minutes, about 5.5 minutes, about 6 minutes, about 6.5 minutes, about 7 minutes, about 7.5 minutes, about 8 minutes, about 8.5 minutes, about 9 minutes, about 9.5 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 24 minutes, about 26 minutes, about 28 minutes, or about 30 minutes. In some embodiments flushing occurs within five minutes of the administration of the wound irrigation solution. Flushing of the site can occur following the administration of the wound irrigation solution in a time period between about 30 seconds and about 45 seconds, about 30 seconds and about 1 minute, about 30 seconds and about 1.5 minutes, about 30 seconds and about 2 minutes, about 30 seconds and about 2.5 minutes, about 30 seconds and about 3 minutes, about 30 seconds and about 3.5 minutes, about 30 seconds and about 4 minutes, about 30 seconds and about 4.5 minutes, about 30 seconds and about 5 minutes, about 30 seconds and about 5.5 minutes, about 30 seconds and about 6 minutes, about 30 seconds and about 6.5 minutes, about 30 seconds and about 7 minutes, about 30 seconds and about 7.5 minutes, about 30 seconds and about 8 minutes, about 30 seconds and about 8.5 minutes, about 30 seconds and about 9 minutes, about 30 seconds and about 9.5 minutes, about 30 seconds and about 10 minutes, about 30 seconds and about 15 minutes, about 30 seconds and about 20 minutes, about 1 minute to about 30 minutes, about 2 minutes to about 30 minutes, about 3 minutes to about 30 minutes, about 4 minutes to about 30 minutes, about 5 minutes to about 30 minutes, about 6 minutes to about 30 minutes, about 7 minutes to about 30 minutes, about 8 minutes to about 30 minutes, about 9 minutes to about 30 minutes, about 10 minutes to about 30 minutes, about 15 minutes to about 30 minutes, about 20 minutes to about 30 minutes, about 25 minutes to about 30 minutes, about 5 minutes to about 30 minutes, about 1 minute to about 2 minutes, about 1 minute to about 3 minutes, about 1 minute to about 4 minutes, about 1 minute to about 5 minutes, about 1 minute to about 6 minutes, about 1 minute to about 7 minutes, about 1 minute to about 8 minutes, about 1 minute to about 9 minutes, about 1 minute to about 10 minutes, about 1 minute to about 15 minutes, about 1 minute to about 20 minutes, about 1 minute to about 25 minutes, about 1 minute to about 30 minutes, about 3 minutes to about 4 minutes, about 3 minutes to about 5 minutes, about 3 minutes to about 6 minutes, about 3 minutes to about 7 minutes, about 3 minutes to about 8 minutes, about 3 minutes to about 9 minutes, about 3 minutes to about 10 minutes, about 3 minutes to about 15 minutes, about 3 minutes to about 20 minutes, about 3 minutes to about 25 minutes, or about 3 minutes to about 30 minutes. In some embodiments, flushing occurs within one to three minutes of the administration of the wound irrigation solution. In this way, any potential toxicity, sensitivity, contact dermatitis and/or allergy associated with the active agent (e.g., an antiseptic agent) can be reduced or eliminated. In the case of a chlorhexidine or octenidine or a pharmaceutically acceptable salt of octenidine, rinsing with an irrigation fluid removes excess chlorhexidine or octenidine that has not bound to, for example, proteins of the skin or other tissue.

In yet another embodiment, a diagnostic agent can be administered using the devices and methods described herein. The diagnostic agent may be, for example, an antibody, protein, or polynucleotide that binds to a target biomolecule. Any such binding may then be visualized utilizing technologies known to those skilled in the art. These technologies include, for example, the use of fluorophores or other labels that can be visualized either by the naked eye or through appropriate detection instruments. The diagnostic applications of the devices and methods described herein include the detection of bacteria, viruses, parasites, and other pathogens. Cancer cells can also be visualized using the diagnostic methods described herein.

In yet another embodiment, the device and method can be used to deliver growth factors and/or protease inhibitors to a target site. Such growth factors and/or protease inhibitors, which can, for example, expedite the healing of wounds, are well known to those skilled in the art.

In yet another embodiment, the methods described herein can be used to deliver oxygenated water and/or "enhanced water" to a target site. The enhanced water can be that which is described in, for example, published U.S.

Patent Application Publication US20050191364A1 and the references cited therein. The use of methods described herein for the effective delivery of such oxygenated or enhanced water can be used to promote tissue healing and reduce infections.

In a further embodiment, the devices, and methods described herein can be used to efficiently deliver antimicrobial peptides (AMPs) to a target site. AMPs are well known in the art. Antimicrobial peptides are predominantly small polypeptides that inhibit the growth of microbes. As effectors of innate immunity, antimicrobial peptides directly kill a broad spectrum of bacteria, fungi, and viruses. In addition, these peptides modify the local inflammatory response and activate mechanisms of cellular and adaptive immunity. Cathelicidins and defensins comprise the major families of AMPs in the skin, although other cutaneous peptides, such as proteinase inhibitors, chemokines, and neuropeptides, also demonstrate antimicrobial activity. See, for example, Braff, M. et al., (2005) "Cutaneous Defense Mechanisms by Antimicrobial Peptides," J Invest Dermatol, 125:9-13.

The Drug Delivery Device

In some embodiments, the device described herein provides a reservoir housing containing a solution with two or more active agents (e.g., a wound irrigation solution), wherein the reservoir housing has attached to it a discharge means having a plurality of ports through which a sufficient volume of the wound irrigation solution can pass at an appropriate pressure for effective delivery of the wound irrigation solution, including the active agent, to a target site.

FIG. 1 shows an exemplary device, wherein the device contains a squeezable reservoir housing having a wall **60** that forms a reservoir that can contain therein an irrigation material including a medicinal agent (e.g., an antiseptic agent, a chlorhexidine, octenidine (e.g., a pharmaceteutically acceptable of octenidine), a synergistic combination of antiseptic agents). The reservoir housing has a mouth **62,** which communicates the reservoir to the outside of the housing. Disposed over the reservoir housing mouth, and affixed to the reservoir housing mouth is a discharge means **80, 100.**

In some embodiments, the nozzle(s) of devices described herein are specifically designed to reduce the pressure loss as the fluid (e.g., the wound irrigation solution) leaves the reservoir housing.

In some embodiments, each nozzle acts as a jet through which fluid is forced, under pressure, to achieve velocities and pressures appropriate for efficient irrigation. The nozzles are designed to reduce friction and turbulence and facilitate achieving sufficient irrigation pressures with minimal operator effort.

In certain embodiments of the devices describe herein, the nozzle is a "shaped" nozzle defined by a shaped passageway (see FIGs. 2A-2D and 3). As used herein, the "shaped passageway" extends the length of the nozzle and is defined by a cylindrical bore 98 that narrows as it approaches the outlet port **96.** The shaped passageway of the nozzle limits the generation of turbulence in the wound irrigation fluid as it passes through the nozzle(s) during the operation of the wound irrigation devices described herein. Therefore, fluid passing through the nozzle experiences laminar flow (or at least a reduction in turbulence) as it passes through and exits the nozzle. Thus, as used herein, reference to the "shaped passageway" refers to a nozzle with a passageway where the cross-sectional area of the inlet port **102** is greater than the cross-sectional area at or near the outlet port **96** wherein the inlet port is curved (not squared off), and the turbulence through the nozzle is less than the turbulence of a nozzle of the same or similar size but having a "squared-off" inlet port and/or constant diameter passageway. This shaped nozzle has been found to be particularly advantageous for achieving desired irrigation fluid pressures and velocities according to the methods and devices described herein. The description of nozzles is set forth in WO 2005/030297.

The nozzle passage area **98** is preferably defined by a funnel shape having a portion with a curved surface, where the nozzle cross-section decreases from an upstream wider end **102** to the downstream end **96.**

FIGs. 2A-2D show a specific embodiment of the elongated, shaped nozzles of a device described herein. In FIG. 2B the conical shaped nozzle is 0.2 inches long (from inlet port to outlet port).

As would be appreciated by a person skilled in the art having the benefit of the current disclosure, the nozzles of the devices described herein can be formed within the material of the discharge means. Thus, if the discharge means is formed of plastic that is sufficiently thick, then the nozzles may simply pass through the material of the discharge means. Alternatively, the nozzles may extend from either side of the discharge means.

In certain embodiments, the discharge means is detachably affixed to the reservoir housing mouth. In such embodiments, the reservoir mouth can include connecting means such as threads, snap fits, grooves, or other mechanical connection configurations for operably connecting the reservoir housing mouth to the discharge means.

The wall of the reservoir housing can be made or molded from any material that is preferably rigid enough to stand upright when the reservoir is filled with wound irrigation solution. In a typical embodiment, the reservoir housing is formed by a molded plastic, which is pliable enough so that the wall of the reservoir housing can be squeezed or compressed by hand to exert pressure on the contents of the reservoir. In some embodiments, the reservoir housing comprises a plastic material that is pliable enough to squeeze by hand and which also has sufficient resilience to return to its original shape when no longer compressed or squeezed. In some embodiments, the reservoir housing returns to the original shape very quickly when no longer compressed or squeezed.

The horizontal cross-sectional shape of the reservoir housing can be circular, square, rectangular, or other geometric shapes as desired or as already available. The walls can be tapering toward one end or the other. Alternatively, other shapes can be made for the reservoir housing according to and adapted for a particular use. For example, part of the reservoir housing wall can be slightly rounded as in a general hourglass shape and/or can be molded for ergonomics to easily fit a hand or otherwise to facilitate handling or compressing the reservoir housing. The reservoir formed by the housing can hold any volume of irrigation solution. The volume of irrigation solution can be between about 50 mL to about 2000 mL, about 100 mL to about 2000 mL, about 150 mL to about 2000 mL, about 200 mL to about 2000 mL, about 250 mL to about 2000 mL, about 300 mL to about 2000 mL, about 350 mL to about 2000 mL, about 400 mL to about 2000 mL, about 450 mL to about 2000 mL, about 500 mL to about 2000 mL, about 550 mL to about 2000 mL, about 600 mL to about 2000 mL, about 650 mL to about 2000 mL, about 700 mL to about 2000 mL, about 750 mL to about 2000 mL, about 800 mL to about 2000 mL, about 850 mL to about 2000 mL, about 900 mL to about 2000 mL, about 950 mL to about 2000 mL, about 1000 mL to about 2000 mL, about 1100 mL to about 2000 mL, about 1200 mL to about 2000 mL, about 1300 mL to about 2000 mL, about 1400 mL to about 2000 mL, about 1500 mL to about 2000 mL, about 1600 mL to about 2000 mL, about 1700 mL to about 2000 mL, about 1800 mL to about 2000 mL, about 1900 mL to about 2000 mL, about 100 mL to about 1000 mL, about 200 mL to about 1800 mL, about 300 mL to about 1600 mL, about 400 mL to about 1400 mL, or about 500 mL to about 1000 mL. The reservoir formed by the housing of devices described herein can typically hold a volume of about 100 ml to 1000 ml, preferably about 250 ml to about 750 ml, and most preferably about 500 ml. Any volume of wound irrigation solution can be applied to the wound in the methods described herein. The volume of applied wound irrigation solution can be between about 50 mL to about 2000 mL, about 100 mL to about 2000 mL, about 150 mL to about 2000 mL, about 200 mL to about 2000 mL, about 250 mL to about 2000 mL, about 300 mL to about 2000 mL, about 350 mL to about 2000 mL, about 400 mL to about 2000 mL, about 450 mL to about 2000 mL, about 500 mL to about 2000 mL, about 550 mL to about 2000 mL, about 600 mL to about 2000 mL, about 650 mL to about 2000 mL, about 700 mL to about 2000 mL, about 750 mL to about 2000 mL, about 800 mL to about 2000 mL, about 850 mL to about 2000 mL, about 900 mL to about 2000 mL, about 950 mL to about 2000 mL, about 1000 mL to about 2000 mL, about 1100 mL to about 2000 mL, about 1200 mL to about 2000 mL, about 1300 mL to about 2000 mL, about 1400 mL to about 2000 mL, about 1500 mL to about 2000 mL, about 1600 mL to about 2000 mL, about 1700 mL to about 2000 mL, about 1800 mL to about 2000 mL, about 1900 mL to about 2000 mL, about 100 mL to about 1000 mL, about 200 mL to about 1800 mL, about 300 mL to about 1600 mL, about 400 mL to about 1400 mL, or about 500 mL to about 1000 mL. In some embodiments, about 250 to about 750 mL of wound irrigation solution is applied to the wound. Advantageously, with manual compression, the devices and methods described herein can deliver 500 ml of irrigation fluid in less than 30 seconds and, typically, in 15 to 25 seconds. The fluid is delivered at about 4 to 20 psi. Lower pressures can be used for irrigating eye wounds. For irrigation of wounds in or around the eye, a pressure of about 1 psi to about 5 psi is preferred.

Further, in some embodiments, the reservoir housing comprises at one end a neck portion formed at the mouth of the reservoir housing. The neck portion of the reservoir housing is generally at least slightly smaller in cross sectional area than the reservoir housing. The reservoir housing neck is preferably integrally molded with the reservoir housing but can be formed or molded separately and affixed to the mouth of the reservoir housing. The material used for the neck portion of the reservoir housing can be the same as the material used to make the reservoir housing cylinder. Alternatively, the neck portion can be a different material, for example, a more rigid or sturdy material than the compressible material forming the reservoir housing wall. For example, the material used to make the neck portion can be a metal or a hard plastic, or the like.

With reservoir housing embodiments that include a neck portion, the discharge means is typically disposed over and affixed to the neck portion. In a related embodiment, the neck portion of the reservoir housing can include a connecting means for detachably affixing a discharge means thereto. The connecting means can include threads, latches, grooves, or other mechanical connection configurations for operably connecting the neck portion to the discharge means. The connecting means can be on the outer face of the neck portion, forming a male connecting end, or can be on the inner face forming a female connecting end of the neck portion.

In some embodiments, the discharge means has a plurality of nozzles **70** whereby the wound irrigation solution in the reservoir passes through in a pressurized and directional manner. A backsplash shield **90** can also be provided either with the reservoir housing or with the discharge means.

The back-splash protective shield protects the health care professional (or other user) from back-splash of human and or animal body fluids that are mixed with and splashed from the wound when the wound is contacted by the discharged wound irrigation solution.

As used herein, reference to a "dispersed" stream of solution (e.g., wound irrigation solution) means that the area from which the stream emanates, or the area which it contacts, is larger than that which can be achieved using a typical syringe for irrigation. A typical syringe, as is well known in the art can be, for example, a 16- or 18-gauge syringe. In one embodiment, the dispersed stream can be achieved using multiple nozzles. The nozzles can be presented in a variety of patterns on a discharge means, such as a circular or square pattern or sizes.

In certain embodiments, the discharge means is designed with connecting means that are threads or grooves, which allow for complementary attachment to currently available irrigation solution bottles. Thus, the discharge means of

the devices described herein can be interchangeable, when desired, with the screw-cap that is provided with an irrigation solution bottle as are available. The screw-top design of the discharge means provides the operator with the option of using the reservoir housing with the nozzles of the devices described herein or to threadably remove the discharge means and pour out or change the irrigation solution.

Each of the nozzles of the discharge means can be of any desirable size, preferably less than one-eighth inch in diameter and having a size between about a 10-gauge hypodermic needle and about a 30-gauge needle, and most preferably having a size ranging from that of a 16-gauge needle to a 25-gauge needle. Specific dimensions and shapes are shown in FIGs. 2A-2D. The outlet port **96** may have, for example, an inner diameter of about 0.02 to about 0.07 inches. For the venturi shaped nozzle (FIG. 3), the diameter of the inlet port **102** (proximal to the reservoir) can be, for example, from about 0.05 to about 0.30 inches, or more.

Each of the nozzles can be the same size or the nozzles can be different sizes and shapes. The different sizes of nozzles allow for the liquid (e.g., the wound irrigation solution) to be expelled from the discharge means at different pressures. For example, the 16-gauge nozzle allows for a stream having about 6 psi pressure when the device is squeezed by the normal adult; the 25-gauge nozzle provides a pressure of up to about 20 psi from each nozzle.

The shaped nozzles of the devices described herein have the added advantage when compared to other nozzles in that little or no release of irrigation material is permitted without pressure being applied to the irrigation material. For example, if a reservoir housing with shaped nozzles is tipped onto its side or even held upside-down with gravitational pull on the irrigation material through the discharge means, there will be little or no release of irrigation material through the shaped nozzles.

In some embodiments, the discharge means **70** comprises four nozzles. Additionally, to discharge the wound irrigation solution at appropriate pressure, the diameter of the nozzles can be about 0.02 to 0.07 inches in diameter.

From the description of the device herein above, a method of using the device would readily be understood and adaptable by those persons having ordinary skill in the art. The reservoir housing and contents can be stored in a sterile environment, e.g., sterile packaging which is opened immediately prior to use. The reservoir housing can be directed towards the wound and squeezed or compressed to expel or discharge the wound irrigation solution in the desired direction, and at the desired pressure to effect irrigation of a wound to remove contaminants or debris and to deliver the active agent(s). See also the Example 1, provided below.

It would also be understood that the described discharge means can be packaged separately from the reservoir housing. The discharge means is packaged in a sterile environment. In one embodiment, the drug delivery device is provided in a sterile laceration tray. The laceration tray has, in addition to the drug delivery device described herein, other items conveniently provided for treating wounds. Contemplated items that can be included in a laceration tray include, but are not limited to, needle holders (e.g., 5" floor-grade smooth); scissors (e.g., 4.5" floor-grade straight Iris scissors); hemostats (e.g., 5" floor-grade curved mosquito hemostat); forceps (e.g., floor-grade tissue forceps with 1×2 teeth); cups (e.g., 2 oz. medicine cups); syringes (e.g., 10 cc Luer Lock syringe); needles (e.g., 25 gauge×⅝" needle; 27 gauge×1.5" needle; 18 gauge×1.5" needle); dressings (e.g., gauze dressings); drapes (e.g., polylined fenestrated drapes); and towels (e.g., absorbent towels).

In a method of use, where a reservoir housing **60** having discharge means **70** affixed thereto is provided. The discharge means **70** is directed towards the wound, and the reservoir housing **60** is compressed, discharging the wound irrigation solution through the discharge means **70.** The wound irrigation solution can be discharged at a range of pressures of about 4-20 $lbs/in^2$, with a preferred pressure of about 7 psi.

The reservoir housing **60** can be compressed manually or via other mechanical means. For example, the operator may compress the reservoir housing either one hand or two hands, to provide increased pressure (e.g., 16 psi). Alternatively, a pressure means can be activated to generate a dispersed stream of wound irrigation solution through the discharge means.

In another method of use, where a reservoir housing **60** and discharge means **70** are provided separately, the discharge means is affixed to the mouth or neck portion of the reservoir housing via complementary connecting means. After the discharge means is affixed to the reservoir housing, the discharge means is directed towards the target site, and the reservoir housing is compressed to discharge a dispersed stream of wound irrigation solution through the nozzles of the discharge means.

Significantly, it is known that more force is required to rid the wound of particles with a small surface area (e.g., bacteria) than to remove particles with a large surface area (e.g., dirt, sand, or vegetation). Minimum recommended volumes of wound irrigation solution vary, but for a moderately sized potentially contaminated wound, for example a laceration 3-6 cm long and less than 2 cm deep, at least 200 to 500 ml, or more should be used. Greater volumes, on the order of one to two liters, may be required for larger or heavily contaminated wounds. Irrigation should continue at least until all visible, loose particulate matter has been removed.

## Properties of Wound Healing

Devices and methods described herein can improve wound healing and properties (e.g., contributing factors, parameters) of wound healing. As demonstrated in Example 7, methods of treatment utilizing synergistic combinations of antiseptic agents (e.g., a chlorhexidine and octenidine or a pharmaceutically acceptable salt thereof) can improve wound healing. Wound healing can be improved in comparison to irrigation of a wound with an analogous method. Such an analogous method can contain the same steps of a method described herein, but differ by the composition of the wound irrigation solution (e.g., utilize an alternative wound irrigation solution). An alternative wound irrigation solution used in an analogous method can be a non-synergistic combination of antiseptic agents (e.g., a solution that contains two or more antiseptic agents that do not act in synergy with each other or do not act in synergy with each other at the concentration present in the solution). An alternative wound irrigation solution can be a solution without an antiseptic agent. An alternative wound irrigation solution can consist of water (e.g., sterile water, water without an antiseptic agent). An alternative wound irrigation solution can be a saline solution (e.g., 0.9% w/w saline, saline without an antiseptic agent). An alternative wound irrigation solution can be a solution containing only one antiseptic agent. An alternative wound irrigation solution can be a solution containing only one antiseptic agent present at the same concentration as any antiseptic present in any synergistic combination of antiseptic agents in an irrigation solution described herein. Wound healing can be improved in comparison to treatment with an alternative wound irrigation solution. Wound healing can be improved in comparison to treatment with a saline solution (e.g., 0.9% w/w saline solution). Wound healing can be improved in comparison to treatment with water (e.g., sterile water, an aqueous solution devoid of antiseptic agent). Wound healing can be improved in comparison to treatment with a solution containing only one antiseptic agent (e.g., not a synergistic mixture of antiseptic agents). Wound healing can be improved in comparison to treatment with a solution containing chlorhexidine at the same concentration as the synergistic combination of antiseptic agents in a wound irrigation solution but without an additional antiseptic agent. Wound healing can be improved in comparison to treatment with a solution containing octenidine at the same concentration as the synergistic combination of antiseptic agents in a wound irrigation solution but without an additional antiseptic agent.

Any property of wound healing can be improved, including but not limited to wound bed scoring, exudate, wound microorganism burden, or combinations of any of the foregoing. Wound bed scoring can be a score of the moisture level of a wound (e.g., a high score can be indicative of a severely moist wound and a low score can be indicative of a dry wound). Methods and devices herein can reduce the moisture level of a wound bed. Methods and devices described herein can reduce the wound bed score by any value, such as reduction of the wound bed score value by about 1, about 2, or about 3. The wound bed score can be reduced by a value between about 1 to about 3, about 1 to about 2, or about 2 to about 3. The wound bed score can be reduced by at least about 1, at least about 2, or at least about 3. Exudate is the mass of cells and fluid that is seeping out of a wound. Exudate can be a scored by the amount of exudate present in a wound (e.g., a high score can be indicative of a severe or high amount of exudate and a low score can be indicative of no or a low amount of exudate from a wound). Methods and devices herein can reduce the amount of exudate from a wound. Methods and devices described herein can reduce the exudate score by any value, such as reduction of the exudate score value by about 1, about 2, or about 3. The exudate score can be reduced by a value between about 1 to about 3, about 1 to about 2, or about 2 to about 3. The exudate score can be reduced by at least about 1, at least about 2, or at least about 3. As described herein, methods and devices can reduce the microorganism burden of a wound. The microorganism burden (e.g., the burden of any microorganism described herein, a bacteria, a virus, a fungus) can be reduced by any value.

Practicing methods and using devices disclosed herein can improve the healing of any tissue of a wound, including but not limited to the epidermis and/or dermis of a wound. As demonstrated in Example 7, treatment of wounds with a wound irrigation solution containing a synergistic combination of antiseptic agents (e.g., chlorhexidine and octenidine) improves the healing of the dermis and epidermis of a wound (FIGs. 6A-6C) in comparison to treatment of wounds with an alternative wound irrigation solution such as a saline solution (e.g., 0.9% w/w saline, FIGs. 4A-4C) or a solution containing only one antiseptic agent (e.g., 0.05% w/w chlorhexidine FIGs. 5A-5C) at the same concentration as the synergistic combination without an additional antiseptic. In some embodiments, the method improves a property of wound healing compared to irrigation of the wound with an analogous method (e.g., an analogous method using an alternative wound irrigation solution). In some embodiments, the alternative wound irrigation solution consists of a saline solution. In some embodiments, the alternative wound irrigation solution consists of chlorhexidine at the same concentration (e.g., the same concentration as the synergistic combination present in the wound irrigation solution) without an additional antiseptic agent. In such embodiments, the property of wound healing can be wound bed scoring, exudate, wound microorganism burden, or a combination of any of the foregoing.

Following are examples that illustrate procedures for practicing the methods described herein.

## EXAMPLE 1

Methods of Irrigation

When a patient presents a wound to a medical or other health care professional skilled in the art, that medical professional assesses the extent of the injury sustained by the patient, including all other life-threatening injuries. Appropriate action regarding these life-threatening injuries is performed and a history is recorded. All wounds are covered to minimize further contamination until the actual repair process begins.

For examination of the wound, it is assumed that a medical professional would have performed a detailed evaluation of the extent of tissue injury, including but not limited to anatomical area considerations, depth of the wound, type of injury, e.g., crash injury, puncture wound, bites, missiles, cuts with sharp objects, or the like. Included in this examination would be a determination of the type(s) of contamination, time elapsed between the occurrence of the injury to presentation, gross contamination of a wound, and other medical factors associated with an increase incidence of infection (for example, diabetics, AIDS patients, and chemotherapeutic patients).

The wound and surrounding tissue, at the option of the health care professional, could be anesthetized using topical, local, or general anesthetics before the wound-cleansing method begins. Alternatively, an anesthetic may be delivered using the devices and methods described herein.

In one embodiment, a device described herein has a discharge means affixed to a reservoir housing. In some embodiments, the devices described herein can be held in either hand as preferred by the user. Normally, it would be held in the dominant hand in a bottle-holding fashion. This allows the medical care professional to gently open the wound if needed, with the opposite hand, preferably protected by a sterile glove, to expose the depths of the wound.

Once the depths of the wound have been exposed, the end of the reservoir housing having the discharge means affixed thereto is directed towards the wound. Manual or mechanically produced pressure is applied to the reservoir housing to expel the wound irrigation solution with active agent through the nozzles of the discharge means. The wound should be irrigated in this fashion until all visible evidence of contamination has been removed. A potentially contaminated wound of any size should be irrigated with a minimum of 150-300 ml of wound irrigation solution. Heavily contaminated or larger wounds may require 2-3 liters of wound irrigation solution. The health care professional could vary the angle of the discharged wound irrigation solution from the discharge means in reference to the wound to further assist with the dislodgement of contaminants.

Following an initial irrigation of the wound, a reexamination of the wound should be undertaken. The wound should be explored to its base to ascertain that no visible foreign bodies or contaminants remain. If foreign bodies or contaminants are found, the irrigation process should be repeated followed by a re-examination. This may continue for several cycles.

Once irrigation has been completed, e.g., no visible contaminants remain, the damaged tissue would be repaired in a standard accepted fashion.

Any skin wounds such as cuts, scrapes, surgical wounds, punctures, and abrasions and the like are suitable for irrigation according to the devices and methods described herein.

EXAMPLE 2

Routes of Administration

Table 1 provides a listing of various routes of administration that can be used according to the methods and devices described herein.

TABLE 1

| Routes of Administration | |
|---|---|
| Delivery Route | Description |
| AURICULAR (OTIC) | Administration to or by way of the ear. |
| BUCCAL | Administration directed toward the cheek, generally from within the mouth. |
| CONJUNCTIVAL | Administration to the conjunctiva, the delicate membrane that lines the eyelids and covers the exposed surface of the eyeball. |
| CUTANEOUS | Administration to the skin. |
| DENTAL | Administration to a tooth or teeth. |
| ENDOCERVICAL | Administration within the canal of the cervix uteri. |

(continued)

| Routes of Administration | |
|---|---|
| Delivery Route | Description |
| ENDOSINUSIAL | Administration within the nasal sinuses of the head. |
| ENDOTRACHEAL | Administration directly into the trachea. |
| ENTERAL | Administration directly into the intestines. |
| EPIDURAL | Administration upon or over the dura mater. |
| EXTRA-AMNIOTIC | Administration to the outside of the membrane enveloping the fetus |
| EXTRACORPOREAL | Administration outside of the body. |
| INFILTRATION | Administration that results in substances passing into tissue spaces or into cells. |
| INTERSTITIAL | Administration to or in the interstices of a tissue. |
| INTRA-ABDOMINAL | Administration within the abdomen. |
| INTRA-ARTICULAR | Administration within a joint. |
| INTRABILIARY | Administration within the bile, bile ducts or gallbladder. |
| INTRABRONCHIAL | Administration within a bronchus. |
| INTRACARDIAC | Administration with the heart. |
| INTRACARTILAGINOUS | Administration within a cartilage; endochondral. |
| INTRACAVERNOUS | Administration within a pathologic cavity, such as occurs in the lung in tuberculosis. |
| INTRACAVITARY | Administration within a non-pathologic cavity, such as that of the cervix, uterus, or penis, or such as that which is formed as a result of a wound. |
| INTRACEREBRAL | Administration within the cerebrum. |
| INTRACORPORUS CAVERNOSUM | Administration within the dilatable spaces of the corpus cavernosa of the penis. |
| INTRADUCTAL | Administration within the duct of a gland. |
| INTRADUODENAL | Administration within the duodenum. |
| INTRADURAL | Administration within or beneath the dura. |
| INTRAESOPHAGEAL | Administration within the esophagus. |
| INTRAGASTRIC | Administration within the stomach. |
| INTRAILEAL | Administration within the distal portion of the small intestine, from the jejunum to the cecum. |
| INTRALESIONAL | Administration within or introduced directly into a localized lesion. |
| INTRALUMINAL | Administration within the lumen of a tube. |
| INTRALYMPHATIC | Administration within the lymph. |
| INTRAMEDULLARY | Administration within the marrow cavity of a bone. |
| INTRAMENINGEAL | Administration within the meninges (the three membranes that envelope the brain and spinal cord). |
| INTRAOCULAR | Administration within the eye. |
| INTRAOVARIAN | Administration within the ovary. |
| INTRAPERICARDIAL | Administration within the pericardium. |
| INTRAPERITONEAL | Administration within the peritoneal cavity. |
| INTRAPLEURAL | Administration within the pleura. |

(continued)

| Routes of Administration | |
|---|---|
| Delivery Route | Description |
| INTRAPROSTATIC | Administration within the prostate gland. |
| INTRAPULMONARY | Administration within the lungs or its bronchi. |
| INTRASINAL | Administration within the nasal or periorbital sinuses. |
| INTRASPINAL | Administration within the vertebral column. |
| INTRASYNOVIAL | Administration within the synovial cavity of a joint. |
| INTRATENDINOUS | Administration within a tendon. |
| INTRATESTICULAR | Administration within the testicle. |
| INTRATHECAL | Administration within the cerebrospinal fluid at any level of the cerebrospinal axis, including injection into the cerebral ventricles. |
| INTRATHORACIC | Administration within the thorax (internal to the ribs); synonymous with the term endothoracic. |
| INTRATUBULAR | Administration within the tubules of an organ. |
| INTRATUMOR | Administration within a tumor. |
| INTRATYMPANIC | Administration within the auris media. |
| INTRAUTERINE | Administration within the uterus. |
| INTRAVESICAL | Administration within the bladder. |
| INTRAVITREAL | Administration within the vitreous body of the eye. |
| IRRIGATION | Administration to bathe or flush open wounds or body cavities. |
| LARYNGEAL | Administration directly upon the larynx. |
| NASAL | Administration to the nose; administered by way of the nose. |
| NASOGASTRIC | Administration through the nose and into the stomach, usually by means of a tube. |
| OCCLUSIVE DRESSING TECHNIQUE | Administration by the topical route which is then covered by a dressing which occludes the area. |
| OPHTHALMIC | Administration to the external eye. |
| ORAL | Administration to or by way of the mouth. |
| OROPHARYNGEAL | Administration directly to the mouth and pharynx. |
| PERCUTANEOUS | Administration through the skin. |
| PERIARTICULAR | Administration around a joint. |
| PERIDURAL | Administration to the outside of the dura mater of the spinal cord. |
| PERINEURAL | Administration surrounding a nerve or nerves. |
| PERIODONTAL | Administration around a tooth. |
| RECTAL | Administration to the rectum. |
| RESPIRATORY (INHALATION) | Administration within the respiratory tract by inhaling orally or nasally for local or systemic effect. |
| RETROBULBAR | Administration behind the pons or behind the eyeball. |
| SOFT TISSUE | Administration into any soft tissue. |
| SUBARACHNOID | Administration beneath the arachnoid. |
| SUBCONJUNCTIVAL | Administration beneath the conjunctiva. |

(continued)

| Routes of Administration | |
|---|---|
| Delivery Route | Description |
| SUBCUTANEOUS | Administration beneath the skin; hypodermic. Synonymous with the term SUBDERMAL. |
| SUBLINGUAL | Administration beneath the tongue. |
| SUBMUCOSAL | Administration beneath the mucous membrane. |
| TOPICAL | Administration to a particular spot on the outer surface of the body. The E2B term TRANSMAMMARY is a subset of the term. |
| TRANSMUCOSAL | Administration across the mucosa. |
| TRANSPLACENTAL | Administration through or across the placenta. |
| TRANSTRACHEAL | Administration through the wall of the trachea. |
| TRANSTYMPANIC | Administration across or through the tympanic cavity. |
| URETERAL | Administration into the ureter. |
| URETHRAL | Administration into the urethra. |
| VAGINAL | Administration into the vagina. |

EXAMPLE 3

Time-kill Evaluation of Antiseptic Formulations

An *in-vitro* time-kill experiment was conducted to evaluate the broad-spectrum antimicrobial properties of antiseptic formulations containing chlorhexidine gluconate and octenidine dihydrochloride. The procedure for the experiment was based on the methodology described in ASTM E2783-22, *Standard Test Method for Assessment of Antimicrobial Activity for Water Miscible Compounds Using a Time-Kill Procedure.* The formulations were tested against *C. albicans* (ATCC 10231), *E. coli* (ATCC BAA-2469), *R. pickettii* (ATCC 27511), *S. aureus* (BSLI 092920MRSA10), and *S. epidermidis* (ATCC 12228). The log10 reduction versus microorganism in the absence of antiseptic formulation was determined following exposure of the microorganism to the antiseptic formulation for 1 minute, 5 minutes, 15 minutes, and 30 minutes. The assay was performed in duplicate. The antiseptic formulation was formulation 1 (chlorhexidine gluconate (500 μg/mL) and octenidine dihydrochloride (3.75 μg/mL) in sterile water). The formulation (chlorhexidine gluconate (500 μg/mL) in sterile water (no octenidine present)) was previously tested and the test data was compared to the data for Formulation 1.

Approximately 48 hours prior to testing, inocula from lyophilized vials or cryogenic cultures containing a test organism (e.g., *C. albicans, E. coli, R. pickettii, S. aureus, S. epidermidis*) were suspended in sterile sodium chloride irrigation solution (0.9% w/w sodium chloride in water) and inoculated onto the surface of a suitable agar contained in Petri dishes. Suitable agar included Sabouraud dextrose agar (SDA, for *C. albicans*), tryptic soy agar (TSA, for *E. coli, R. pickettii, S. aureus,* and *S. epidermidis*). Inoculated agar plates were incubated at a suitable temperature (i.e., *C. albicans* was incubated at 30 ± 2 °C, bacteria were incubated at 35 ± 2 °C) for about 24 hours. Following incubation, a suspension of each test organism was prepared by removing growth from the inoculated agar plates and suspending in sterile sodium chloride irrigation solution. The resulting inoculated sodium chloride irrigation solution was transferred to a suitable agar plate and incubated at a suitable temperature as previously indicated.

The initial population of the inoculated sodium chloride irrigate solution was determined by plating at 10-fold dilutions (0.1 mL) on an agar plate and incubating at a suitable temperature for the test organism. An aliquot of the of the inoculated sodium chloride irrigate solution containing no less than 1 x $10^8$ colony forming units per mL (CFU/mL) was transferred to a tube containing the antiseptic formulation (10 mL). The resulting solution was mixed thoroughly by a vortex mixer and/or a positive displacement pipettor. The test organism was incubated with the antiseptic formulation for 1 minute, 5 minutes, 15 minutes, or 30 minutes. Following the incubation period with antiseptic formulation, an aliquot (1.0 mL) of the resulting solution was transferred to a sterile test tube containing the neutralizer solution (9.0 mL,) and mixed thoroughly using a vortex mixer. The neutralizer solution was Butterfield's phosphate buffer solution with product neutralizers (BBP++). Ten-fold dilutions of the suspensions of each test organism were prepared in the neutralizer solution by mixing thoroughly using a vortex mixer between each dilution. The resulting diluted solutions were aliquoted (0.1 mL to 1.0 mL) onto suitable agar plates (containing neutralizer, e.g., SDA with neutralizer (SDA+), TSA with neutralizer (TSA+)) and

incubated as outlined above. Following incubation, the colonies on the agar plates were manually counted using a hand-tally counter. Counts in the range of 25 to 250 CFU were used in the data calculations. If no counts in this range were observed, only agar plates with the closest colony counts to 25 to 250 CFU were used for data calculations.

The results of this experiment are shown in Tables 2 and 3. Briefly, the addition of octenidine dihydrochloride in combination with chlorhexidine gluconate reduced the exposure time needed to obtain maximum CFU reduction (as observed by logarithmic base ten reduction of the post-exposure population versus the initial population). The formulation comprising chlorhexidine gluconate (500 μg/mL) and octenidine dihydrochloride (3.75 μg/mL) led to a large reduction of exposure time required for maximal growth reduction of *E. coli, R. pickettii,* and *S. aureus* MRSA compared to the formulation comprising chlorhexidine gluconate as the sole antiseptic agent. Under the test conditions, the formulations comprising chlorhexidine (500 μg/mL) and octenidine (3.75 μg/mL) achieved maximum possible growth reduction of both *R. pickettii* and *S. aureus* MRSA compared to the formulation comprising chlorhexidine gluconate as the sole antiseptic agent.

Table 2. Time-kill Experimental Results for Chlorhexidine (500 μg/mL) and Octenidine Dihydrochloride (3.75 μg/mL) Formulation

| Microorganism Species | Exposure Time (minutes) | $Log_{10}$ Reduction* |
|---|---|---|
| *C. albicans* (ATCC 10231) | 1 | 1.26 |
| | 5 | 4.24 |
| | 15 | 4.87 |
| | 30 | >5.40 |
| *E. coli* (ATCC BAA-2469) | 1 | 4.01 |
| | 5 | >5.26 |
| | 15 | >5.26 |
| | 30 | >5.26 |
| *R. pickettii* (ATCC 27511) | 1 | 1.01 |
| | 5 | 2.57 |
| | 15 | >3.27 |
| | 30 | >3.27 |
| *S. aureus* MRSA (BSLI 092920MRSA10) | 1 | 1.81 |
| | 5 | 4.68 |
| | 15 | >5.14 |
| | 30 | >5.14 |
| *S. epidermidis* (ATCC 12228) | 1 | 5.50 |
| | 5 | >5.59 |
| | 15 | >5.59 |
| | 30 | >5.59 |
| * Represent average values. | | |

Table 3. Time-kill Experimental Results for Chlorhexidine (500 μg/mL) Only Formulation

| Microorganism Species | Exposure Time (minutes) | $Log_{10}$ Reduction* |
|---|---|---|
| *C. albicans* (ATCC 10231) | 1 | 3.7 |
| | 5 | 5.4 |
| | 15 | |
| | 30 | 6.6 |

(continued)

| Microorganism Species | Exposure Time (minutes) | $Log_{10}$ Reduction* |
|---|---|---|
| *E. coli* (ATCC BAA-2469) | 1 | 3.2 |
| | 5 | 5.1 |
| | 15 | 6.5 |
| | 30 | 6.5 |
| *R. pickettii* (ATCC 27511) | 1 | <0.3 |
| | 5 | |
| | 15 | <0.3 |
| | 30 | <0.3 |
| *S. aureus* MRSA (BSLI 092211MRSA1) | 1 | 0.6 |
| | 5 | 1.4 |
| | 15 | 3.1 |
| | 30 | 4.2 |
| *S. epidermidis* (ATCC 12228) | 1 | 4.3 |
| | 5 | 5.6 |
| | 15 | 5.6 |
| | 30 | 5.6 |
| * Represent average values. | | |

Example 4

Minimum Inhibitory Concentration Determination for Antiseptics

The minimal inhibitory concentration (MIC, the minimum inhibitory concentration required for 90% growth reduction) of octenidine was determined against *S. aureus* (ATCC 6538), *P. aeruginosa* (ATCC 15442), and *E. coli* (ATCC BAA 1427) by serial 2-fold dilution experiments. A solution of chlorhexidine (0.025% w/w) was used as a positive control and comparator. Experiments were performed in triplicate and the average result is shown in Table 4. The MIC of octenidine that inhibited greater than 90% growth of *S. aureus, P. aeruginosa,* and *E. coli* was 0.9375 μg/mL, 1.875 μg/mL, and 1.875 μg/mL, respectively. The control chlorhexidine (0.025% w/w) showed an average inhibition of 121%, 81%, and 63% for *S. aureus, P. aeruginosa,* and *E. coli,* respectively. Overall, the MIC of octenidine across all organisms tested (i.e., *S. aureus, P. aeruginosa,* and *E. coli*) ranged between 0.9375 μg/mL to 1.875 μg/mL.

Table 4. Average Growth Inhibition Testing for Antiseptic Agents

| Antiseptic Agent | Microorganism | Concentration (μg/mL) | Average Growth Inhibition (%) | Standard Deviation |
|---|---|---|---|---|
| Octenidine dihydrochloride | *S. aureus* (ATCC 6538) | 60 | 73 | 0.0085 |
| | | 30 | 92 | 0.0016 |
| | | 15 | 97 | 0.0017 |
| | | 7.5 | 98 | 0.0394 |
| | | 3.75 | 101 | 0.0004 |
| | | 1.875 | 101 | 0.0036 |
| | | 0.9375 | 101 | 0.0011 |
| | | 0.46875 | 50 | 0.8068 |
| Chlorhexidine gluconate | | 250 | 121 | 0.101 |

(continued)

| Antiseptic Agent | Microorganism | Concentration (μg/mL) | Average Growth Inhibition (%) | Standard Deviation |
|---|---|---|---|---|
| Octenidine dihydrochloride | *P. aeruginosa* (ATCC 15442) | 60 | 91 | 0.0064 |
| | | 30 | 97 | 0.0022 |
| | | 15 | 99 | 0.0007 |
| | | 7.5 | 100 | 0.0006 |
| | | 3.75 | 100 | 0.0003 |
| | | 1.875 | 100 | 0.0005 |
| | | 0.9375 | 4 | 0.0127 |
| | | 0.46875 | 2 | 0.0272 |
| Chlorhexidine gluconate | | 250 | 81 | 0.0301 |
| Octenidine dihydrochloride | *E. coli* (ATCC BAA 1427) | 60 | 91 | 0.0011 |
| | | 30 | 97 | 0.0033 |
| | | 15 | 99 | 0.0009 |
| | | 7.5 | 100 | 0.0018 |
| | | 3.75 | 100 | 0.0003 |
| | | 1.875 | 100 | 0.0004 |
| | | 0.9375 | 12 | 0.0198 |
| | | 0.46875 | 6 | 0.0096 |
| Chlorhexidine gluconate | | 250 | 63 | 0.116 |

Example 5

Minimal Bactericidal Concentration Determination for Antiseptics

The minimal bactericidal concentration (MBC) of octenidine dihydrochloride was determined against *S. aureus* (ATCC 6538), *P. aeruginosa* (ATCC 15442), and *E. coli* (ATCC BAA 1427) by serial 2-fold dilution experiments. A solution of chlorhexidine (0.025% w/w) was used as a positive control and comparator. Experiments were performed in triplicate and the average result is shown in Table 5. *S. aureus, P. aeruginosa,* and *E. coli* had octenidine dihydrochloride concentration MBC values (defined as a concentration of antiseptic agent having less than 500 CFU/mL) of 0.9375 μg/mL, 3.75 μg/mL, and 1.875 μg/mL, respectively. Overall, the MBC values ranged between 7.5 μg/mL to 60 μg/mL for all tested organisms. This result indicates a great variability in susceptibility to killing (e.g., MBC value variability) by octenidine dihydrochloride between different bacterial species. The control chlorhexidine (0.025% w/w) showed no colonies (e.g., above MBC value for chlorhexidine) for all test organisms.

Table 5. Average Bactericidal Testing for Antiseptic Agents

| Antiseptic Agent | Microorganism | Concentration (μg/mL) | Number of Colonies* | Standard Deviation | CFU/mL |
|---|---|---|---|---|---|
| Octenidine dihydrochlorid e | *S. aureus* (ATCC 6538) | 60 | 0 | 0 | 0 |
| | | 30 | 0 | 0 | 0 |
| | | 15 | 0 | 0 | 0 |
| | | 7.5 | 0 | 0 | 0 |
| | | 3.75 | 5.6667 | 9.815 | 566.67 |
| | | 1.875 | 0 | 0 | 0 |
| | | 0.9375** | 0 | 0 | 0 |
| | | 0.46875 | Lawn | Lawn | Lawn |
| Chlorhexidine gluconate | | 250 | 0 | 0 | 0 |
| Octenidine dihydrochlorid e | *P. aeruginosa* (ATCC 15442) | 60 | 0 | 0 | 0 |
| | | 30 | 0 | 0 | 0 |
| | | 15 | 0 | 0 | 0 |
| | | 7.5 | 0 | 0 | 0 |
| | | 3.75** | 0 | 0 | 0 |
| | | 1.875 | 9.667 | 4.04 | 966.67 |
| | | 0.9375 | Lawn | Lawn | Lawn |
| | | 0.46875 | Lawn | Lawn | Lawn |
| Chlorhexidine gluconate | | 250 | 0 | 0 | 0 |
| Octenidine dihydrochlorid e | *E. coli* (ATCC BAA 1427) | 60 | 0 | 0 | 0 |
| | | 30 | 0 | 0 | 0 |
| | | 15 | 0 | 0 | 0 |
| | | 7.5 | 0 | 0 | 0 |
| | | 3.75 | 0 | 0 | 0 |
| | | 1.875** | 0 | 0 | 0 |
| | | 0.9375 | Lawn | Lawn | Lawn |
| | | 0.46875 | Lawn | Lawn | Lawn |
| Chlorhexidine gluconate | | 250 | 0 | 0 | 0 |
| * Number of colonies is average over triplicate experiments. 'Lawn' indicates too many colonies to accurately count (e.g., greater than 250 colonies on an agar plate).<br>** Indicates an MBC value of less than 500 CFU/mL. | | | | | |

Example 6

Fractional Inhibitory Concentration (FIC) and Synergy Analysis of Antiseptic Agents

Chlorhexidine gluconate (CHG) and octenidine dihydrochloride (OCT) were analyzed by chequerboard assay with each test organism to determine potential additive, synergistic, and antagonistic activity. The test organisms *S. aureus, P. aeruginosa,* and *E. coli* were tested at ranges of chlorhexidine gluconate (0.122 - 15.625 μg/mL, 1.25 - 160 μg/mL, and 0.125-16 μg/mL, respectively) and octenidine dihydrochloride (0.029 - 3.75 μg/mL, 0.029 - 3.75 μg/mL, and 0.029 - 3.75 μg/mL, respectively). The results of the chequerboard experiment are shown in Table 6. FIC was calculated by the following Equation 2:

$$FIC = \frac{OCT\ MIC\ in\ combination\ with\ CHG}{OCT\ MIC} + \frac{CHG\ MIC\ in\ combination\ with\ OCT}{OCT\ MIC} \text{ (Equation 2).}$$

The results of the synergy analysis (displayed in Table 6) show that the combination of octenidine and chlorhexidine is synergistic in the inhibition of E. coli as indicated by an FIC of 0.515 (e.g., an FIC equal to or less than about 0.5).

Table 6. Synergy Analysis of Octenidine and Chlorhexidine Gluconate

| **Test Organism** | **MIC of CHG Alone (μg/mL)** | **MIC of OCT Alone (μg/mL)** | **MIC of CHG in Presence of OCT (μg/mL)** | **MIC of OCT in Presence of CHG (μg/mL)** | **Fractional Inhibitory Concentration (FIC)** |
|---|---|---|---|---|---|
| *S. aureus* | 4 | 0.9375 | 2 | 0.234 | 0.750 |
| *P. aeruginosa* | 32 | 1.875 | 16 | 0.9375 | 1.000 |
| *E. coli* | 16 | 1.875 | 8 | 0.029 | 0.515 |

Example 7

In vivo Delayed Wound Healing Model

Wound lavage treatments of chlorhexidine gluconate (0.05% w/w) were evaluated against 1) a solution containing both chlorhexidine gluconate (0.05% w/w) and octenidine dihyrdrochloride (3.75 μg/mL) and 2) saline (0.9% w/w) treatments. A solution containing both chlorhexidine gluconate (0.05% w/w) and octenidine dihydrochloride (3.75 μg/mL) was made by dissolving octenidine dihydrochloride in an aqueous solution of chlorhexidine in sterile glass vials (10 mL). All dosing formulations were prepared prior to the study start and stored as daily aliquots at 4 °C. An aliquot of each formulation was removed from 4 °C each day of the study and allowed to warm to room temperature prior to administration. Sterile 0.9% w/w saline was stored at ambient temperature in sterile 10 mL vials. All treatment administrations were conducted in a biosafety cabinet.

Thirty (30), 9-week-old, male BKS.Cg-Dock7m +/+ Leprdb/J diabetic mice were obtained from Jackson Laboratories. Animals were allowed to acclimate for six days prior to wound creation and study initiation. The study design is shown in Table 7. The duration of the study was 21 days. Health checks on animals were completed at least once daily for general health, mortality and moribundity.

Table 7. In vivo Delayed Wound Healing Model Study Design

| Study Group (n = 6/group) | Wound Creation | Treatment (up to 0.5 mL) | Conc. (μg/mL) | Dose Route | Study End Point |
|---|---|---|---|---|---|
| 1 | On Day 0, skin biopsy (10 mm) was be removed from the dorsal right side | 0.9% w/w saline (negative control) | NA | Applied topically, up to 500 μL, on Days 3, 7, 10, 14, and 17, plus Tegaderm™ | Day 21 |
| 2 | | 0.05% w/w CHG | 500 | | |
| 3 | | OCT (3.75 μg/mL) + 0.05% w/w CHG | 3.75 | | |

All study animals had wounds debrided on study day 14 by sharp debridement utilizing surgical blades (No. 22 blade). All wounds were measured by digital calipers both pre- and post-debridement. On day 14, all wound assessments were completed post-debridement.

*Wound Creation-* On study day 0, animals were anesthetized using isoflurane on $O_2$ (1-4%) and placed on their abdomens. Sterile, 10-mm disposable skin biopsy punches were used to remove a single disk of skin from the dorsal right side of shaved animals. A 5 mm 3D printed biofilm (containing *S. aureus* and *P. aeruginosa*) was placed in each wound. The wound was covered with Tegaderm™ (transparent film dressing, 3M Company) and the biofilm was allowed to incorporate into the wound until treatment initiation (day 3). Animals were provided supplement heat until recovered from anesthesia

and returned to their cages. Treatments (up to 500 μL of formulation and Tegaderm™) were administered from day 3 until study conclusion.

*Treatments/Dose Regimen-* Treatments and control administrations were performed under anesthesia. Animals were administered treatments or control on study days 3, 7, 10, 14, and 17 (i.e., twice per week) by topical administration at a dose volume of 500 μL/animal as outlined in Table 7. Treatments groups included: Group 1 (0.9% w/w saline), Group 2 (0.05% chlorhexidine solution), and Group 3 (the combination formulation containing both chlorhexidine gluconate (0.05% w/w) and octenidine dihydrochloride (3.75 μg/mL)). Treatments were dispensed onto a gauze pad that was placed over the wound. Each gauze pad remained in place for 1 minute to allow the treatment to saturate the wound bed, followed by removal of the gauze. Excess treatment present in gauze was wrung out into the wound, the peri-wound area was wiped dry, and the wound was covered with a sterile Tegaderm™ dressing.

*Wound Assessments-* Each animal had wound assessments on study day 0 (post-wound creation prior to 3D biofilm placement); prior to each treatment on study days 3, 7, 10, 14, and 17; and on study day 21 prior to termination. All animals had wounds assessed (visually) and scored for wound bed and exudate.

*Wound Bed Scoring-* Wound beds were scored for each animal on study days 0, 3, 7, 10, 14, 17, and 21. Wound bed score values included 0 (dry), 1 (mildly moist), 2 (moderately moist), or 3 (severely moist). The results show that on study day 3, treatment with the combination formulation (containing both chlorhexidine gluconate (0.05% w/w) and octenidine dihydrochloride (3.75 μg/mL)) had significantly improved wound bed scoring compared to the 0.9% saline treatment and the chlorhexidine (0.05% w/w) alone treatment, with p-values of less than 0.0001 and 0.0049, respectively. This result demonstrates that treatment with a combination of chlorhexidine and octenidine results in an improved wound bed score compared to saline treatment or treatment with chlorhexidine alone.

Table 8. Mean Assessments of Wound Bed Score

| Study Group | Day 0 | Day 3 | Day 7 | Day 10 | Day 14 | Day 17 | Day 21 |
|---|---|---|---|---|---|---|---|
| Group 1-0.9% w/w saline | 1.0 | 2.0 * | 1.2 | 0.3 | 0.0 | 0.5 | 0.0 |
| Group 2-0.05% w/w CHG | 1.0 | 2.2 # | 0.5 | 0.0 | 0.0 | 0.0 | 0.5 |
| Group 3-OCT (3.75 μg/mL) + 0.05% w/w CHG | 1.0 | 1.0 *, # | 0.5 | 0.0 | 0.0 | 0.0 | 0.0 |
| * Significantly different exudate scores (p-value less than 0.0001)<br># Significantly different exudate scores (p-value of 0.0049) | | | | | | | |

*Exudate-* Wounds were scored for wound exudate. Exudate score values included 0 (none), 1 (slight), 2 (moderate), or 3 (severe). Treatment with the combination formulation (containing both chlorhexidine gluconate (0.05% w/w) and octenidine dihydrochloride (3.75 μg/mL)) had significantly lower exudate score on day 3 than the treatment with 0.9% saline or the treatment with chlorhexidine (0.05% w/w) alone, with p-values of less than 0.0001, and 0.0210, respectively.

Table 9. Mean Assessments of Exudate Score

| Study Group | Day 0 | Day 3 | Day 7 | Day 10 | Day 14 | Day 17 | Day 21 |
|---|---|---|---|---|---|---|---|
| Group 1-0.9% w/w saline | 0 | 2.0 * | 1.2 | 0.7 | 1.0 | 0.8 | 0.2 |
| Group 2-0.05% w/w CHG | 0 | 1.8 # | 0.7 | 0.7 | 1.0 | 0.7 | 0.8 |
| Group 3-OCT (3.75 μg/mL) + 0.05% w/w CHG | 0 | 1.0 *, # | 0.8 | 1.2 | 1.5 | 1.0 | 0.2 |
| * Significantly different exudate scores (p-value less than 0.0001)<br># Significantly different exudate scores (p-value of 0.0210) | | | | | | | |

*Body Weight-* Animal body weights were measured prior to wound creation (study day 0) and on study days 1, 2, 3, 7, 10, 14, 17, and 21. Several animals presented with body loss greater than 10% of the day 0 body weight measurements. Animals that presented with body weight loss greater than 10% had body weights measured daily to ensure that weight loss did not exceed 20% change/decrease in body weight from their day 0 body weight. Additionally, these animals were offered peanut butter until weight loss was less than 10% of their day 0 body weight. No animals exceeded 20% change from their day 0 body weight.

The body weight of all animals decreased slightly during the study period. There was no statistical difference in the body weights of animals between study groups suggesting the treatments were well tolerated (e.g., the treatment of both chlorhexidine gluconate (0.05% w/w) and octenidine dihydrochloride (3.75 μg/mL) was well tolerated and non-toxic).

Table 10. Mean Body Weight Assessment

| Study Group | Day 0 | Day 1 | Day 2 | Day 3 | Day 7 | Day 10 | Day 14 | Day 17 | Day 21 |
|---|---|---|---|---|---|---|---|---|---|
| Group 1-0.9% w/w saline | 48.87 | 47.22 | 46.29 | 46.54 | 45.59 | 45.16 | 46.29 | 45.21 | 44.99 |
| Group 2-0.05% w/w CHG | 47.71 | 46.33 | 45.63 | 45.77 | 45.17 | 44.36 | 44.67 | 44.28 | 44.28 |
| Group 3-OCT (3.75 μg/mL) + 0.05% w/w CHG | 45.95 | 45.24 | 44.43 | 45.16 | 44.69 | 43.54 | 44.04 | 42.65 | 42.60 |

*Colony Forming Unit (CFU) Quantification, Day 21-* Bacterial counts of the wound were determined at the termination of the study. Three animals per group were designated to have wound tissue analyzed for CFU quantification. Wound tissue and the surrounding area were harvested on study day 21 and placed into a conical tube (10 mL) containing phosphate buffered saline (PBS, 1 mL). Samples were transferred for same day processing. The tissue was resuspended in 1 mL of PBS and sonicated (3 x 30 seconds, 1 minute break between sonication bursts). An aliquot (100 μL) of the sonicated solution was serially diluted and an aliquot (100 μL) of each dilution was plated on tryptic soy agar (TSA) to determine the total bacterial counts in each specimen.

Treatment with the combination formulation (containing both chlorhexidine gluconate (0.05% w/w) and octenidine dihydrochloride (3.75 μg/mL)) significantly decreased the bacterial wound burden (as assessed by CFU) compared to saline treatment. The CFU was decreased by about 72-fold (72.52-fold). The p-value was 0.0062. Treatment with the chlorhexidine gluconate (0.05% w/w) formulation also significantly decreased the bacterial wound burden (as assessed by CFU). The CFU was decreased by about 4-fold (4.248 fold) compared to saline treatment. The p-value was 0.0282. Therefore, treatment with the combination formulation (containing chlorhexidine gluconate and octenidine dihydrochloride) led to a decrease in wound bacterial burden (in comparison to saline treatment) that was both greater in magnitude (about 17-fold) and more significant than treatment with chlorhexidine alone.

Table 11. Wound bacterial burdens in CFU (CFU/mL)

| Study Group | Animal 1 CFU | Animal 2 CFU | Animal 3 CFU | Average CFU | Standard Deviation |
|---|---|---|---|---|---|
| Group 1- 0.9% w/w saline | 5.35E+07 | 2.81E+07 | 5.22E+07 | 4.46E+07 *, # | 1.43E+07 |
| Group 2- 0.05% w/w CHG | 1.35E+06 | 3.83E+06 | 2.64E+07 | 1.05E+07 # | 1.38E+07 |
| Group 3- OCT (3.75 μg/mL) + 0.05% w/w CHG | 5.20E+05 | 1.27E+06 | 6.00E+04 | 6.15E+05 * | 6.08E+05 |
| * Significantly different wound CFU values (p-value less than 0.0062)<br># Significantly different wound CFU values (p-value of 0.0282) | | | | | |

*Histopathology Assessment-* Wound tissue from three animals per group was analyzed for histology by hematoxylin and eosin (H&E staining) on study day 21. Wound tissue and the surrounding area were harvested and fixed in paraformaldehyde (4% w/w) solution in 1x PHEM (PIPES, HEPES, EGTA, magnesium sulfate) buffer and stored at 2 - 8 °C until transferred for embedding and processing. Samples were progressively transferred to increasing amounts of ethanol until all samples were in 100% ethanol. Representative histological images are shown in FIGs. 4A-4C, FIGs. 5A-5C, and FIGs. 6A-6C. The histological images show that treatment with the combination formulation (containing both chlorhexidine gluconate (0.05% w/w) and octenidine dihydrochloride (3.75 μg/mL), FIGs. 6A-6C) resulted in improved healing of the epidermis and dermis compared to treatment with 0.9% w/w saline (FIGs. 4A-4C) or treatment with chlorhexidine gluconate (0.05% w/w) alone (FIGs. 5A-5C). This result demonstrates that wound irrigation solutions containing a synergistic combination of chlorhexidine and octenidine improve wound healing in comparison to saline treatments (a treatment without an antiseptic agent) or chlorhexidine alone (a treatment without a synergistic combination of antiseptic agents).

It should be understood that the examples and embodiments described herein are for illustrative purpose only and that various modifications or changes in light thereof will be suggested to persons skilled in the art.

## Claims

**1.** A non-sterile or sterile wound irrigation solution for use in a method for irrigating a wound comprising the steps of:

(a) providing the non-sterile or sterile wound irrigation solution in a wound irrigation device comprising a reservoir housing, wherein the wound irrigation device comprises a discharge means that directs a pressurized stream of the wound irrigation solution when the reservoir housing is pressurized, and wherein the wound irrigation solution comprises (i) sterile water, purified water, or water for pharmaceutical purposes; (ii) a chlorhexidine; and (iii) octenidine (OCT) or a pharmaceutically acceptable salt thereof;
(b) directing the discharge means and the reservoir housing so as to discharge the wound irrigation solution toward the wound; and
(c) discharging a stream of the wound irrigation solution from the reservoir housing and through the discharge means directed at the wound;

**characterized in that** the concentration of chlorhexidine is between about 100 ug/mL to about 5 mg/mL and the concentration of octenidine is between about 1ug/mL to about 100 ug/ml.

2. The non-sterile or sterile wound irrigation solution for use according to claim 1, wherein the wound irrigation solution consists of (i) sterile water, purified water, or water for pharmaceutical purposes; (ii) the chlorhexidine at a concentration that is between about 100 μg/mL to about 5 mg/mL; and (iii) the octenidine (OCT) or the pharmaceutically acceptable salt thereof at a concentration that is between about 1 μg/mL to about 100 μg/mL.

3. The non-sterile or sterile wound irrigation solution for use according to claim 1, wherein the chlorhexidine is in the form of chlorhexidine gluconate.

4. The non-sterile or sterile wound irrigation solution for use according to claim 1, wherein the concentration of the chlorhexidine is between about 200 μg/mL to about 1 mg/mL or about 100 μg/mL to about 500 μg/mL, preferably wherein the concentration of the chlorhexidine is about 500 μg/mL.

5. The non-sterile or sterile wound irrigation solution for use according to claim 1, wherein the octenidine (OCT) or the pharmaceutically acceptable salt thereof is octenidine dihydrochloride.

6. The non-sterile or sterile wound irrigation solution for use according to claim 1, wherein the concentration of the octenidine (OCT) or the pharmaceutically acceptable salt thereof is between about 1 μg/mL to about 20 μg/mL, preferably wherein the concentration of the octenidine (OCT) or the pharmaceutically acceptable salt thereof is between about 1 μg/mL to about 5 μg/mL or about 2 μg/mL to about 10 μg/mL, more preferably wherein the concentration of the octenidine (OCT) or the pharmaceutically acceptable salt thereof is about 1.875 μg/mL or about 3.75 μg/mL.

7. The non-sterile or sterile wound irrigation solution for use according to claim 1, wherein an amount of irrigation time required to reduce a bacterial burden of the wound or a fungal burden of the wound by about 100-fold compared to an analogous method using an alternative irrigation solution comprising the same concentration of chlorhexidine without octenidine is reduced by about 50%.

8. The non-sterile or sterile wound irrigation solution for use according to claim 1, wherein the irrigation solution comprises a combination of the chlorhexidine and the octenidine (OCT) or the pharmaceutically acceptable salt thereof that is synergistically microbiocidal or microbiostatic to a fungus or a bacterium.

9. The non-sterile or sterile wound irrigation solution for use according to claim 8, wherein the fungus or the bacterium is selected from Candida species, E. coli, Staphylococcus species, Ralstonia species, Pseudomonas species, or a combination thereof.

10. The non-sterile or sterile wound irrigation solution for use according to claim 7, wherein the amount of irrigation time required is within 15 minutes.

11. The non-sterile or sterile wound irrigation solution for use according to claim 1, wherein about 10 ml to about 1000 ml of wound irrigation solution is applied to the wound.

12. The non-sterile or sterile wound irrigation solution for use according to claim 1, wherein the wound irrigation solution is used to kill a microorganism selected from the group consisting of Candida species, Escherichia species Staphylococcus species, Ralstonia species, Pseudomonas species, and a combination thereof, or used to kill C. albicans, E. coli, methicillin-resistant Staphylococcus aureus (MRSA), Staphylococcus aureus, Staphylococcus epidermidis,

Ralstonia pickettii, P. aeruginosa, or a combination thereof.

13. The non-sterile or sterile wound irrigation solution for use according to claim 1, wherein the wound is a human skin wound and the method is used to treat the human skin wound.

14. The non-sterile or sterile wound irrigation solution for use according to claim 1, wherein the wound is selected from the group consisting of cuts, scrapes, surgical wounds, punctures, and abrasions.

15. The non-sterile or sterile wound irrigation solution for use according to claim 1, wherein a property of wound healing is improved compared to irrigation of the wound using an analogous method using an alternative wound irrigation solution which either

(i) consists of a saline solution, or
(ii) consists of the same chlorhexidine concentration without octenidine,

wherein the property of wound healing is wound bed scoring, exudate, wound microorganism burden, or a combination thereof.

## Patentansprüche

1. Nicht-sterile oder sterile Wundspüllösung zur Verwendung in einem Verfahren zum Spülen einer Wunde, umfassend die folgenden Schritte:

(a) Bereitstellen der nicht-sterilen oder sterilen Wundspüllösung in einer Wundspülvorrichtung, die ein Reservoirgehäuse umfasst, wobei die Wundspülvorrichtung ein Abgabemittel umfasst, das einen druckbeaufschlagten Strom der Wundspüllösung richtet, wenn das Reservoirgehäuse druckbeaufschlagt ist, und wobei die Wundspüllösung (i) steriles Wasser, gereinigtes Wasser oder Wasser für pharmazeutische Zwecke; (ii) Chlorhexidin; und (iii) Octenidin (OCT) oder ein pharmazeutisch verträgliches Salz davon umfasst;
(b) Richten des Verabreichungsmittels und des Reservoirgehäuses so, dass die Wundspüllösung in Richtung der Wunde verabreicht wird; und
(c) Verabreichen eines Stroms der Wundspüllösung aus dem Reservoirgehäuse und durch das auf die Wunde gerichtete Verabreichungsmittel;

**dadurch gekennzeichnet, dass** die Konzentration von Chlorhexidin zwischen etwa 100 μg/ml und etwa 5 mg/ml beträgt und die Konzentration von Octenidin zwischen etwa 1 μg/ml und etwa 100 μg/ml beträgt.

2. Nicht-sterile oder sterile Wundspüllösung zur Verwendung nach Anspruch 1, wobei die Wundspüllösung besteht aus (i) sterilem Wasser, gereinigtem Wasser, oder Wasser für pharmazeutische Zwecke; (ii) Chlorhexidin in einer Konzentration, die zwischen etwa 100 μg/ml und etwa 5 mg/ml beträgt; und (iii) Octenidin (OCT) oder dem pharmazeutisch verträglichen Salz davon in einer Konzentration, die zwischen etwa 1 μg/ml und etwa 100 μg/ml beträgt.

3. Nicht-sterile oder sterile Wundspüllösung zur Verwendung nach Anspruch 1, wobei Chlorhexidin in Form von Chlorhexidindgluconat vorliegt.

4. Nicht-sterile oder sterile Wundspüllösung zur Verwendung nach Anspruch 1, wobei die Konzentration an Chlorhexidin zwischen etwa 200 μg/ml und etwa 1 mg/ml oder etwa 100 μg/ml und etwa 500 μg/ml beträgt, wobei bevorzugt die Konzentration an Chlorhexidin etwa 500 μg/ml beträgt.

5. Nicht-sterile oder sterile Wundspüllösung zur Verwendung nach Anspruch 1, wobei Octenidin (OCT) oder das pharmazeutisch verträgliche Salz davon Octenidindihydrochlorid ist.

6. Nicht-sterile oder sterile Wundspüllösung zur Verwendung nach Anspruch 1, wobei die Konzentration an Octenidin (OCT) oder des pharmazeutisch verträglichen Salzes davon zwischen etwa 1 μg/ml und etwa 20 μg/ml beträgt, wobei bevorzugt die Konzentration an Octenidin (OCT) oder des pharmazeutisch verträglichen Salzes davon zwischen etwa 1 μg/ml und etwa 5 μg/ml oder etwa 2 μg/ml und etwa 10 μg/ml beträgt, wobei besonders bevorzugt die Konzentration an Octenidin (OCT) oder des pharmazeutisch verträglichen Salzes davon etwa 1,875 μg/ml oder etwa

3,75 µg/ml beträgt.

**7.** Nicht-sterile oder sterile Wundspüllösung zur Verwendung nach Anspruch 1, wobei eine Spüldauer, die nötig ist, um eine Bakterienbelastung der Wunde oder eine Pilzbelastung der Wunde um etwa das 100-fache im Vergleich zu einem analogen Verfahren, das eine alternative Spüllösung verwendet, die die gleiche Konzentration an Chlorhexidin ohne Octenidin umfasst, zu verringern, um etwa 50 % verkürzt ist.

**8.** Nicht-sterile oder sterile Wundspüllösung zur Verwendung nach Anspruch 1, wobei die Spüllösung eine Kombination aus Chlorhexidin und Octenidin (OCT) oder des pharmazeutisch verträglichen Salzes davon umfasst, die gegenüber einem Pilz oder einem Bakterium synergistisch mikrobizid oder mikrobiostatisch ist.

**9.** Nicht-sterile oder sterile Wundspüllösung zur Verwendung nach Anspruch 8, wobei der Pilz oder das Bakterium ausgewählt ist aus Candida-Spezies, E. coli, Staphylococcus-Spezies, Ralstonia-Spezies, Pseudomonas-Spezies oder einer Kombination davon.

**10.** Nicht-sterile oder sterile Wundspüllösung zur Verwendung nach Anspruch 7, wobei die nötige Spüldauer innerhalb von 15 Minuten liegt.

**11.** Nicht-sterile oder sterile Wundspüllösung zur Verwendung nach Anspruch 1, wobei etwa 10 ml bis etwa 1000 ml Wundspüllösung auf die Wunde angewendet werden.

**12.** Nicht-sterile oder sterile Wundspüllösung zur Verwendung nach Anspruch 1, wobei die Wundspüllösung verwendet wird, um einen Mikroorganismus abzutöten, der ausgewählt ist aus der Gruppe bestehend aus Candida-Spezies, Escherichia-Spezies, Staphylococcus-Spezies, Ralstonia-Spezies, Pseudomonas-Spezies und einer Kombination davon, oder verwendet wird, um C. albicans, E. coli, Methicillinresistenten Staphylococcus aureus (MRSA), Staphylococcus aureus, Staphylococcus epidermidis, Ralstonia pickettii, P. aeruginosa oder eine Kombination davon abzutöten.

**13.** Nicht-sterile oder sterile Wundspüllösung zur Verwendung nach Anspruch 1, wobei die Wunde eine menschliche Hautwunde ist und das Verfahren zum Behandeln der menschlichen Hautwunde verwendet wird.

**14.** Nicht-sterile oder sterile Wundspüllösung zur Verwendung nach Anspruch 1, wobei die Wunde ausgewählt ist aus der Gruppe bestehend aus Schnitten, Kratzern, Operationswunden, Einstichen und Abschürfungen.

**15.** Nicht-sterile oder sterile Wundspüllösung zur Verwendung nach Anspruch 1, wobei eine Wundheilungseigenschaft im Vergleich zur Spülung der Wunde mittels eines analogen Verfahrens, das eine alternative Wundspüllösung verwendet, die entweder

(i) aus einer physiologischen Kochsalzlösung besteht oder
(ii) aus der gleichen Chlorhexidinkonzentration ohne Octenidin besteht, verbessert ist,

wobei die Wundheilungseigenschaft Wundbettbewertung, Exsudat, Mikroorganismenbelastung der Wunde oder eine Kombination davon ist.

## Revendications

**1.** Solution non stérile ou stérile pour l'irrigation des plaies, destinée à être utilisée dans un procédé d'irrigation de plaie, comprenant les étapes consistant à :

(a) fournir la solution non stérile ou stérile pour l'irrigation des plaies dans un dispositif d'irrigation des plaies comprenant un boîtier de réservoir, le dispositif d'irrigation des plaies comprenant un moyen d'évacuation qui dirige un flux mis sous pression de la solution pour l'irrigation des plaies lorsque le boîtier de réservoir est mis sous pression, et la solution d'irrigation de plaie comprenant (i) de l'eau stérile, de l'eau purifiée ou de l'eau à des fins pharmaceutiques ; (ii) de la chlorhexidine ; et (iii) de l'octénidine (OCT) ou un sel de celle-ci pharmaceutiquement acceptable ;
b) diriger le moyen d'évacuation et le boîtier du réservoir de manière à évacuer la solution pour l'irrigation des plaies en direction de la plaie ; et

c) évacuer un flux de la solution pour l'irrigation des plaies provenant du boîtier du réservoir et à travers le moyen d'évacuation dirigé sur la plaie ;

**caractérisée en ce que** la concentration de chlorhexidine est comprise entre environ 100 μg/ml et environ 5 mg/ml et la concentration d'octénidine est comprise entre environ 1 μg/ml et environ 100 μg/ml.

**2.** Solution non stérile ou stérile pour l'irrigation des plaies destinée à être utilisée selon la revendication 1, dans laquelle la solution pour l'irrigation des plaies consiste en (i) de l'eau stérile, de l'eau purifiée ou de l'eau à des fins pharmaceutiques ; (ii) de la chlorhexidine à une concentration qui est comprise entre environ 100 μg/ml et environ 5 mg/ml ; et (iii) de l'octénidine (OCT) ou un sel de celle-ci pharmaceutiquement acceptable à une concentration qui est comprise entre environ 1 μg/ml et environ 100 μg/ml.

**3.** Solution non stérile ou stérile pour l'irrigation des plaies destinée à être utilisée selon la revendication 1, dans laquelle la chlorhexidine se présente sous la forme de gluconate de chlorhexidine.

**4.** Solution non stérile ou stérile pour l'irrigation des plaies destinée à être utilisée selon la revendication 1, dans laquelle la concentration de la chlorhexidine est comprise entre environ 200 μg/ml et environ 1 mg/ml ou entre environ 100 μg/ml et environ 500 μg/ml, de préférence dans laquelle la concentration de la chlorhexidine est d'environ 500 μg/ml.

**5.** Solution non stérile ou stérile pour l'irrigation des plaies destinée à être utilisée selon la revendication 1, dans laquelle l'octénidine (OCT) ou le sel de celle-ci pharmaceutiquement acceptable est le dichlorhydrure d'octénidine.

**6.** Solution non stérile ou stérile pour l'irrigation des plaies destinée à être utilisée selon la revendication 1, dans laquelle la concentration de l'octénidine (OCT) ou du sel de celle-ci pharmaceutiquement acceptable est comprise entre environ 1 μg/ml et environ 20 μg/ml, de manière préférée dans laquelle la concentration de l'octénidine (OCT) ou du sel de celle-ci pharmaceutiquement acceptable est comprise entre environ 1 μg/ml et environ 5 μg/ml ou entre environ 2 μg/ml et environ 10 μg/ml, de manière plus préférée dans laquelle la concentration de l'octénidine (OCT) ou du sel de celle-ci pharmaceutiquement acceptable est d'environ 1,875 μg/ml ou d'environ 3,75 μg/ml.

**7.** Solution non stérile ou stérile pour l'irrigation des plaies destinée à être utilisée selon la revendication 1, dans laquelle une durée d'irrigation requise pour réduire une charge bactérienne de la plaie ou une charge fongique de la plaie d'environ 100 fois par rapport à un procédé analogue utilisant une solution d'irrigation alternative comprenant la même concentration de chlorhexidine sans octénidine, est réduite d'environ 50 %.

**8.** Solution non stérile ou stérile pour l'irrigation des plaies destinée à être utilisée selon la revendication 1, dans laquelle la solution d'irrigation comprend une combinaison de la chlorhexidine et de l'octénidine (OCT) ou du sel de celle-ci pharmaceutiquement acceptable qui est synergiquement microbiocide ou microbiostatique vis-à-vis d'un fonge ou d'une bactérie.

**9.** Solution non stérile ou stérile pour l'irrigation des plaies destinée à être utilisée selon la revendication 8, dans laquelle le fonge ou la bactérie est choisi parmi l'espèce Candida, E. coli, l'espèce Staphylococcus, l'espèce Ralstonia, l'espèce Pseudomonas ou une combinaison de ceux-ci.

**10.** Solution non stérile ou stérile pour l'irrigation des plaies destinée à être utilisée selon la revendication 7, dans laquelle la durée d'irrigation requise est de moins de 15 minutes.

**11.** Solution non stérile ou stérile pour l'irrigation des plaies destinée à être utilisée selon la revendication 1, dans laquelle environ 10 ml à environ 1 000 ml de solution pour l'irrigation des plaies sont appliqués à la plaie.

**12.** Solution non stérile ou stérile pour l'irrigation des plaies destinée à être utilisée selon la revendication 1, dans laquelle la solution pour l'irrigation des plaies est utilisée pour tuer un micro-organisme choisi parmi le groupe constitué de l'espèce Candida, de l'espèce Escherichi, de l'espèce Staphylococcus, de l'espèce Ralstonia, de l'espèce Pseudomonas et d'une combinaison de celles-ci, ou est utilisée pour tuer C. albicans, E. coli, Staphylococcus aureus résistant à la méthicilline (MRSA), Staphylococcus aureus, Staphylococcus epidermidis, Ralstonia pickettii, P. aeruginosa ou une combinaison de ceux-ci.

**13.** Solution non stérile ou stérile pour l'irrigation des plaies destinée à être utilisée selon la revendication 1, dans laquelle la plaie est une plaie cutanée humaine et le procédé est utilisé pour traiter la plaie cutanée humaine.

**14.** Solution non stérile ou stérile pour l'irrigation des plaies destinée à être utilisée selon la revendication 1, dans laquelle la plaie est choisie parmi le groupe constitué de coupures, d'égratignures, de plaies chirurgicales, de perforations, et d'écorchures.

**15.** Solution non stérile ou stérile pour l'irrigation des plaies destinée à être utilisée selon la revendication 1, dans laquelle une propriété de cicatrisation de plaie est améliorée par rapport à l'irrigation de la plaie en utilisant un procédé analogue utilisant une solution alternative pour l'irrigation des plaies qui ou

(i) se compose d'une solution saline, ou
(ii) se compose de la même concentration de chlorhexidine sans octénidine,

dans laquelle la propriété de cicatrisation de plaie est la préparation d'un lit de la plaie, un exsudat, une charge de micro-organismes de plaie ou une combinaison de ceux-ci.

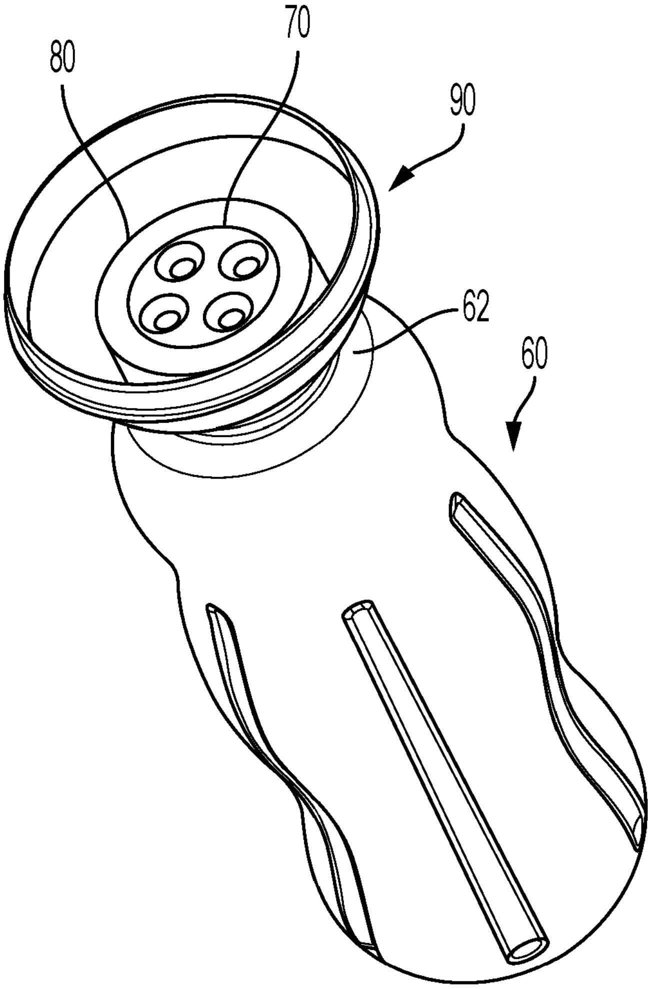


FIG. 1

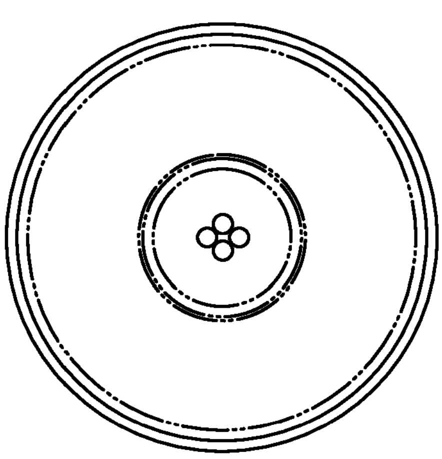

FIG. 2A

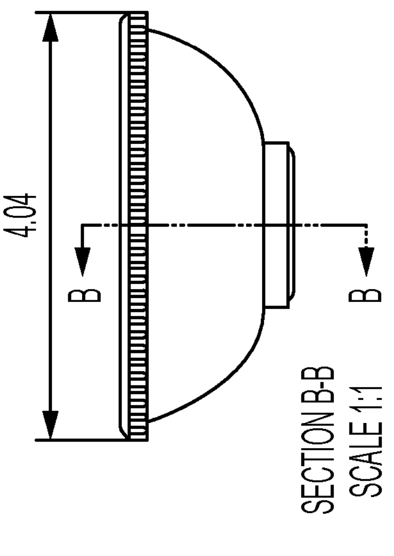


FIG. 2B

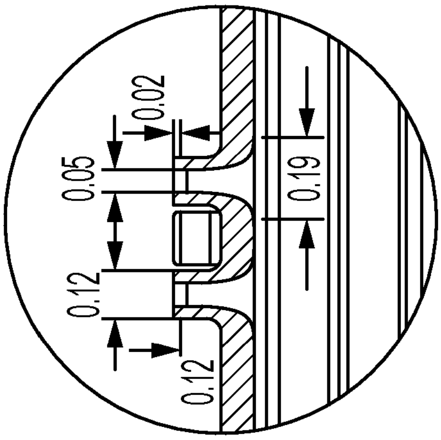


FIG. 2C

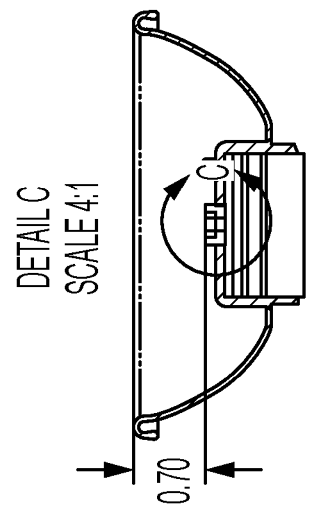


FIG. 2D

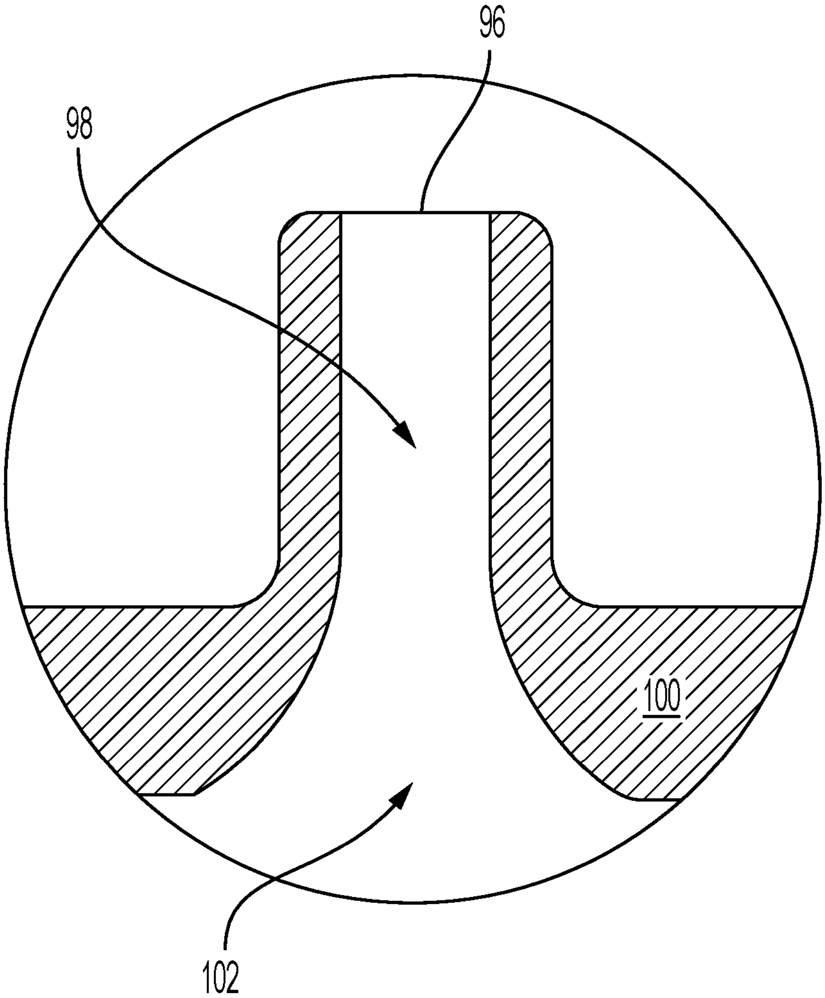


FIG. 3

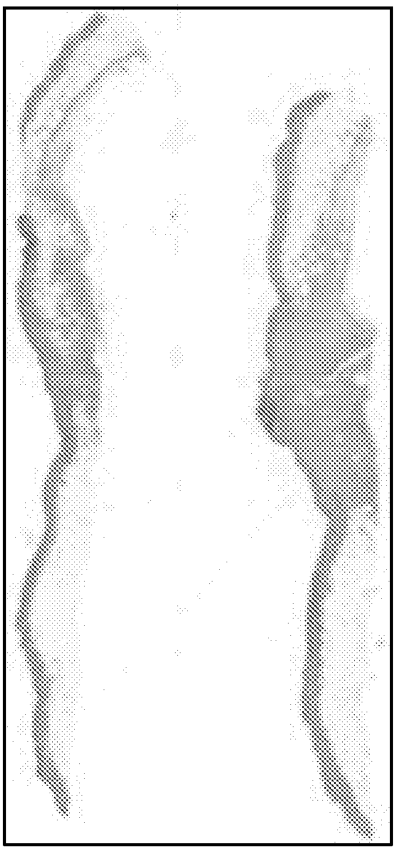

FIG. 4A

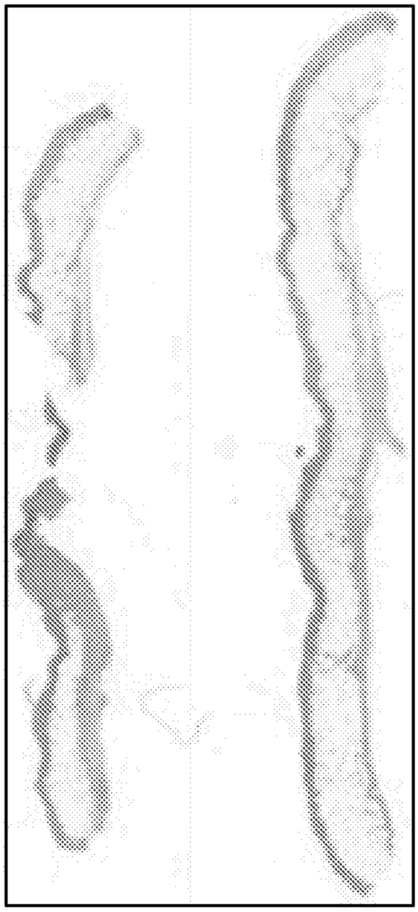

FIG. 4B

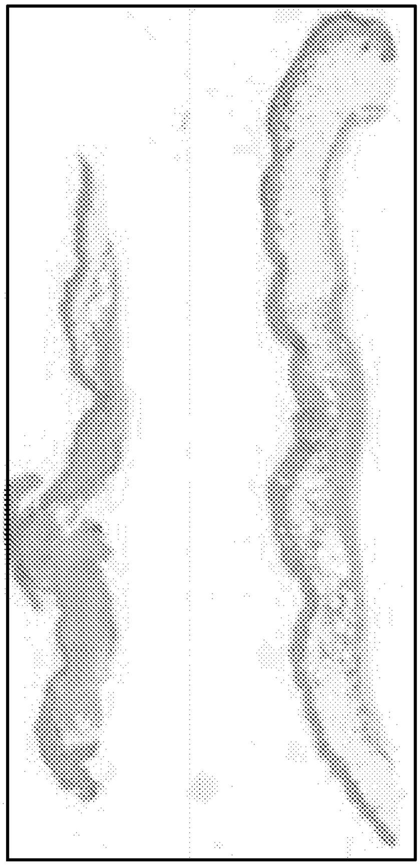

FIG. 4C

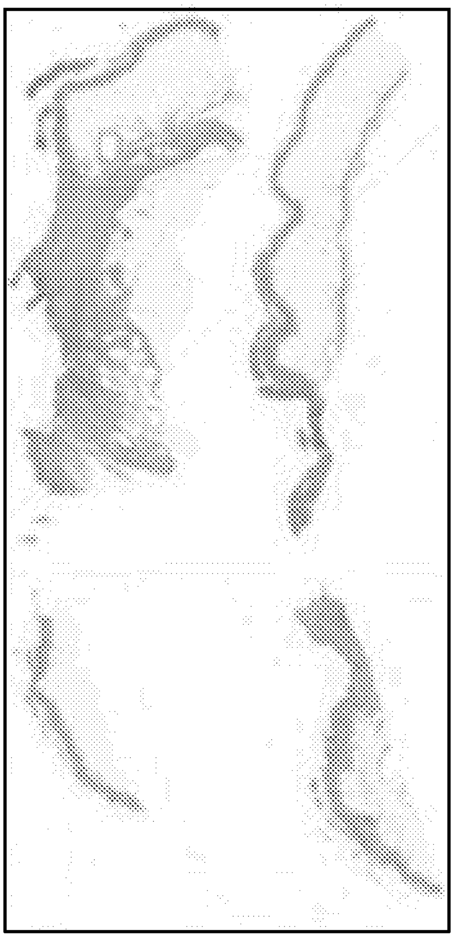

FIG. 5A

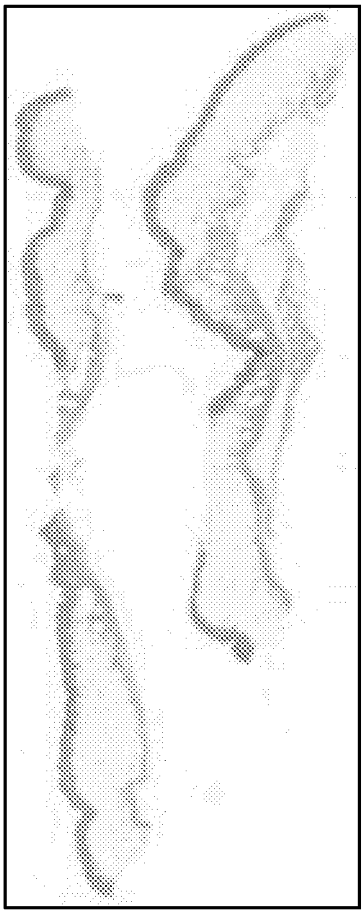

FIG. 5B

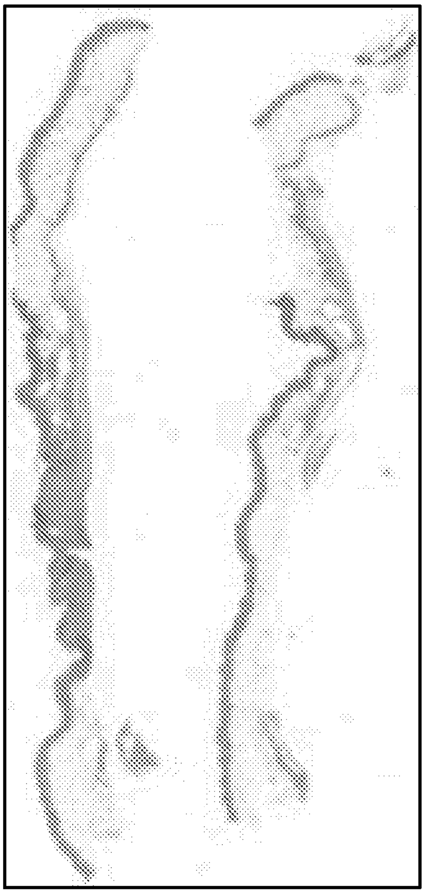

FIG. 5C

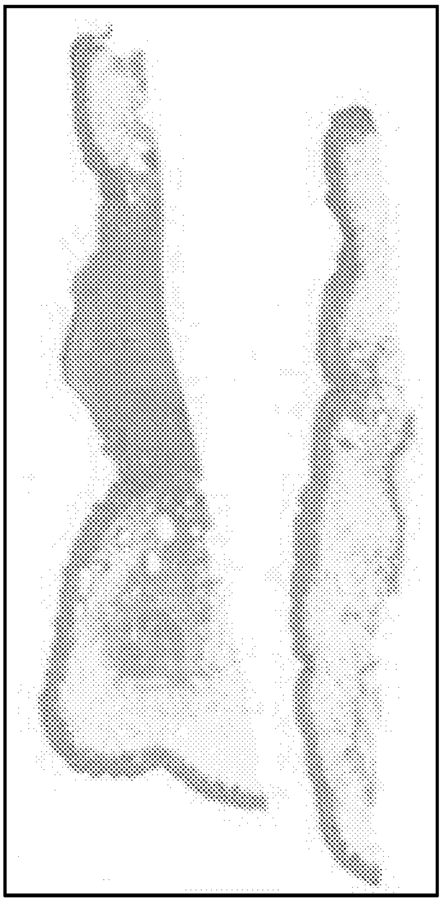

FIG. 6A

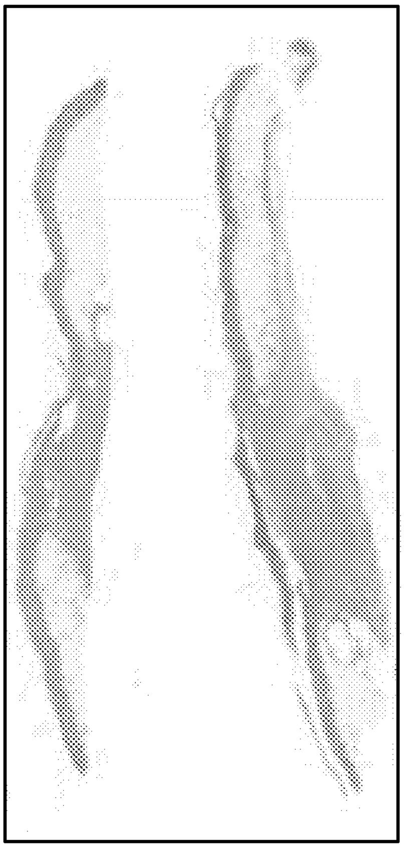

FIG. 6B

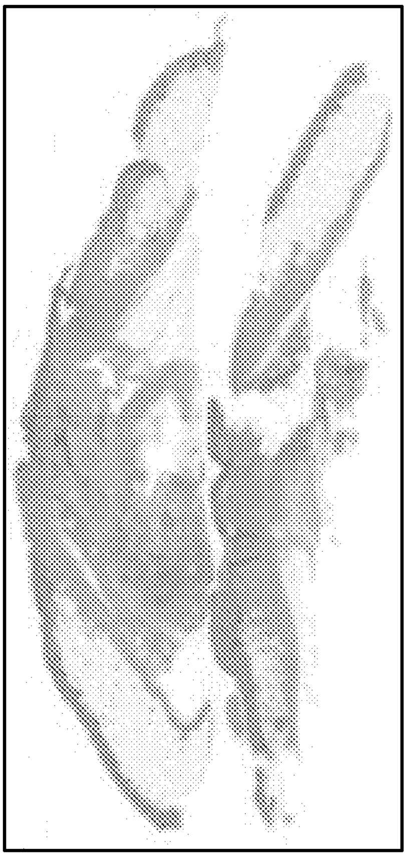

FIG. 6C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5071104 A
- US 5133701 A
- US 5830197 A
- US 6468253 B
- WO 200015279 A1
- WO 2002007799 A2
- US 2022054734 A
- US 20050191364 A1
- WO 2005030297 A

### Non-patent literature cited in the description


- **DIRE, DANIEL I.** A comparison of Wound Irrigation Solutions Used in the Emergency Department. *Annals of Emergency Medicine*, 1990, vol. 19 (6), 704-708
- Maximizing A Wound's Potential For Healing. Emergency Medicine Reports. 1989, vol. 10, 83-89
- Soft Tissue Procedures: Principles of Wound Management. **LAMMERS, RICHARD L.** Clinical Procedures in Emergency Medicine. W.B. Saunders Company, 1991, 515-521
- Minor Lacerations and Abrasions. **CRACROFT, DAVIS**. Emergency Medicine: A Comprehensive Review. Aspen Publishing Co., 1987, 107-110
- Management of Wounds. **MULLIKEN, JOHN B**. Emergency Medicine. John Wiley & Sons, 1984, 283-286
- **F. P. DEUS** ; **A. OUANOUNOU**. *Chlorhexidine in Dentistry: Pharmacology, Uses, and Adverse Effects. Int. Dent. J.*, 2022, vol. 72 (3), 269-277
- **Y. H. HUANG** ; **J. T. HUANG**. Use of chlorhexidine to eradicate oropharyngeal SARS-CoV-2 in COVID-19 patients. *J. Med. Virol.*, 2021, vol. 93 (7), 4370-4373
- **BRAFF, M. et al.** Cutaneous Defense Mechanisms by Antimicrobial Peptides. *J Invest Dermatol*, 2005, vol. 125, 9-13